# EUROPEAN PATENT SPECIFICATION

(11) **EP 3 392 242 B1**
(45) Date of publication and mention of the grant of the patent: **24.02.2021**
(21) Application number: 17804787.4
(22) Date of filing: 20.07.2017
(51) Int. Cl.: H01L 51/54, C07D 251/24, C07D 403/10, C07D 209/82, C07D 307/91, C07D 333/76, C09K 11/06

(54) **NOVEL HETEROCYCLIC COMPOUND AND ORGANIC LIGHT-EMITTING DEVICE USING SAME**
NEUARTIGE HETEROCYCLISCHE VERBINDUNG UND ORGANISCHE LICHTEMITTIERENDE VORRICHTUNG DAMIT
NOUVEAU COMPOSÉ HÉTÉROCYLIQUE ET DISPOSITIF ÉLECTROLUMINESCENT ORGANIQUE UTILISANT CELUI-CI

(30) Priority: 20.07.2016 KR 20160092206
(43) Date of publication of application: 24.10.2018
(73) Proprietor: LG Chem, Ltd., Seoul 07336 (KR)
(72) Inventor: JUNG, Min Woo, Daejeon 34122 (KR); LEE, Dong Hoon, Daejeon 34122 (KR); HUH, Jungoh, Daejeon 34122 (KR); JANG, Boonjae, Daejeon 34122 (KR); KANG, Minyoung, Daejeon 34122 (KR); HEO, Dong Uk, Daejeon 34122 (KR); HAN, Mi Yeon, Daejeon 34122 (KR)
(74) Representative: Hoffmann Eitle
(86) International application number: PCT/KR2017/007840
(87) International publication number: WO 2018/016898

(56) References cited:
- EP-A1- 3 072 943
- WO-A1-2016/013867
- JP-A- 2011 256 143
- KR-A- 20110 076 892
- KR-A- 20110 108 313
- KR-A- 20120 025 006
- KR-A- 20150 014 368
- KR-A- 20160 028 979
- US-A1- 2014 252 333
- WEI LI ET AL: "Bipolar host materials for high-efficiency blue phosphorescent and delayed-fluorescence OLEDs", JOURNAL OF MATERIALS CHEMISTRY C, vol. 3, no. 48, 1 January 2015 (2015-01-01), pages 12529-12538, XP055544709, UK ISSN: 2050-7526, DOI: 10.1039/C5TC02997J

## Description

### [TECHNICAL FIELD]

### Cross-reference to Related Application

This application claims the benefit of Korean Patent Application No. 10-2016-0092206 filed on July 20, 2016 with the Korean Intellectual Property Office.

The present disclosure relates to a novel heterocyclic compound and an organic light emitting device comprising the same.

### [BACKGROUND OF ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, excellent luminance, driving voltage and response speed, and thus many studies have proceeded.

The organic light emitting device generally has a structure which includes an anode, a cathode, and an organic material layer interposed between the anode and the cathode. The organic material layer frequently have a multilayered structure that includes different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode to the organic material layer, and when the injected holes and the electrons meet each other, an exciton is formed, and light is emitted when the exciton falls to a ground state again

Heterocyclic compounds are already disclosed in Patent Literature 0002 and 0003.

There is a continuing demand for developing a new material for organic materials used in such organic light emitting devices.

### [PRIOR ART LITERATURE]

### [PATENT LITERATURE]

(PATENT LITERATURE 0001) Korean Patent Publication No. 10-2000-0051826
(Patent Literature 0002) KR 2012 0025006 A
(Patent Literature 0003) KR 2015 0014368 A.

### [DETAILED DESCRIPTION OF THE INVENTION]

### [Technical Problem]

The present disclosure relates to a novel heterocyclic compound and an organic light emitting device comprising the same.

### [Technical Solution]

The present disclosure provides a compound represented by the following Chemical Formula 1 as defined in claim 1.

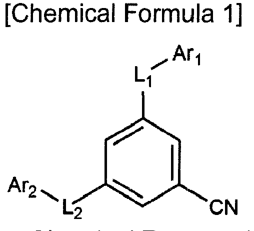

In addition, the present disclosure provides an organic light emitting device including a first electrode; a second electrode provided to face the first electrode; and one or more organic material layers provided between the first electrode and the second electrode, wherein one or more of the organic material layers comprise the compound represented by the Chemical Formula 1.

### [ADVANTAGEOUS EFFECTS]

The compound represented by the Chemical Formula 1 may be used as a material of an organic material layer of an organic light emitting device and may improve the efficiency, the low driving voltage and/or life time of the organic light emitting device. In particular, the compound represented by the Chemical Formula 1 may be used as a material for hole injection, hole transport, hole injection and transport, light emission, electron transport, or electron injection.

### [BRIEF DESCRIPTION OF DRAWINGS]

FIG. 1 shows an example of an organic light emitting device including a substrate (1), an anode (2), a light emitting layer (3), and a cathode (4).
FIG. 2 shows an example of an organic light emitting device including a substrate (1), an anode (2), a hole injection layer (5), a hole transport layer (6), a light emitting layer (7), and a cathode (4).

### [DETAILED DESCRIPTION OF THE EMBODIMENTS]

Hereinafter, the present disclosure will be described in more detail to help understanding of the present invention.

The present disclosure provides the compound represented by the Chemical Formula 1.

In the present disclosure, 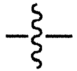 means a single bond connected to another substituent group.

As used herein, the term "substituted or unsubstituted" means that one or more substituent groups selected from the group consisting of deuterium; a halogen group; a nitrile group; a nitro group; a hydroxyl group; a carbonyl group; an ester group; an imide; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl groups; an aralkenyl group; an alkylaryl group; an alkylamine group; an aralkylamine group; a heteroarylamine group; an arylamine group; an arylphosphine group; or a heterocyclic group containing at least one of N, O, and S atoms are substituted or unsubstituted, or a substituent group where two or more substituent groups of the exemplified substituent groups are connected is substituted or unsubstituted. For example, the term "substituent group where two or more substituent groups are connected" may be a biphenyl group. That is, the biphenyl group may be an aryl group, or may be interpreted as a substituent group where two phenyl groups are connected.

In the present disclosure, the number of carbon atoms in a carbonyl group is not particularly limited, but 1 to 40 is preferable. Specifically, the carbonyl group may be a compound represented by following structures, but is not limited thereto.

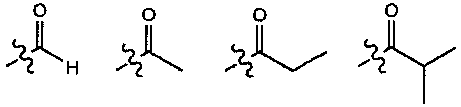

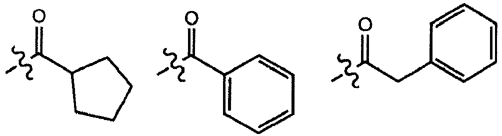

In the present disclosure, oxygen of an ester group may be substituted by a linear-chain, branched-chain, or cyclic alkyl group having 1 to 25 carbon atoms, or an aryl group having 6 to 25 carbon atoms. Specifically, the ester group may be a compound represented by following structures, but is not limited thereto.

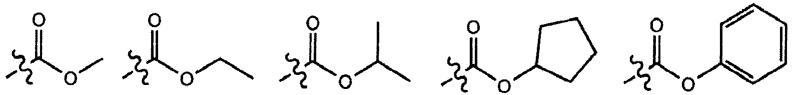

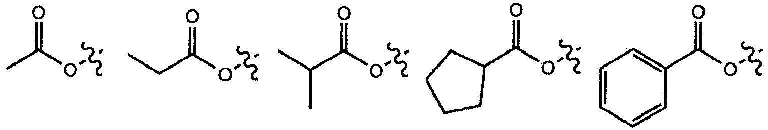

In the present disclosure, the number of carbon atoms in an imide group is not particularly limited, but 1 to 25 is preferable. Specifically, the imide group may be a compound represented by following structures, but is not limited thereto.

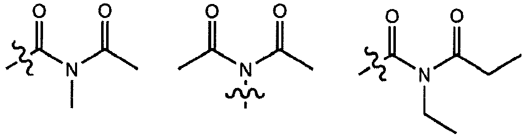

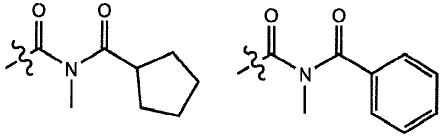

In the present disclosure, the silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present disclosure, the boron group specifically includes a trimethylboron group, a triethylboron group, a t-butyldimethylboron group, a triphenylboron group, and a phenylboron group, and the like, but is not limited thereto.

In the present disclosure, examples of a halogen group include fluorine, chlorine, bromine, or iodine.

In the present disclosure, an alkyl group may be a linear chain or a branched chain, and the number of carbon atoms thereof is not particularly limited but is preferably 1 to 40. According to one embodiment, the alkyl group has 1 to 20 carbon atoms. According to another embodiment, the alkyl group has 1 to 10 carbon atoms. According to another embodiment, the alkyl group has 1 to 6 carbon atoms. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present disclosure, the alkenyl group may be a linear chain or a branched chain, and the number of carbon atoms thereof is not particularly limited but is preferably 2 to 40. According to one embodiment, the alkenyl group has 2 to 20 carbon atoms. According to another embodiment, the alkenyl group has 2 to 10 carbon atoms. According to another embodiment, the alkenyl group has 2 to 6 carbon atoms. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the number of carbon atoms thereof is preferably 3 to 60. According to one embodiment, the cycloalkyl group has 3 to 30 carbon atoms. According to another embodiment, the cycloalkyl group has 3 to 20 carbon atoms. According to another embodiment, the cycloalkyl group has 3 to 6 carbon atoms. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, the aryl group is not particularly limited, but preferably has 6 to 60 carbon atoms, and may be a monocyclic aryl group or a polycyclic aryl group. According to one embodiment, the aryl group has 6 to 30 carbon atoms. According to one embodiment, the aryl group has 6 to 20 carbon atoms. The aryl group may be a phenyl group, a biphenyl group, a terphenyl group or the like as the monocyclic aryl group, but is not limited thereto. Examples of the polycyclic aryl group include a naphthyl group, an anthracenyl group, a phenanthryl group, a pyrenyl group, a perylenyl group, a chrysenyl group and a fluorenyl group or the like, but are not limited thereto.

In the present disclosure, a fluorenyl group may be substituted, and two substituent groups may be bonded to each other to form a spiro structure. In the case where the fluorenyl group is substituted, 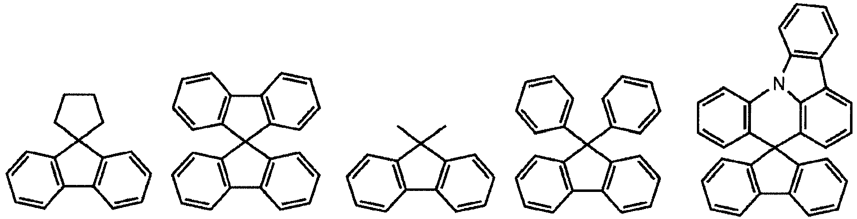 and the like can be formed. However, it is not limited thereto.

In the present disclosure, the heterocyclic group is a heterocyclic group including at least one of O, N, Si and S as a heteroatom, and the number of carbon atoms thereof is not particularly limited, but is preferably 2 to 60. Examples of the heterocyclic group include a thiophene group, a furan group, a pyrrole group, an imidazole group, a triazole group, an oxazole group, an oxadiazole group, a triazole group, a pyridyl group, a bipyridyl group, a pyrimidyl group, a triazine group, a triazole group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, a dibenzofuranyl group, and the like, but are not limited thereto.

In the present disclosure, the aryl group in the aralkyl group, the aralkenyl group, the alkylaryl group, and the arylamine group is the same as the above-mentioned examples of the aryl group. In the present disclosure, the alkyl group in the aralkyl group, the alkylaryl group and the alkylamine group is the same as the above-mentioned examples of the alkyl group. In the present disclosure, the heteroaryl in the heteroarylamines can be applied to the above-mentioned description of the heterocyclic group. In the present disclosure, the alkenyl group in the aralkenyl group is the same as the above-mentioned examples of the alkenyl group. In the present disclosure, the above-mentioned description of the aryl group may be applied except that the arylene is a divalent group. In the present disclosure, the above-mentioned description of the heterocyclic group can be applied except that the heteroarylene is a divalent group. In the present disclosure, the above-mentioned description of the aryl group or cycloalkyl group can be applied except that the hydrocarbon ring is not a monovalent group but formed by combining two substituent groups. In the present disclosure, the above-mentioned description of the heterocyclic group can be applied, except that the heterocycle is not a monovalent group but formed by combining two substituent groups.

Preferably, L₁ is a single bond, or any one selected from the group consisting of following structural formulae:

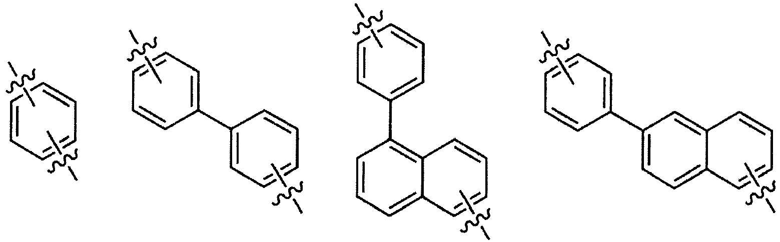

More preferably, L₁ is a single bond, or any one selected from the group consisting of following structural formulae:

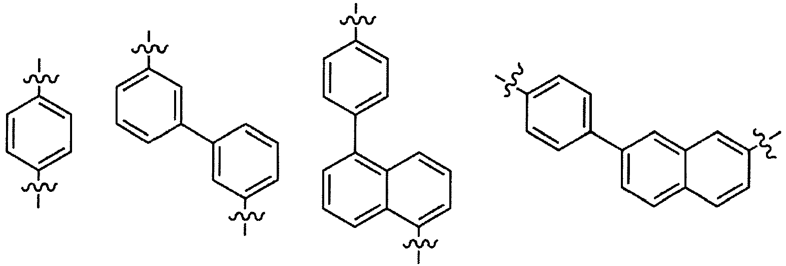

Preferably, L₂ is a single bond, 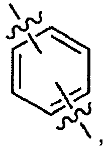 or 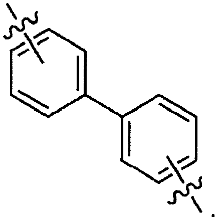 More preferably, L₂ is a single bond,

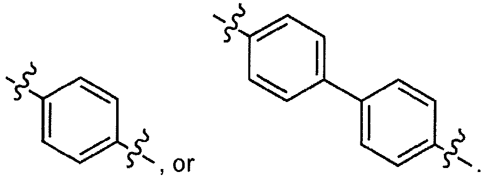

Ar₁ is any one selected from the group consisting of following structural formulae:

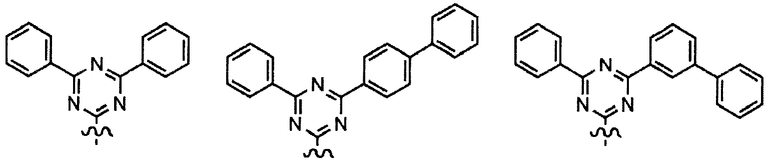

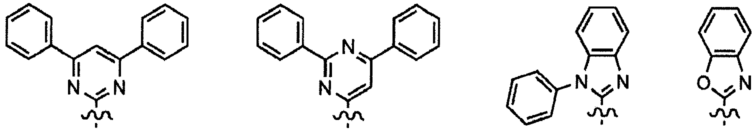

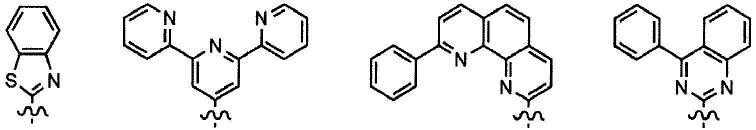

Preferably, X is S, or O.

R₁ is cyano, trifluoromethyl, or trifluoromethoxy.

Preferably, R₂ to R₁₀ and R₁₃ to R₁₉ are hydrogen.

The compound represented by the Chemical Formula 1 may be selected from the group consisting of following compounds:

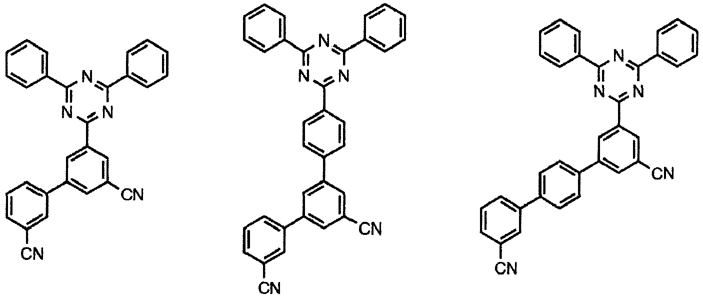

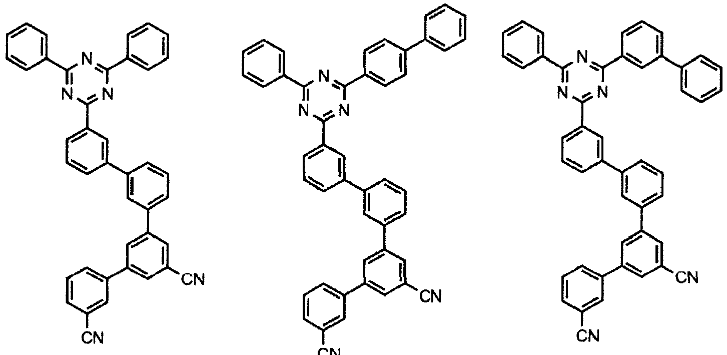

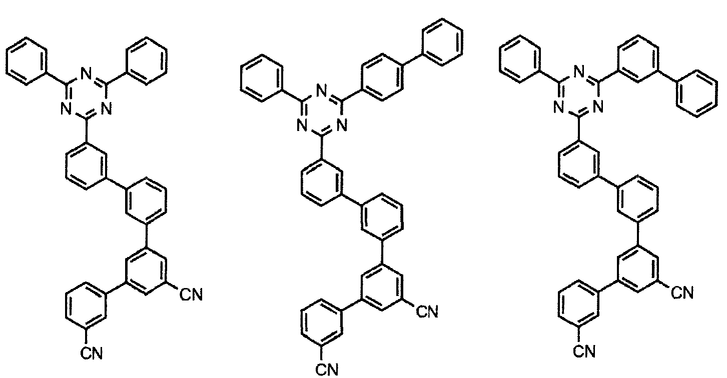

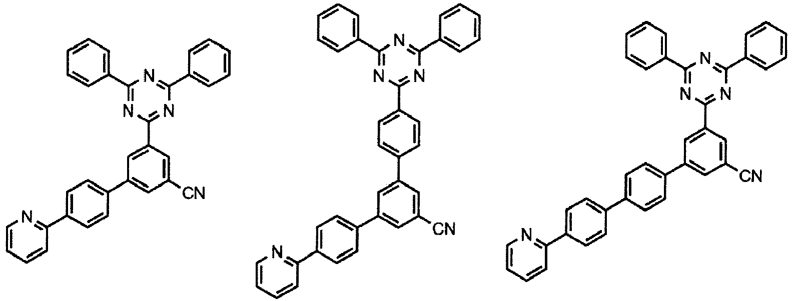

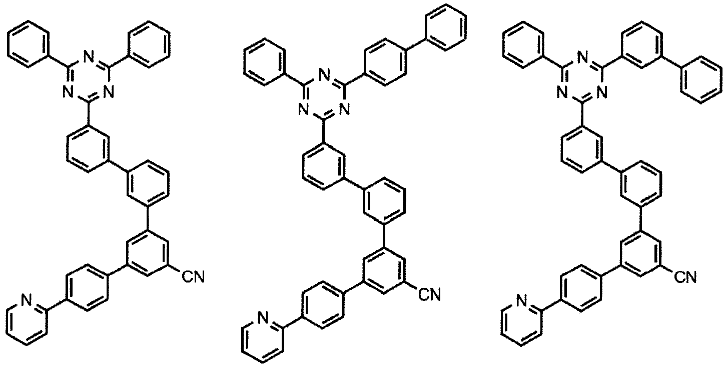

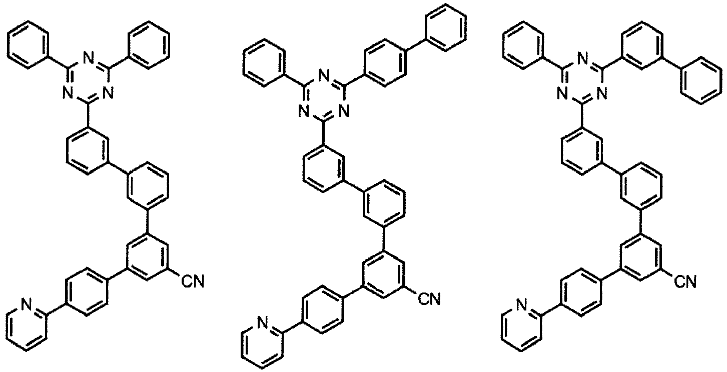

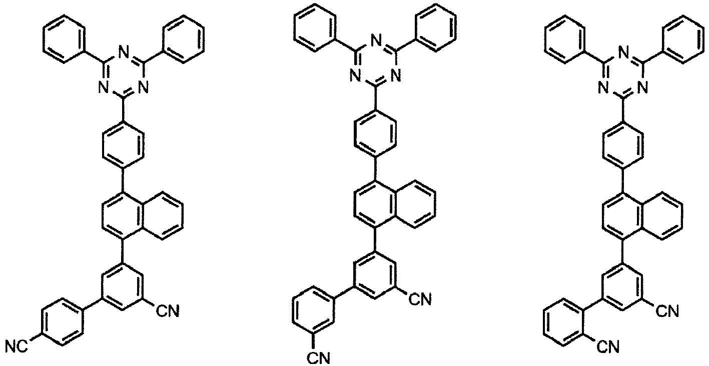

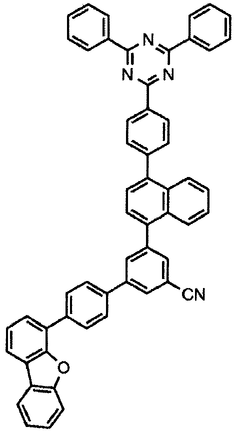

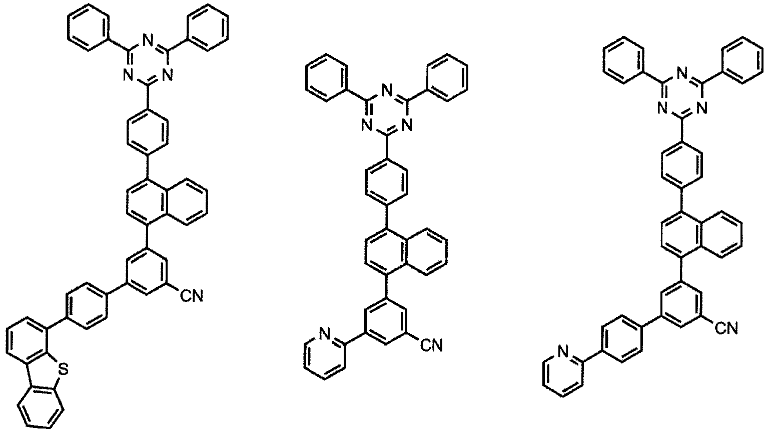

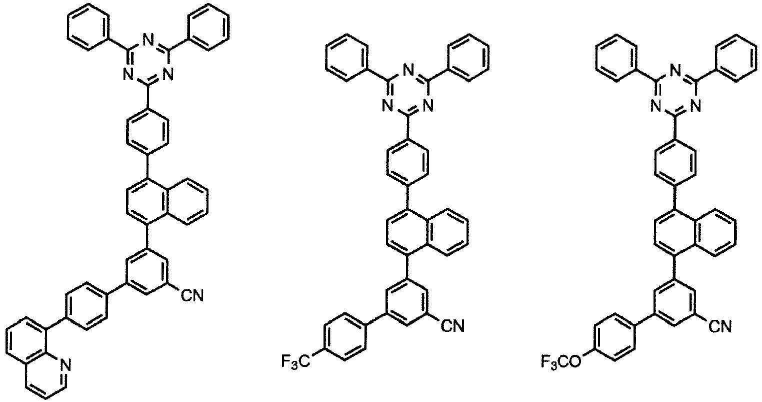

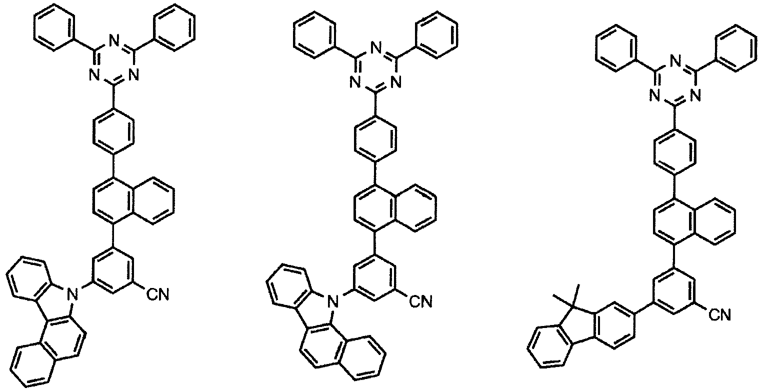

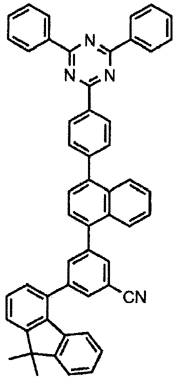

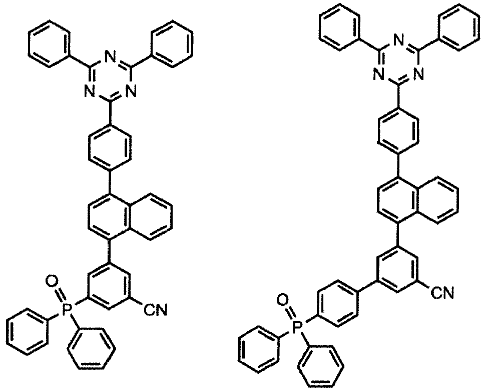

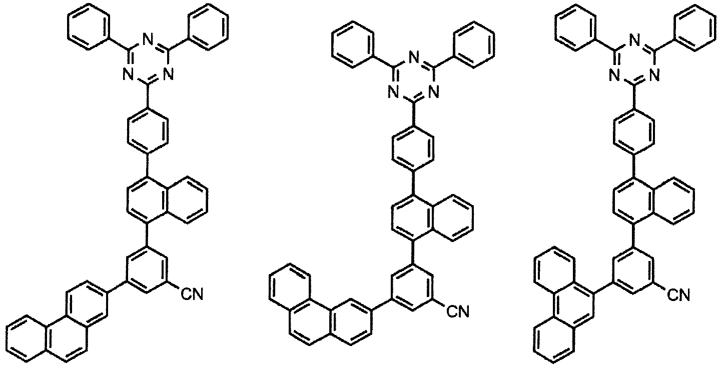

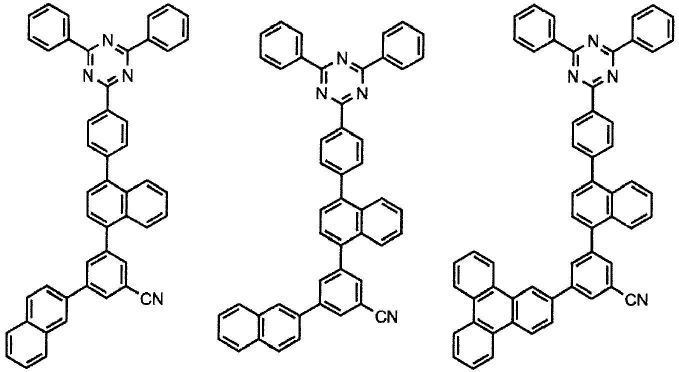

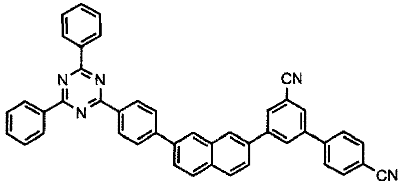

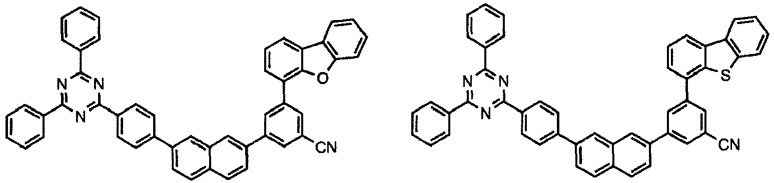

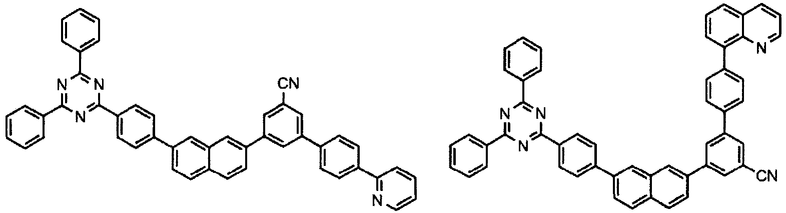

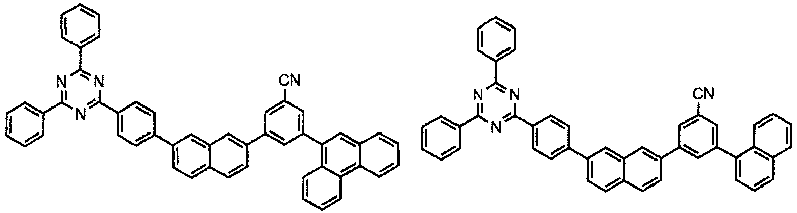

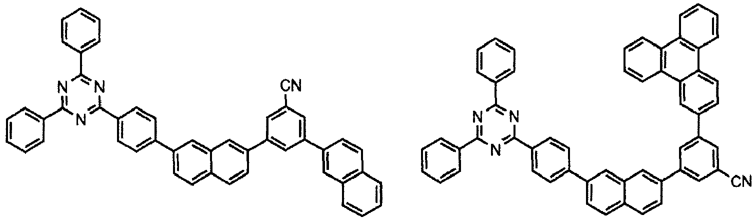

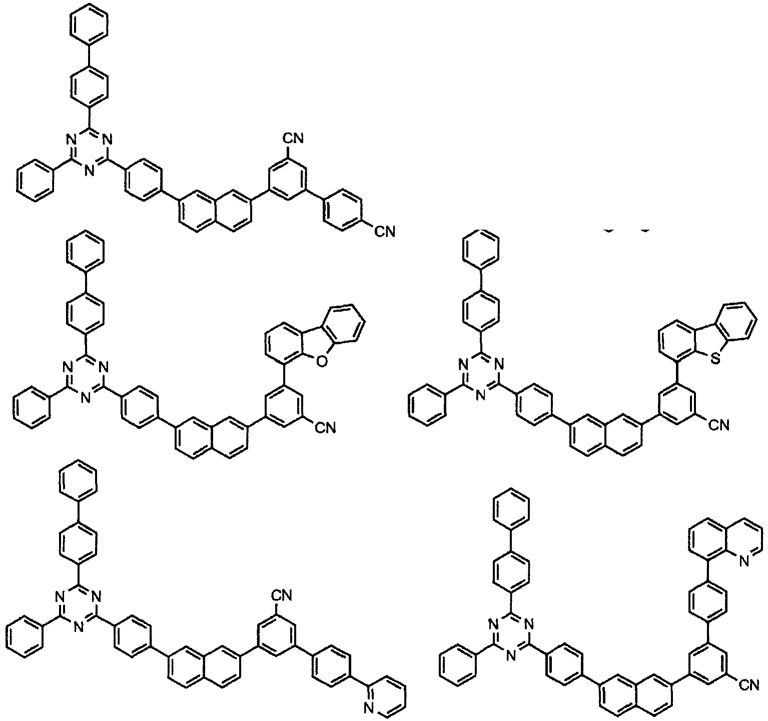

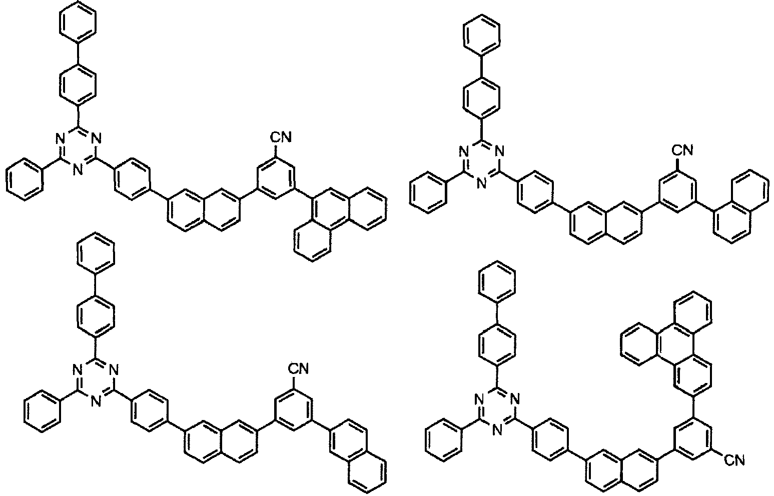

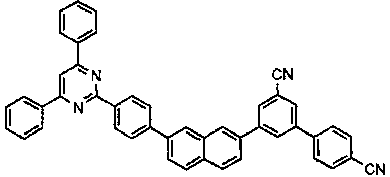

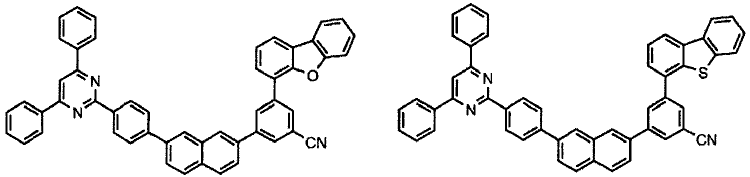

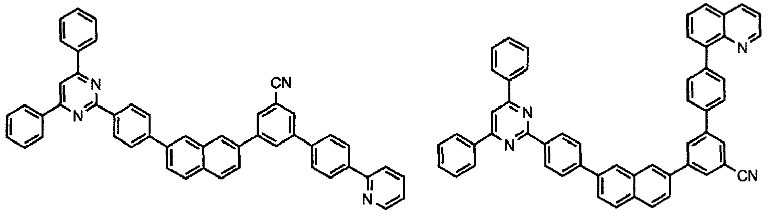

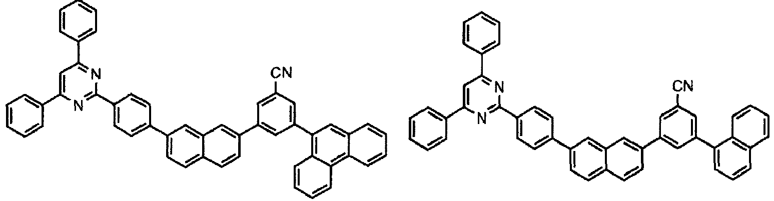

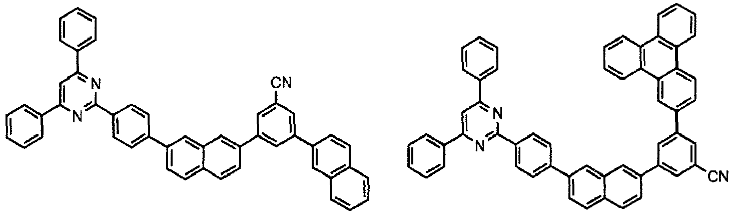

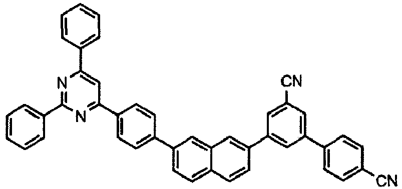

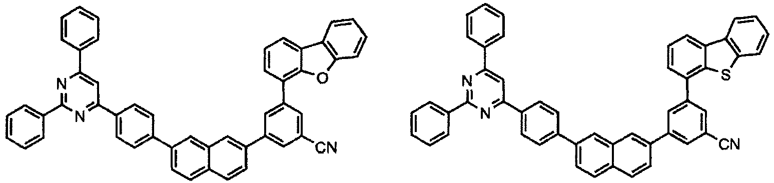

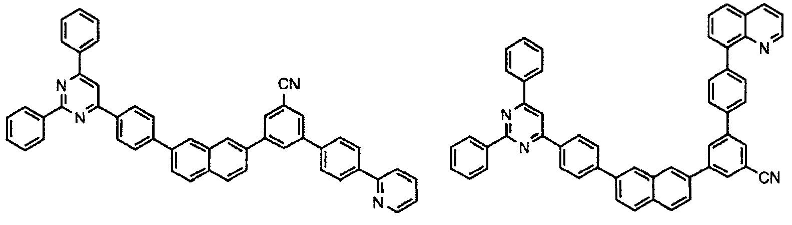

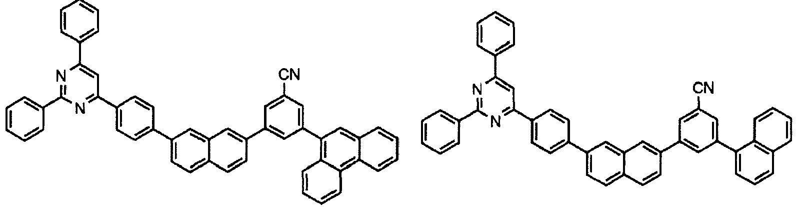

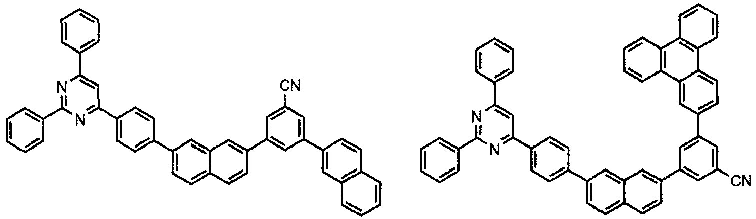

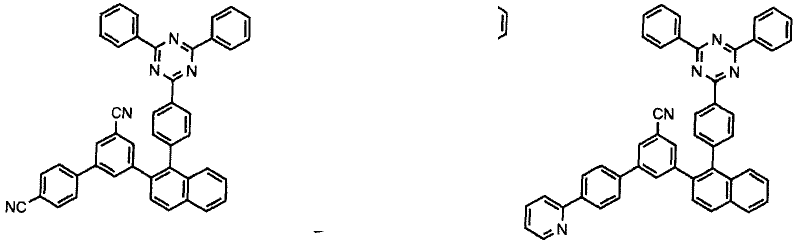

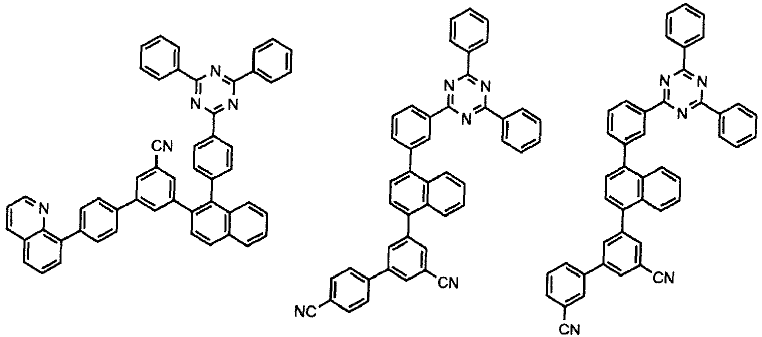

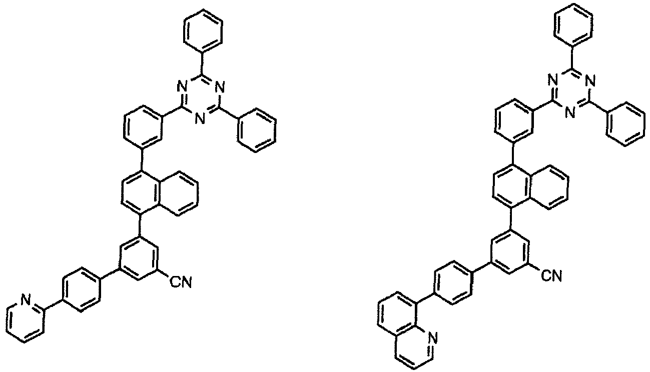

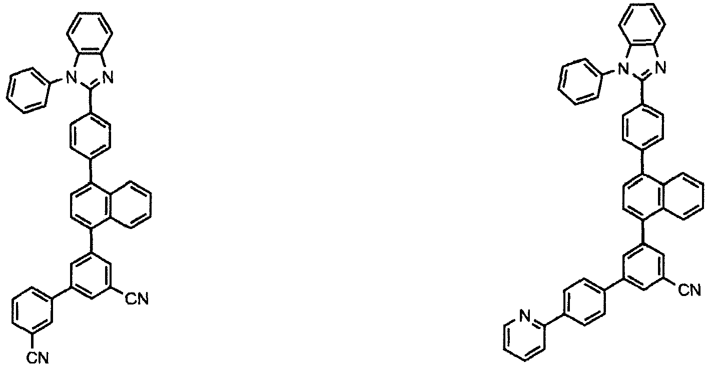

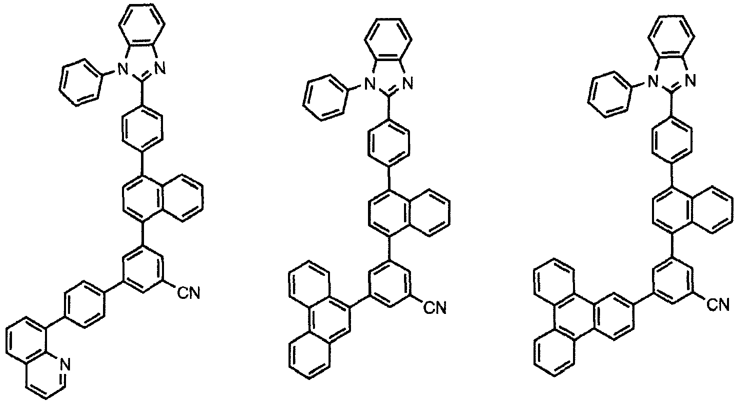

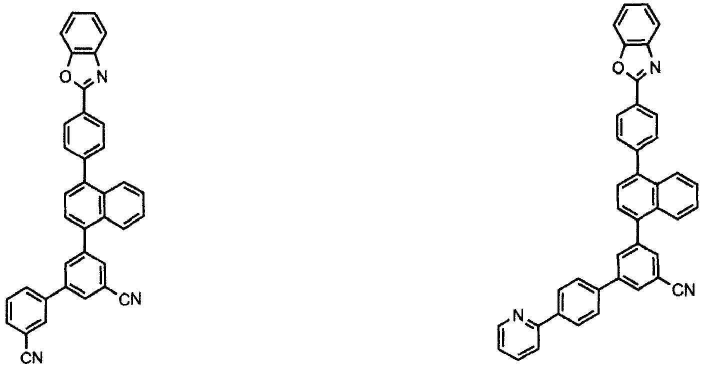

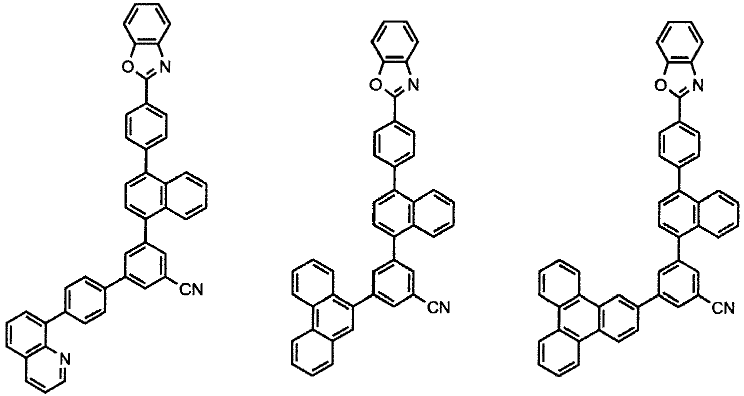

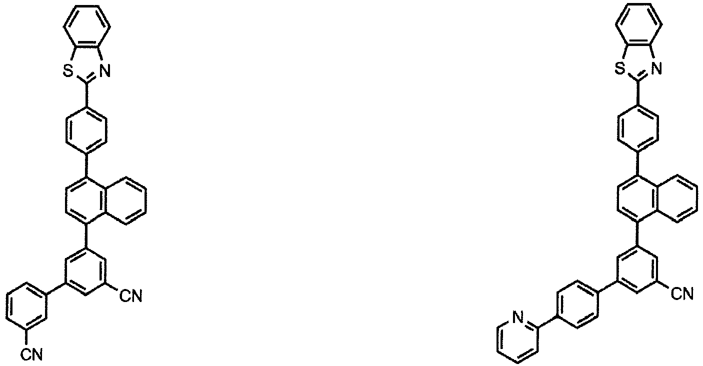

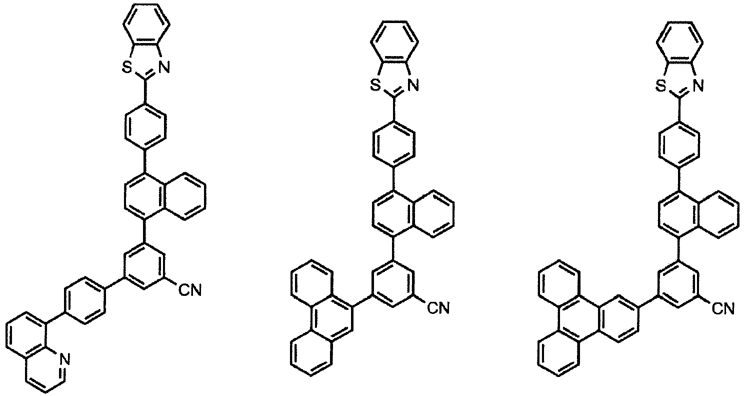

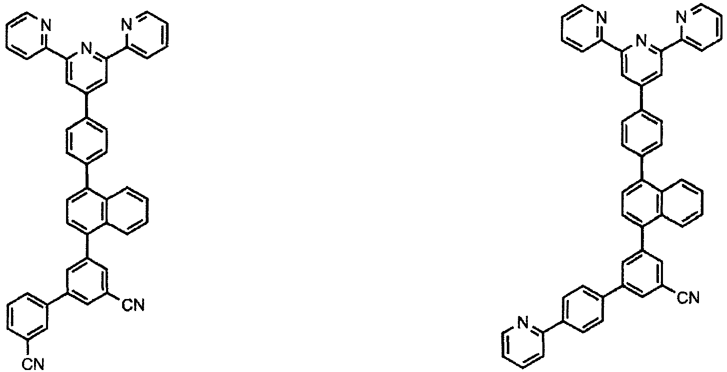

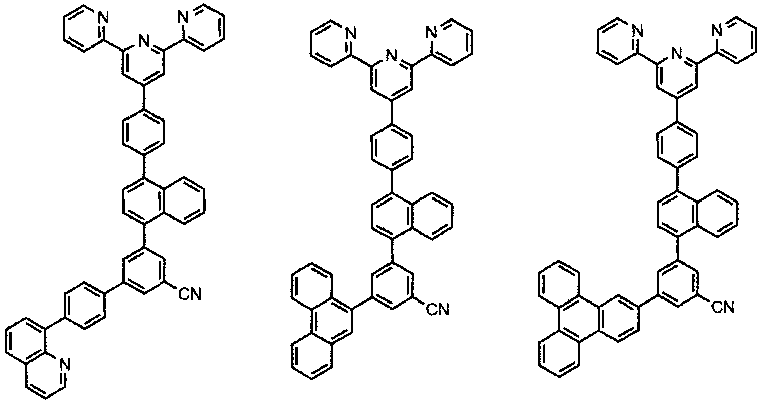

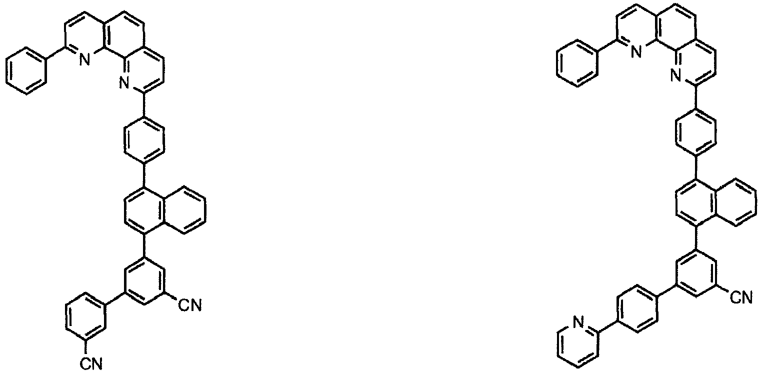

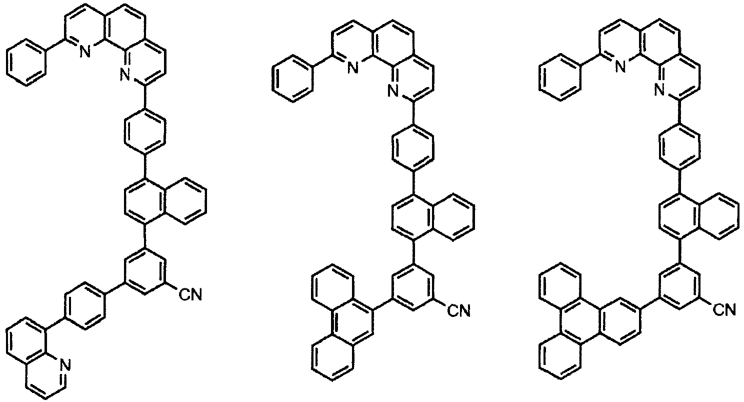

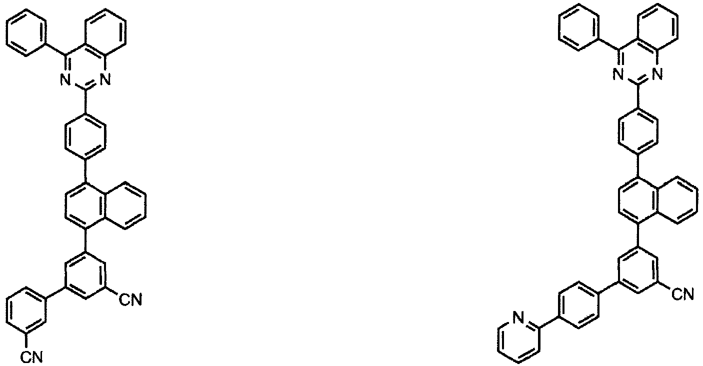

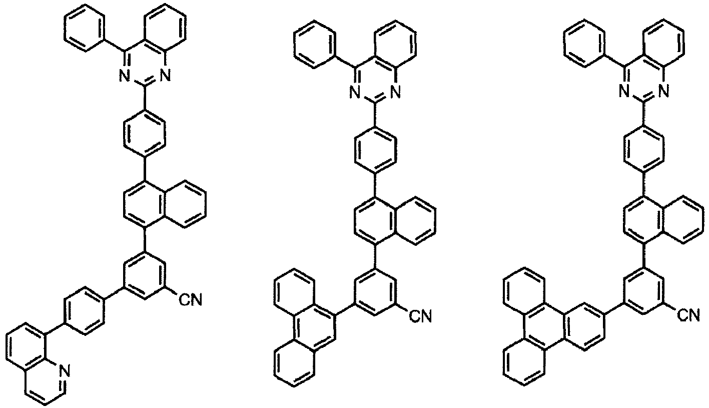

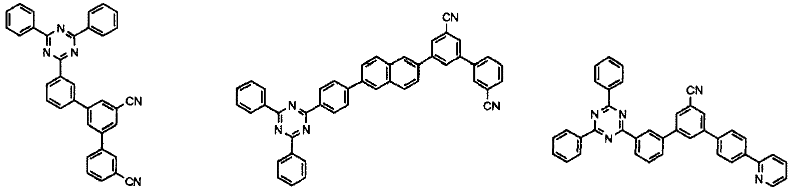

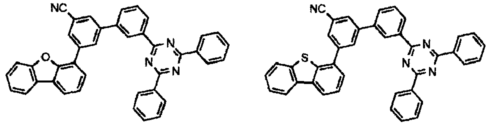

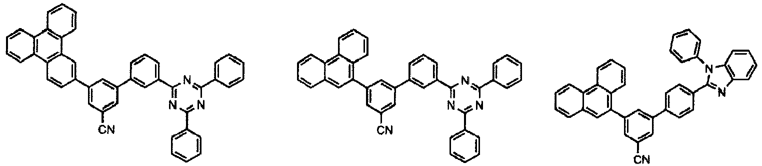

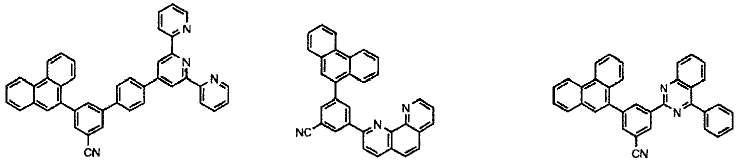

The compound represented by the Chemical Formula 1 may be prepared as in following Reaction Formula 1:

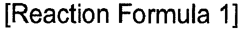

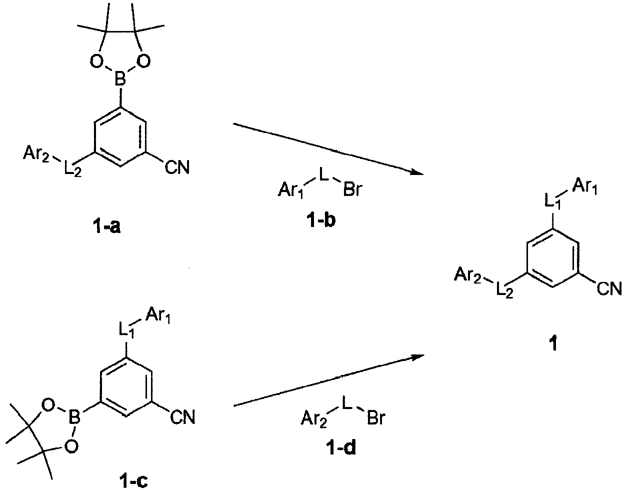

The reaction may be carried out by reacting the compound represented by the Chemical Formula 1-a with the compound represented by the Chemical Formula 1-b, or by reacting the compound represented by the Chemical Formula 1-c and the compound represented by the Chemical Formula 1-d to prepare the compound represented by the Chemical Formula 1. The reaction is a Suzuki coupling reaction, and preferably carried out in the presence of a palladium catalyst and a base. The functional group for the Suzuki coupling reaction may be changed as known in the art. The above preparation method may be further specified in the Preparation Examples described later.

Further, the present disclosure provides an organic light emitting device including the compound represented by the Chemical Formula 1. In one example, the present disclosure provides an organic light emitting device including a first electrode; a second electrode provided to face the first electrode; and one or more organic material layers provided between the first electrode and the second electrode, wherein one or more of the organic material layers include the compound of the Chemical Formula 1.

The organic material layer of the organic light emitting device of the present disclosure may have a single layer structure, or a multilayered structure in which two or more organic material layers are laminated. For example, the organic light emitting device of the present disclosure may have a structure including a hole injection layer, a hole transport layer, a light emitting layer, an electron transport layer, an electron injection layer, and the like as the organic material layer. However, the structure of the organic light emitting device is not limited thereto, but may include a smaller number of organic layers.

Moreover, the organic material layer may include a hole injection layer, a hole transport layer, or a layer that injects and transports holes simultaneously, and the hole injection layer, the hole transport layer, or the layer that injects and transports holes simultaneously includes the compound represented by the Chemical Formula 1.

Further, the organic material layer may include a light emitting layer, and the light emitting layer includes the compound represented by the Chemical Formula 1.

In addition, the organic material layer may include an electron transport layer, or an electron injection layer, and the electron transport layer, or the electron injection layer includes the compound represented by the Chemical Formula 1.

In addition, the organic material layer may include an electron transport layer, or an electron injection layer, and the electron transport layer, or the electron injection layer includes the compound represented by the Chemical Formula 1.

Further, the organic material layer includes a light emitting layer and an electron transport layer, and the electron transport layer may include the compound represented by the Chemical Formula 1.

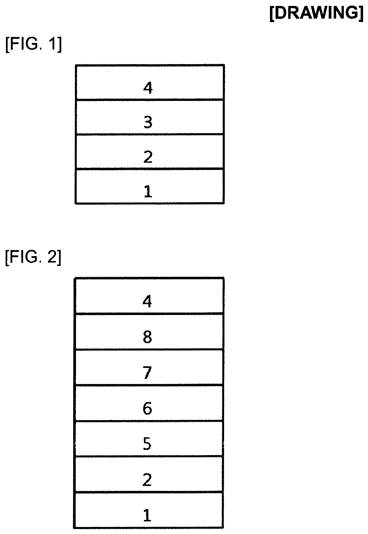

Further, the organic light emitting device according to the present disclosure may be an organic light emitting device having a structure (normal type) where an anode, one or more organic material layers, and a cathode are sequentially laminated on a substrate. Further, the organic light emitting device according to the present disclosure may be an organic light emitting device having an inverted direction structure (inverted type) where the cathode, one or more organic material layers, and the anode are sequentially laminated on the substrate. For example, the structure of the organic light emitting device according to the present disclosure is illustrated in FIGS. 1 and 2.

FIG. 1 illustrates an example of an organic light emitting device including a substrate (1), an anode (2), a light emitting layer (3), and a cathode (4). In such a structure, the compound represented by the Chemical Formula 1 may be included in the light emitting layer.

FIG. 2 illustrates an example of an organic light emitting device including a substrate (1), an anode (2), a hole injection layer (5), a hole transport layer (6), a light emitting layer (7), an electron transport layer (8), and a cathode (4). In such a structure, the compound represented by the Chemical Formula 1 may be included in one or more layers of the hole injection layer, the hole transport layer, the light emitting layer, and the electron transport layer.

The organic light emitting device according to the present disclosure may be prepared by using materials and methods known in the art, except that one or more of organic material layers include the compound represented by the Chemical Formula 1. Further, in the case where the organic light emitting device includes a plurality of organic material layers, the organic material layers may be formed of the same materials or different materials.

For example, the organic light emitting device according to the present disclosure may be prepared by sequentially laminating the first electrode, the organic material layer, and the second electrode on the substrate. In this case, the organic light emitting device may be prepared by depositing a metal, metal oxides having conductivity, or an alloy thereof on the substrate by using a PVD (physical vapor deposition) method such as a sputtering method or an e-beam evaporation method to form the anode, forming the organic material layer including the hole injection layer, the hole transport layer, the light emitting layer, and the electron transport layer thereon, and then depositing a material that can be used as the cathode thereon. In addition to such a method, the organic light emitting device may be prepared by sequentially depositing a cathode material, the organic material layer, and an anode material on the substrate.

Further, the compound represented by the Chemical Formula 1 may be formed as the organic material layer by a vacuum deposition method as well as a solution coating method during the production of the organic light emitting device. Herein, the solution coating method means spin coating, dip coating, doctor blading, inkjet printing, screen printing, a spray method, roll coating, or the like, but is not limited thereto.

In addition to such a method, the organic light emitting device may be prepared by sequentially depositing a cathode material, an organic material layer, and an anode material on a substrate (International Publication WO 2003/012890). However, the preparation method is not limited thereto.

In one example, the first electrode is the anode, and the second electrode is the cathode, and alternatively, the first electrode is the cathode, and the second electrode is the anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:AI or SnO₂:Sb; conductive polymers such as poly(3-methylthiophene), poly[3,4-(ethylene-1,2-dioxy)thiophene] (PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer injecting the holes from the electrode, and the hole injection material is preferably a compound which has an ability of transporting the holes, a hole injection effect in the anode and an excellent hole injection effect to the light emitting layer or the light emitting material, prevents movement of an exciton generated in the light emitting layer to the electron injection layer or the electron injection material, and has an excellent thin film forming ability. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer receiving the holes from the hole injection layer and transporting the holes to the light emitting layer, and the hole transport material is a material that can receive the holes from the anode or the hole injection layer and transport the holes to the light emitting layer, and a material having large mobility to the holes is suitable. Specific examples thereof include an arylamine-based organic material, a conductive polymer, a block copolymer in which a conjugate portion and a non-conjugate portion are present together, and the like, but are not limited thereto.

The light emitting material is a material that can receive the holes and the electrons from the hole transport layer and the electron transport layer, respectively, and bond the holes and the electrons to emit light in a visible ray region. The light emitting material is preferably a material having good quantum efficiency to fluorescence or phosphorescence. Specific examples thereof include a 8-hydroxy-quinoline aluminum complex (Alq₃); a carbazole-based compound; a dimerized styryl compound; BAlq; a 10-hydroxybenzoquinoline-metal compound; benzoxazole, benzthiazole, and benzimidazole-based compounds; a poly(p-phenylenevinylene) (PPV)-based polymer; a spiro compound; polyfluorene, lubrene, and the like, but are not limited thereto.

The light emitting layer may include a host material and a dopant material. Examples of the host material include a condensation aromatic cycle derivative, a heterocycle-containing compound, or the like. Specific examples of the compensation aromatic cycle derivative include an anthracene derivative, a pyrene derivative, a naphthalene derivative, a pentacene derivative, a phenanthrene compound, a fluoranthene compound, and the like, and specific examples of the heterocycle-containing compound include a carbazole derivative, a dibenzofuran derivative, a ladder-type furan compound, a pyrimidine derivative, and the like, but are not limited thereto.

Examples of the dopant material include an aromatic amine derivative, a styrylamine compound, a boron complex, a fluoranthene compound, a metal complex, and the like. Specifically, the aromatic amine derivative is a compensation aromatic cycle derivative having a substituted or unsubstituted arylamino group, examples thereof include pyrene, anthracene, chrysene, and periflanthene having the arylamino group, and the like, the styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

The electron transport material is a layer receiving the electrons from the electron injection layer and transporting the electrons to the light emitting layer. For the electron transport material, a material having large mobility to the electrons, which can receive the electrons well from the cathode and transport the electrons to the light emitting layer, is suitable. Specific examples thereof include an 8-hydroxyquinoline Al complex; a complex including Alq₃; an organic radical compound; a hydroxyflavone-metal complex, and the like, but are not limited thereto. The electron transport layer may be used together with a predetermined desired cathode material as used according to the prior art. Particularly, an example of an appropriate cathode material is a general material having the low work function and followed by an aluminum layer or a silver layer. Specific examples thereof include cesium, barium, calcium, ytterbium, and samarium, and each case is followed by the aluminum layer or the silver layer.

The electron injection layer is a layer injecting the electrons from the electrode, and a compound which has an ability of transporting the electrons, an electron injection effect from the cathode, and an excellent electron injection effect to the light emitting layer or the light emitting material, prevents movement of an exciton generated in the light emitting layer to the hole injection layer, and has an excellent thin film forming ability is preferable. Specific examples thereof include fluorenone, anthraquinodimethane, diphenoquinone, thiopyran dioxide, oxazole, oxadiazole, triazole, imidazole, perylene tetracarboxylic acid, fluorenylidene methane, anthrone, and the like, and derivatives thereof, a metal complex compound, a nitrogen-containing 5-membered cycle derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present disclosure may be a front side emission type, a back side emission type, or a double side emission type according to the used material.

Further, the compound represented by the Chemical Formula 1 may be included in an organic solar cell or an organic transistor in addition to the organic light emitting device.

The preparation of the compound represented by the Chemical Formula 1 and the organic light emitting device including the same will be described in detail in the following examples. However, these examples are presented for illustrative purposes only, and the scope of the present invention is not limited thereto.

### Preparation Example 1: Preparation of Compound A2

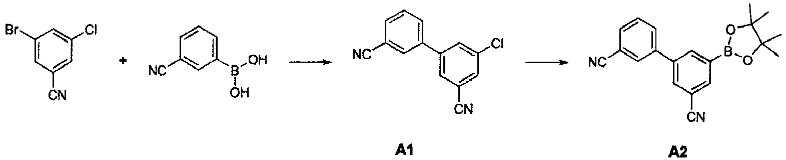

### 1) Preparation of Compound A1

3-bromo-5-chlorobenzonitrile (30 g, 139 mmol), and (3-cyanophenyl)boronic acid (22 g, 152 mmol) were dissolved in 300 mL of tetrahydrofuran. 2 M solution of sodium carbonate (Na₂CO₃) (210 mL), and tetrakis(triphenylphosphine)palladium(0) (2 g, 1 mmol%) were added thereto, and refluxed for 12 hours. After the reaction was completed, the mixture was cooled down to room temperature, and the resulting mixture was extracted three times with water and toluene. The toluene layer was separated, dried with magnesium sulfate, and the filtrate was distilled under reduced pressure. The resulting mixture was recrystallized three times using chloroform and ethanol to prepare Compound A1 (20 g, yield 61%; MS:[M+H]⁺=239).

### 2) Preparation of Compound A2

Compound A1 (20 g, 84 mmol), bis(pinacolato)diboron (23 g, 92 mmol), potassium acetate (25 g, 251 mmol), dibenzylideneacetonepalladium (1.4 g, 2.5 mmol), and tricyclohexylphosphine (1.4 g, 5.0 mmol) were added to dioxane (300 mL), and refluxed for 12 hours. After the reaction was completed, the reaction mixture was cooled down to room temperature and distilled under reduced pressure to remove the solvent. And then it was dissolved in chloroform, and washed three times with water. The organic layer was separated and dried with magnesium sulfate. And then it was distilled under reduced pressure to prepare Compound A2 (22 g, yield 78%; MS:[M+H]⁺=331).

### Preparation Example 2: Preparation of Compound B2

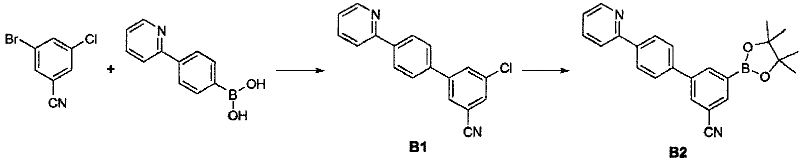

### 1) Preparation of Compound B1

Compound B1 (28 g, yield 70%; MS:[M+H]⁺=291) was prepared in the same manner as in the Preparation Example 1 of Compound A1, except that (4-(pyridin-2-yl)phenyl)boronic acid (30 g, 152 mmol) was used instead of (3-cyanophenyl)boronic acid (22 g, 152 mmol).

### 2) Preparation of Compound B2

Compound B2 (20 g, yield 70%; MS:[M+H]⁺=382) was prepared in the same manner as in the Preparation Example 1 of Compound A2, except that Compound B1 (20 g, 69 mmol) was used instead of Compound A1.

### Preparation Example 3: Preparation of Compound C2

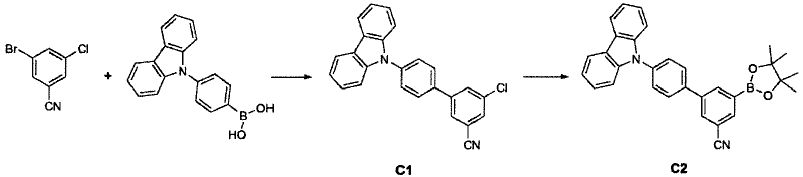

### 1) Preparation of Compound C1

Compound C1 (32 g, yield 61%; MS:[M+H]⁺=379) was prepared in the same manner as in the Preparation Example 1 of Compound A1, except that (4-(9H-carbazol-9-yl)phenyl)boronic acid (44 g, 152 mmol) was used instead of (3-cyanophenyl)boronic acid (22 g, 152 mmol).

### 2) Preparation of Compound C2

Compound C2 (20 g, yield 81%; MS:[M+H]⁺=471) was prepared in the same manner as in the Preparation Example 1 of Compound A2, except that Compound C1 (20 g, 53 mmol) was used instead of Compound A1.

### Preparation Example 4: Preparation of Compound D2

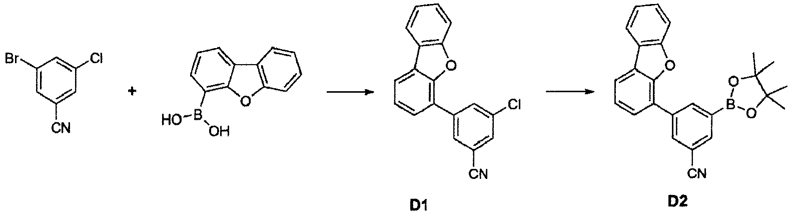

### 1) Preparation of Compound D1

Compound D1 (27 g, yield 65%; MS:[M+H]⁺=304) was prepared in the same manner as in the Preparation Example 1 of Compound A1, except that dibenzo[b,d]furan-4-ylboronic acid (32 g, 152 mmol) was used instead of (3-cyanophenyl)boronic acid (22 g, 152 mmol).

### 2) Preparation of Compound D2

Compound D2 (20 g, yield 81%; MS:[M+H]⁺=471) was prepared in the same manner as in the Preparation Example 1 of Compound A2, except that Compound D1 (20 g, 53 mmol) was used instead of Compound A1.

### Preparation Example 5: Preparation of Compound E2

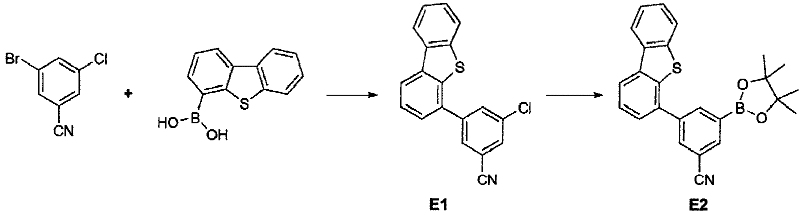

### 1) Preparation of Compound E1

Compound E1 (35 g, yield 80%; MS:[M+H]⁺=320) was prepared in the same manner as in the Preparation Example 1 of Compound A1, except that dibenzo[b,d]thiophen-4-ylboronic acid (35 g, 152 mmol) was used instead of (3-cyanophenyl)boronic acid (22 g, 152 mmol).

### 2) Preparation of Compound E2

Compound E2 (20 g, yield 78%; MS:[M+H]⁺=411) was prepared in the same manner as in the Preparation Example 1 of Compound A2, except that Compound E1 (20 g, 53 mmol) was used instead of Compound A1.

### Preparation Example 6: Preparation of Compound F2

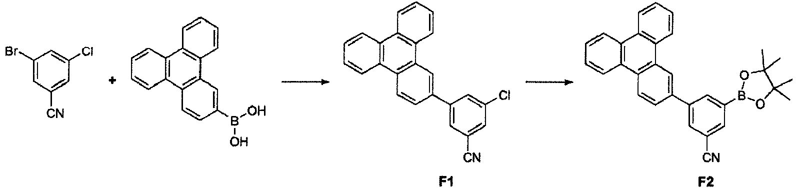

### 1) Preparation of Compound F1

Compound F1 (39 g, yield 77%; MS:[M+H]⁺=364) was prepared in the same manner as in the Preparation Example 1 of Compound A1, except that triphenylene-2-ylboronic acid (42 g, 152 mmol) was used instead of (3-cyanophenyl)boronic acid (22 g, 152 mmol).

### 2) Preparation of Compound F2

Compound F2 (20 g, yield 79%; MS:[M+H]⁺=456) was prepared in the same manner as in the Preparation Example 1 of Compound A2, except that Compound F1 (20 g, 55 mmol) was used instead of Compound A1.

### Preparation Example 7: Preparation of Compound G2

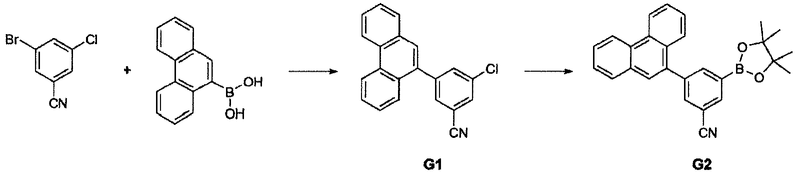

### 1) Preparation of Compound G1

Compound G1 (30 g, yield 70%; MS:[M+H]⁺=314) was prepared in the same manner as in the Preparation Example 1 of Compound A1, except that phenanthrene-9-ylboronic acid (34 g, 152 mmol) was used instead of (3-cyanophenyl)boronic acid (22 g, 152 mmol).

### 2) Preparation of Compound G2

Compound G2 (17 g, yield 65%; MS:[M+H]⁺=406) was prepared in the same manner as in the Preparation Example 1 of Compound A2, except that Compound G1 (20 g, 55 mmol) was used instead of Compound A1.

### Preparation Example 8: Preparation of Compound sub 1

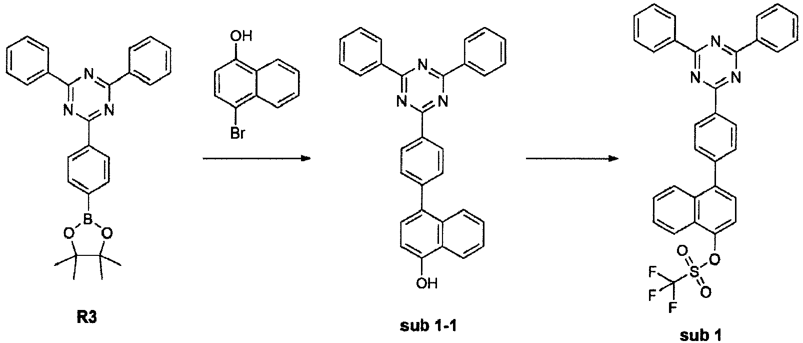

### 1) Preparation of Compound sub 1-1

Compound R3 (50 g, 114 mmol), 4-bromonaphthalen-1-ol (21 g, 114 mmol) were added to 300 mL of tetrahydrofuran, and the mixture was stirred and refluxed. Potassium carbonate (48 g, 345 mmol) was dissolved in 100 mL of water, and then it was added to the mixture. After the mixture was sufficiently stirred, tetrakistriphenyl-phosphinepalladium (4 g, 3 mmol) was added thereto. After 12 hours of reaction, the temperature was cooled down to room temperature, and filtered. The filtrate was extracted with chloroform and water, and then the organic layer was dried with magnesium sulfate. The organic layer was then distilled under reduced pressure and recrystallized using ethanol. The resulting solid was filtered and dried to prepare Compound sub 1-1 (41 g, yield 80%, MS:[M+H]⁺=452).

### 2) Preparation of Compound sub 1

Compound sub 1-1 (30 g, 66 mmol) was added to 300 mL of acetonitrile, and stirred. Potassium carbonate (18 g, 133 mmol) was mixed with 50 mL of water, and added thereto. Then, 1-butanesulfonyl fluoride (15 g, 100 mmol) was added slowly while heating with a water bath. After about 1 hour of reaction, it was terminated. After the temperature was cooled down to room temperature, the water layer and the organic layer were separated, and the organic layer was distilled under reduced pressure to prepare Compound sub 1 (34 g, yield 90%, MS:[M+H]⁺=584).

### Preparation Example 9: Preparation of Compound sub 2

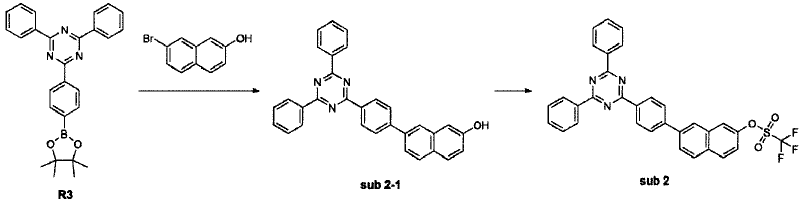

### 1) Preparation of Compound sub 2-1

Compound sub 2-1 (39 g, yield 75%; MS:[M+H]⁺=452) was prepared in the same manner as in the Preparation Example 8 of Compound sub 1-1, except that 7-bromonaphthalen-2-ol (34 g, 152 mmol) was used instead of 4-bromonaphthalen-1-ol (22 g, 152 mmol).

### 2) Preparation of Compound sub 2

Compound sub 2 (36 g, yield 92%; MS:[M+H]⁺=584) was prepared in the same manner as in the Preparation Example 8 of Compound sub 1, except that Compound sub 2-1 (30 g, 66 mmol) was used instead of Compound sub 1-1 (30g, 66 mmol).

### Preparation Example 10: Preparation of Compound sub 3

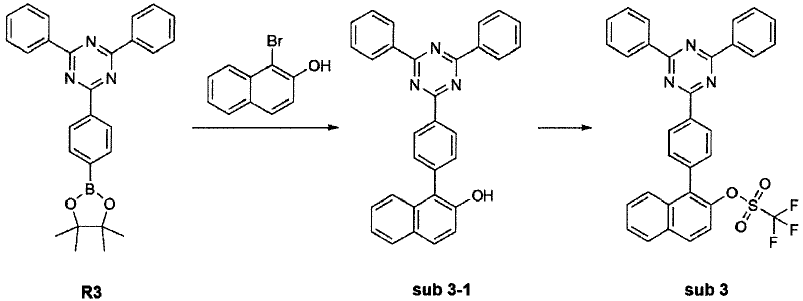

### 1) Preparation of Compound sub 3-1

Compound sub 3-1 (31 g, yield 61%; MS:[M+H]⁺=452) was prepared in the same manner as in the Preparation Example 8 of Compound sub 1-1, except that 1-bromonaphthalen-2-ol (34 g, 152 mmol) was used instead of 4-bromonaphthalen-1-ol (22 g, 152 mmol).

### 2) Preparation of Compound sub 3

Compound sub 3 (34 g, yield 88%; MS:[M+H]⁺=584) was prepared in the same manner as in the Preparation Example 8 of Compound sub 1, except that Compound sub 3-1 (30 g, 66 mmol) was used instead of Compound sub 1-1 (30g, 66 mmol).

### Preparation Example 11: Preparation of Compound sub 4

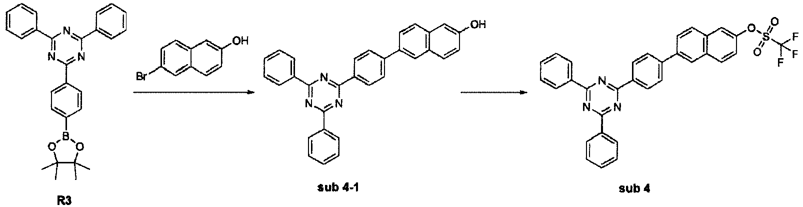

### 1) Preparation of Compound sub 4-1

Compound sub 4-1 (38 g, yield 73%; MS:[M+H]⁺=452) was prepared in the same manner as in the Preparation Example 8 of Compound sub 1-1, except that 6-bromonaphthalen-2-ol (34 g, 152 mmol) was used instead of 4-bromonaphthalen-1-ol (22 g, 152 mmol).

### 2) Preparation of Compound sub 4

Compound sub 4 (34 g, yield 88%; MS:[M+H]⁺=584) was prepared in the same manner as in the Preparation Example 8 of Compound sub 1, except that Compound sub 4-1 (30 g, 66 mmol) was used instead of Compound sub 1-1 (30g, 66 mmol).

### Preparation Example 12: Preparation of Compound sub 5

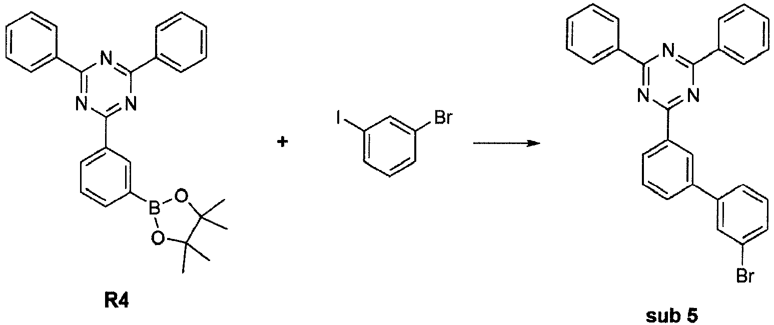

Compound R4 (30 g, 69 mmol), 1-bromo-3-iodobenzene (20 g, 69 mmol) were added to 300 mL of tetrahydrofuran, and the mixture was stirred and refluxed. Potassium carbonate (29 g, 206 mmol) was dissolved in 90 mL of water, and then it was added to the mixture. After the mixture was sufficiently stirred, tetrakistriphenyl-phosphinepalladium (2 g, 2 mmol) was added thereto. After 12 hours of reaction, the temperature was cooled down to room temperature, and filtered. The filtrate was extracted with chloroform and water, and then the organic layer was dried with magnesium sulfate. The organic layer was then distilled under reduced pressure and recrystallized using ethanol. The resulting solid was filtered and dried to prepare Compound sub 5 (20 g, yield 63%, MS:[M+H]⁺=464).

### Example 1: Preparation of Compound 1

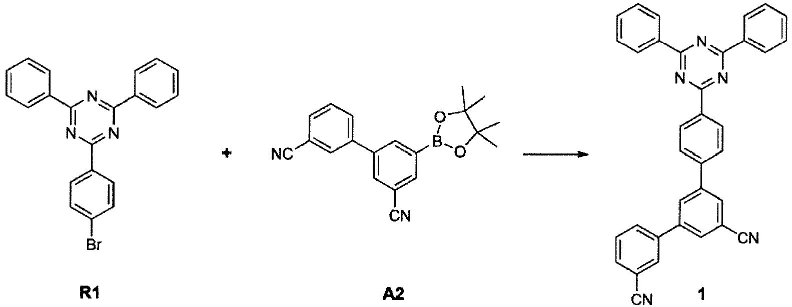

After the Compound R1 (20 g, 51 mmol) and Compound A2 (17 g, 51 mmol) were dispersed in tetrahydrofuran (150 mL), 2M aqueous potassium carbonate solution (aq. K₂CO₃)(77 mL) was added thereto, and then tetrakistriphenylphosphinepalladium [Pd(PPh₃)₄] (1 g, 1 mol%) was added thereto, followed by stirring and refluxing for 12 hours. The temperature was cooled down to room temperature and the resulting solid was filtered. The filtered solid was recrystallized using tetrahydrofuran and ethyl acetate, filtered and then dried to prepare Compound 1 (15 g, yield 60%; MS:[M+H]⁺=512).

### Example 2: Preparation of Compound 2

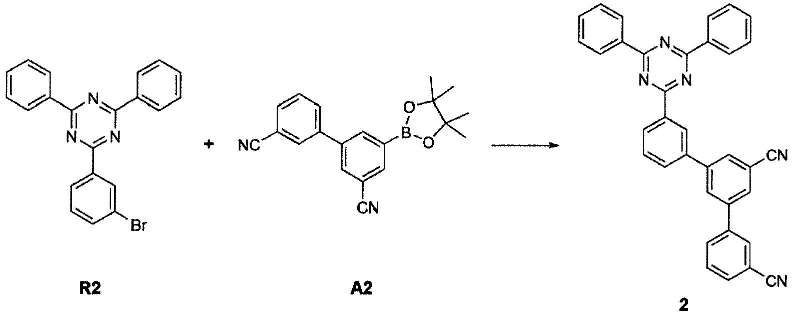

Compound 2 (14 g, yield 55%; MS:[M+H]⁺=512) was prepared in the same manner as in the Example 1 of Compound 1, except that Compound R2 (20 g, 51 mmol) was used instead of Compound R1.

### Example 3: Preparation of Compound 3

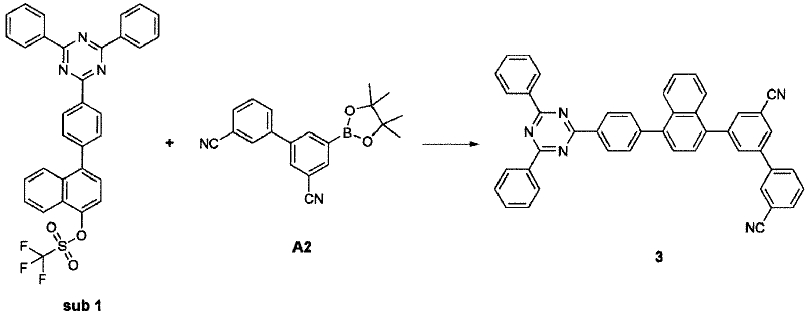

Compound 3 (20 g, yield 61%; MS:[M+H]⁺=638) was prepared in the same manner as in the Example 1 of Compound 1, except that Compound sub 1 (30 g, 51 mmol) was used instead of Compound R1.

### Example 4: Preparation of Compound 4

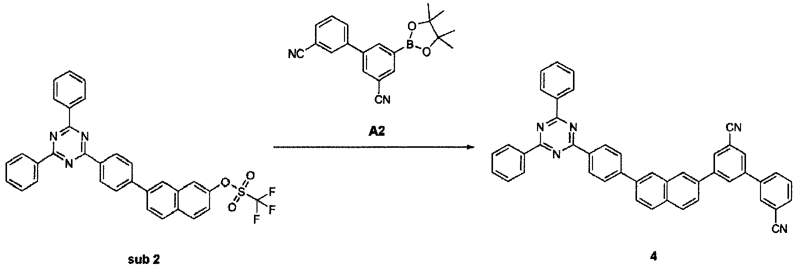

Compound 4 (23 g, yield 71%; MS:[M+H]⁺=638) was prepared in the same manner as in the Example 1 of Compound 1, except that Compound sub 2 (30 g, 51 mmol) was used instead of Compound R1.

### Example 5: Preparation of Compound 5

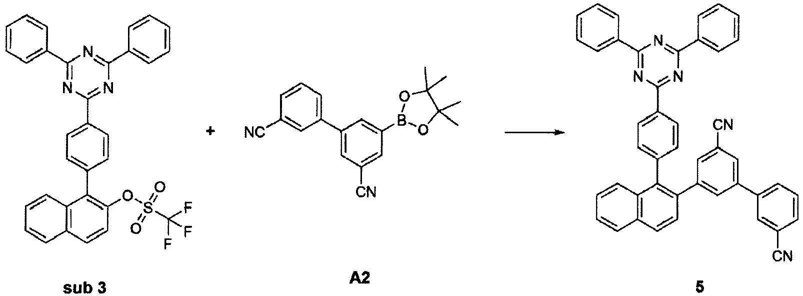

Compound 5 (15 g, yield 48%; MS:[M+H]⁺=638) was prepared in the same manner as in the Example 1 of Compound 1, except that Compound sub 3 (30 g, 51 mmol) was used instead of Compound R1.

### Example 6: Preparation of Compound 6

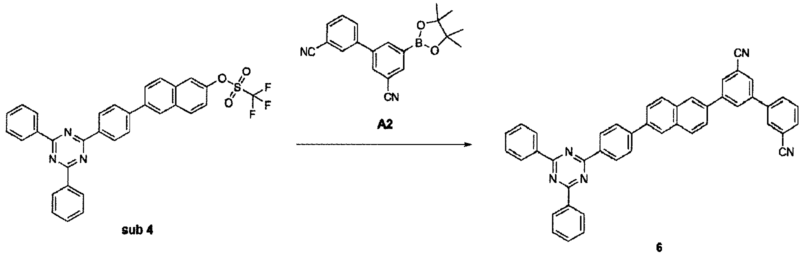

Compound 6 (25 g, yield 75%; MS:[M+H]⁺=638) was prepared in the same manner as in the Example 1 of Compound 1, except that Compound sub 4 (30 g, 51 mmol) was used instead of Compound R1.

### Example 7: Preparation of Compound 7

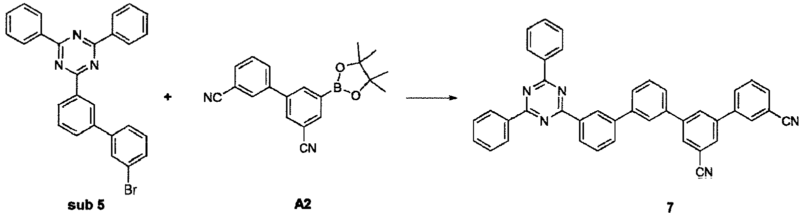

Compound 7 (11 g, yield 39%; MS:[M+H]⁺=588) was prepared in the same manner as in the Example 1 of Compound 1, except that Compound sub 5 (24 g, 51 mmol) was used instead of Compound R1.

### Example 8: Preparation of Compound 8

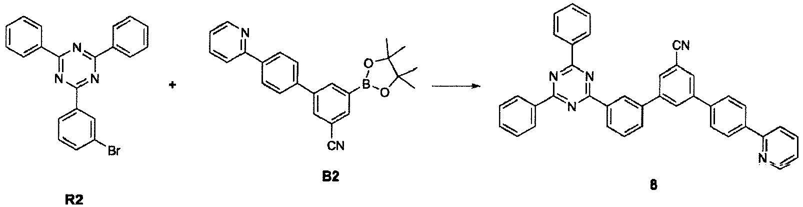

After Compound R2 (20 g, 51 mmol) and Compound B2 (20 g, 51 mmol) were dispersed in tetrahydrofuran (150 mL), 2M aqueous potassium carbonate solution (aq. K₂CO₃) (77 mL) was added thereto, and then tetrakistriphenylphosphinepalladium [Pd(PPh₃)₄] (1 g, 1 mol%) was added thereto, followed by stirring and refluxing for 12 hours. The temperature was cooled down to room temperature and the resulting solid was filtered. The filtered solid was recrystallized using tetrahydrofuran and ethyl acetate, filtered and then dried to prepare Compound 8 (13 g, yield 42%; MS:[M+H]⁺=588).

### Example 9: Preparation of Compound 9 (not forming part of the invention)

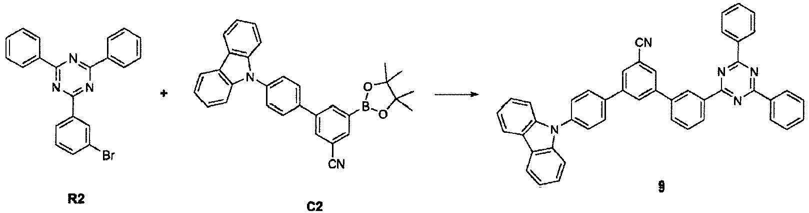

Compound 9 (18 g, yield 55%; MS:[M+H]⁺=652) was prepared in the same manner as in the Example 8 of Compound 8, except that Compound C2 (24 g, 51 mmol) was used instead of Compound B2.

### Example 10: Preparation of Compound 10

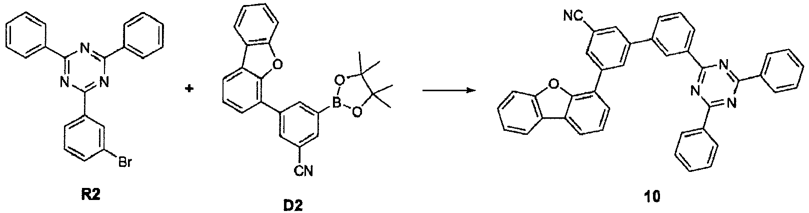

Compound 10 (18 g, yield 63%; MS:[M+H]⁺=577) was prepared in the same manner as in the Example 8 of Compound 8, except that Compound D2 (20 g, 51 mmol) was used instead of Compound B2.

### Example 11: Preparation of Compound 11

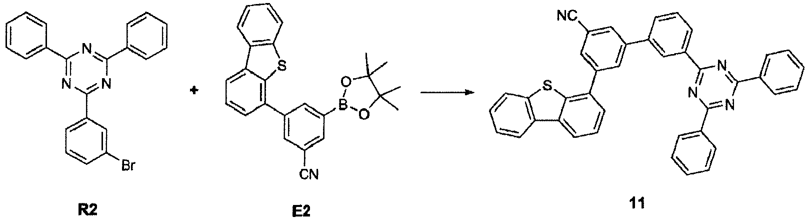

Compound 11 (18 g, yield 60%; MS:[M+H]⁺=592) was prepared in the same manner as in the Example 8 of Compound 8, except that Compound E2 (20 g, 51 mmol) was used instead of Compound B2.

### Example 12: Preparation of Compound 12

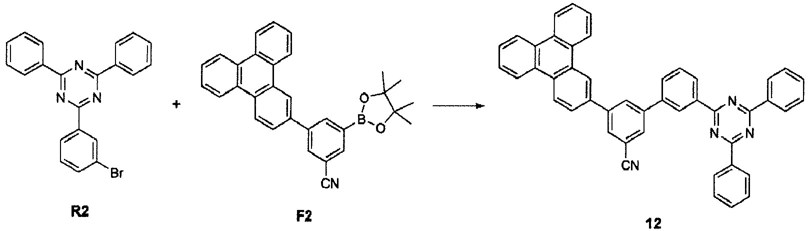

Compound 12 (23 g, yield 71%; MS:[M+H]⁺=636) was prepared in the same manner as in the Example 8 of Compound 8, except that Compound F2 (24 g, 51 mmol) was used instead of Compound B2.

### Example 13: Preparation of Compound 13

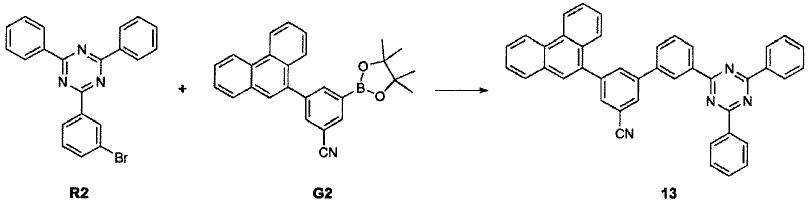

Compound 13 (19 g, yield 64%; MS:[M+H]⁺=587) was prepared in the same manner as in the Example 8 of Compound 8, except that Compound G2 (21 g, 51 mmol) was used instead of Compound B2.

### Example 14: Preparation of Compound 14

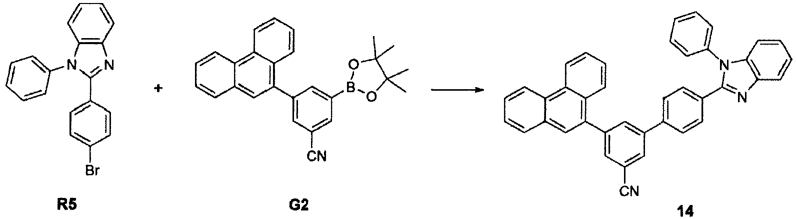

After Compound R5 (18 g, 51 mmol) and Compound G2 (21 g, 51 mmol) were dispersed in tetrahydrofuran (150 mL), 2M aqueous potassium carbonate solution (aq. K₂CO₃) (77 mL) was added thereto, and then tetrakistriphenylphosphinepalladium [Pd(PPh₃)₄] (1 g, 1 mol%) was added thereto, followed by stirring and refluxing for 12 hours. The temperature was cooled down to room temperature and the resulting solid was filtered. The filtered solid was recrystallized using tetrahydrofuran and ethyl acetate, filtered and then dried to prepare Compound 14 (11 g, yield 39%; MS:[M+H]⁺=587).

### Example 15: Preparation of Compound 15

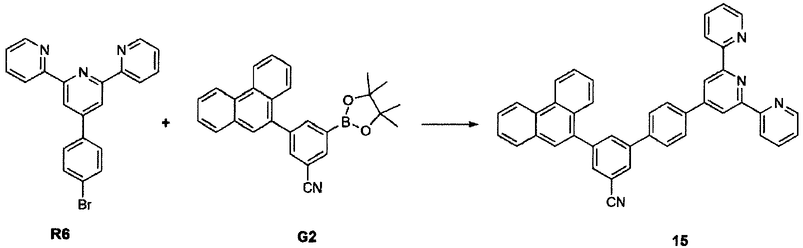

Compound 15 (25 g, yield 73%; MS:[M+H]⁺=663) was prepared in the same manner as in the Example 14 of Compound 14, except that Compound R6 (20 g, 51 mmol) was used instead of Compound R5.

### Example 16: Preparation of Compound 16

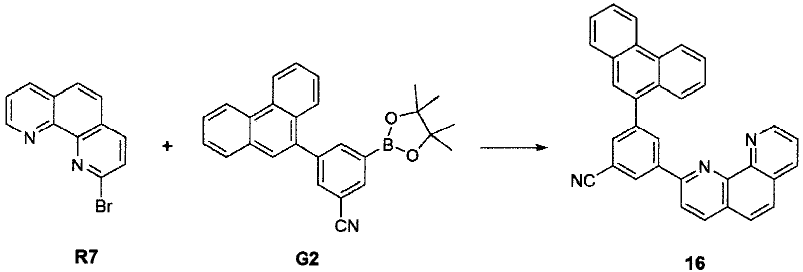

Compound 16 (16 g, yield 57%; MS:[M+H]⁺=534) was prepared in the same manner as in the Example 14 of Compound 14, except that Compound R7 (13 g, 51 mmol) was used instead of Compound R5.

### Example 17: Preparation of Compound 17

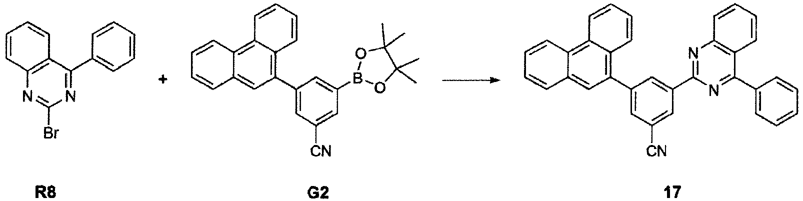

Compound 17 (18 g, yield 62%; MS:[M+H]⁺=560) was prepared in the same manner as in the Example 14 of Compound 14, except that Compound R8 (15 g, 51 mmol) was used instead of Compound R5.

### Experimental Example 1

The glass substrate (corning 7059 glass) on which a thin film of ITO (indium tin oxide) was applied in a thickness of 1,000 Å was put into distilled water having the detergent dissolved therein and washed by the ultrasonic wave. The used detergent was a product commercially available from Fisher Co. and the distilled water was one which had been twice filtered by using a filter commercially available from Millipore Co. After the ITO was washed for 30 minutes, washing with ultrasonic waves was repeated twice for 10 minutes by using distilled water. After the washing with distilled water was finished, washing with ultrasonic waves was performed in the order to isopropyl alcohol, acetone, and methanol solvent, and dried.

Following Compound HI-1 was thermally deposited under vacuum in a thicknesses of 500 Å on the ITO transparent electrode prepared above to form a hole injection layer. On the hole injection layer, following Compound HT-1 was deposited under vacuum in a thicknesses of 400 Å to form a hole transport layer, and a host H1 and a dopant compound D1 (2.5 wt%) were deposited under vacuum as a light emitting layer in a thickness of 300 Å. On the light emitting layer, following Compound ET-A was deposited under vacuum in a thicknesses of 50 Å to form an a-electron transport layer. Compound 1 prepared in Example 1 and LiQ (Lithium Quinolate) were deposited under vacuum at a weight ratio of 1: 1 on the a-electron transport layer to form an electron injection and transport layer having a thickness of 350 Å. Lithium fluoride (LiF) and aluminum were sequentially deposited in a thickness of 12 Å and 2,000Å respectively on the electron injection and transport layer to form a cathode.

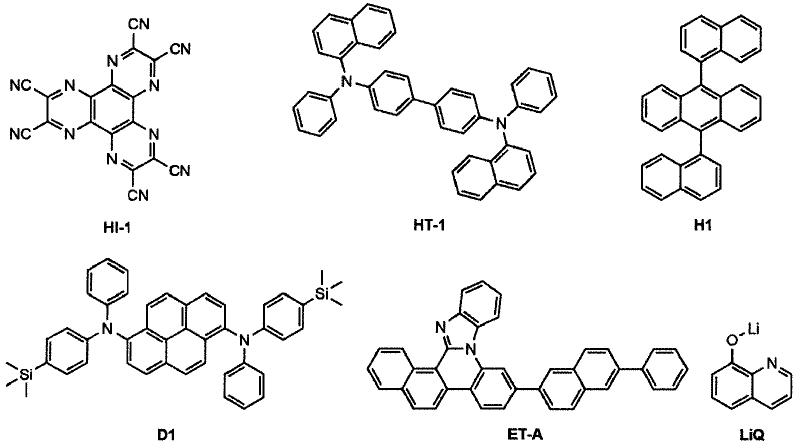

In the above process, the vapor deposition rate of the organic material was maintained at 0.4 to 0.7 Å/sec, the deposition rate of the lithium fluoride of the cathode was maintained at 0.3 Å/sec, the deposition rate of the aluminum was maintained at 2 Å/sec, and the degree of vacuum during vapor deposition was maintained at 2×10⁻⁷ to 5×10⁻⁶ torr to fabricate an organic light emitting device.

### Experimental Example 2 to 17

An organic light emitting device was prepared in the same manner as in Experimental Example 1, except that the compound described in Table 1 below was used instead of Compound 1 Experimental Example 9 does not form part of the invention.

### Comparative Experimental Example 1 to 3

An organic light emitting device was prepared in the same manner as in Experimental Example 1, except that the compound described in Table 1 below was used instead of Compound 1. In the following Table 1, Compound A, Compound B, and Compound C are as below:

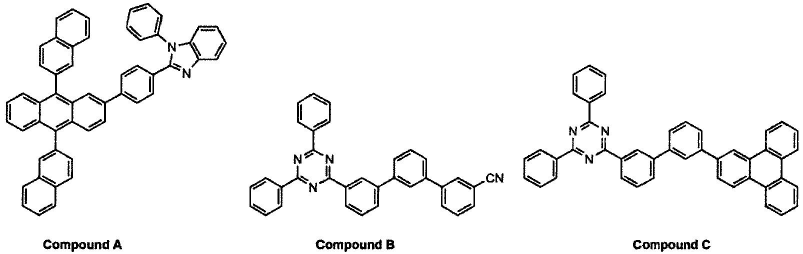

The driving voltage, the efficiency, the color coordinate, and the lifetime were measured by applying a current to the organic light emitting devices prepared in the Experimental Examples and Comparative Experimental Examples, and the results are shown in Table 1 below. The lifetime (LT₉₅) is defined as the time required for the luminance to decrease to 95% when the initial luminance is taken as 100%, and is measured at a current density of 50 mA/cm².

**[Table 1]**

| | Compound | Voltage (V) (@10 mA/cm²) | Efficiency (Cd/A) (@ 10 mA/cm²) | Color coordinate (x,y) | Life Time(h) T95 at 50 mA/cm² |
|---|---|---|---|---|---|
| Experimental Example 1 | Example 1 | 4.21 | 5.21 | 0.134, 0.133 | 340 |
| Experimental Example 2 | Example 2 | 4.15 | 5.32 | 0.133, 0.133 | 300 |
| Experimental Example 3 | Example 3 | 4.10 | 5.40 | 0.134, 0.132 | 258 |
| Experimental Example 4 | Example 4 | 4.12 | 5.30 | 0.134, 0.132 | 271 |
| Experimental Example 5 | Example 5 | 4.09 | 5.61 | 0.134, 0.132 | 240 |
| Experimental Example 6 | Example 6 | 4.18 | 5.31 | 0.134, 0.133 | 300 |
| Experimental Example 7 | Example 7 | 4.09 | 5.70 | 0.133, 0.133 | 261 |
| Experimental Example 8 | Example 8 | 4.18 | 5.74 | 0.134, 0.132 | 201 |
| Experimental Example 9 | Example 9 | 4.08 | 5.66 | 0.134, 0.132 | 188 |
| Experimental Example 10 | Example 10 | 4.07 | 6.32 | 0.134, 0.132 | 171 |
| Experimental Example 11 | Example 11 | 4.08 | 6.40 | 0.132, 0.126 | 156 |
| Experimental Example 12 | Example 12 | 4.07 | 6.29 | 0.132, 0.126 | 175 |
| Experimental Example 13 | Example 13 | 4.05 | 6.36 | 0.132, 0.126 | 157 |
| Experimental Example 14 | Example 14 | 3.88 | 5.91 | 0.134, 0.133 | 125 |
| Experimental Example 15 | Example 15 | 4.10 | 5.99 | 0.134, 0.133 | 151 |
| Experimental Example 16 | Example 16 | 3.70 | 6.21 | 0.134, 0.133 | 100 |
| Experimental Example 17 | Example 17 | 4.08 | 5.90 | 0.134, 0.133 | 90 |
| Comparative Experimental Example 1 | Compound A | 3.88 | 5.83 | 0.133, 0.128 | 60 |
| Comparative Experimental Example 2 | Compound B | 4.11 | 5.94 | 0.132, 0.127 | 90 |
| Comparative Experimental Example 3 | Compound C | 3.62 | 6.57 | 0.133, 0.127 | 33 |

As shown in Table 1, it was confirmed that when the compound of the present disclosure was used as an electron transport layer material, it exhibited excellent stability as compared with the case of using the compound of Comparative Examples 1 to 3.

**[DESCRIPTION OF SYMBOLS]**

| | |
|---|---|
| 1: substrate | 2: anode |
| 3: light emitting layer | 4: cathode |
| 5: hole injection layer | 6: hole transport layer |

## Claims

1. A compound represented by the following Chemical Formula 1: 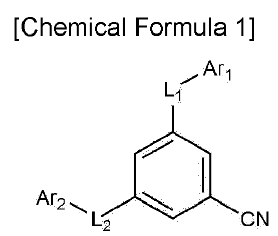 in Chemical Formula 1,
L₁ is a single bond, or substituted or unsubstituted C₆₋₆₀ arylene;
L₂ is a single bond, substituted or unsubstituted C₆₋₆₀ arylene, or substituted or unsubstituted C₂₋₆₀ heteroarylene containing at least one of O, N, Si and S;
Ar₁ is any one of the following structural formulae: 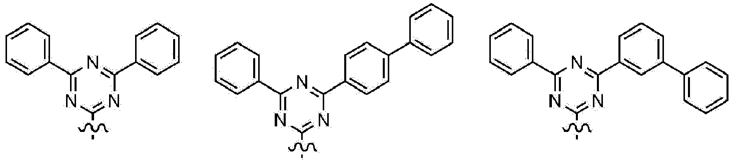 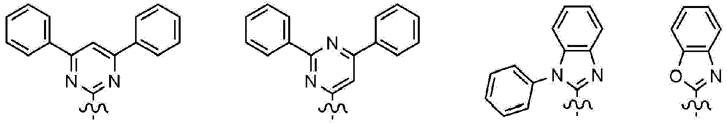 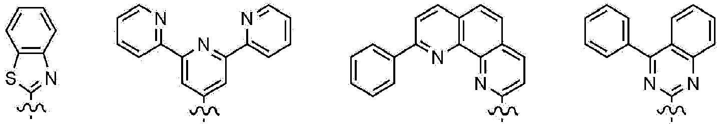
Ar₂ is any one of following structural formulae: 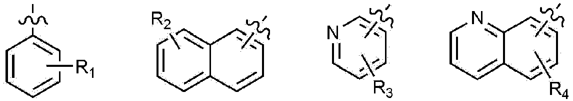 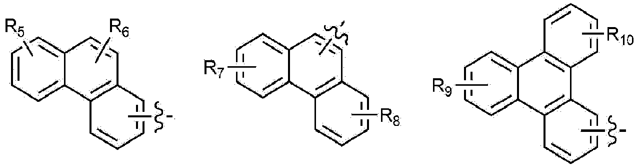 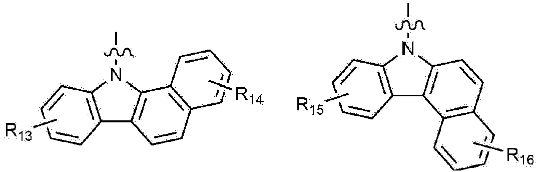 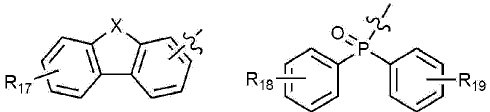
X is C(R'₂)(R'₃), Si(R'₄)(R'₅), S, or O;
R₁ is cyano, trifluoromethyl, or trifluoromethoxy; and
R'₂ to R'₅, R₂ to R₁₀, and R₁₃ to R₁₉ are each independently hydrogen deuterium; halogen, cyano, nitro, amino, substituted or unsubstituted C₁₋₆₀ alkyl, C₁₋₆₀ haloalkyl, C₁₋₆₀ haloalkoxy, substituted or unsubstituted C₃₋₆₀ cycloalkyl, substituted or unsubstituted C₂₋₆₀alkenyl, substituted or unsubstituted C₆₋₆₀ aryl, or a substituted or unsubstituted C₂₋₆₀ heterocyclic group containing at least one of O, N, Si and S.

2. The compound of claim 1, wherein L₁ is a single bond or any one of following structural formulae:

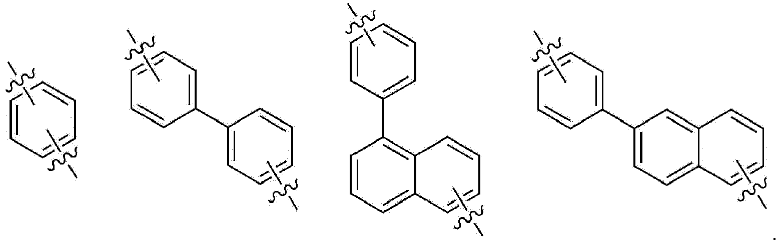

3. The compound of claim 1, wherein L₂ is a single bond, 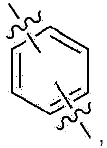 or

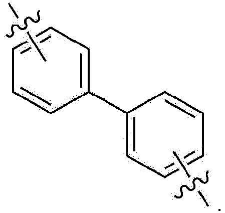

4. The compound of claim 1, wherein R₂ to R₁₀ and R₁₃ to R₁₉ are hydrogen.

5. The compound of claim 1, which is selected from the following compounds:

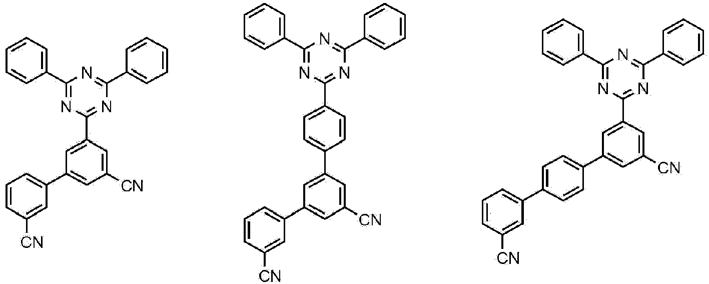

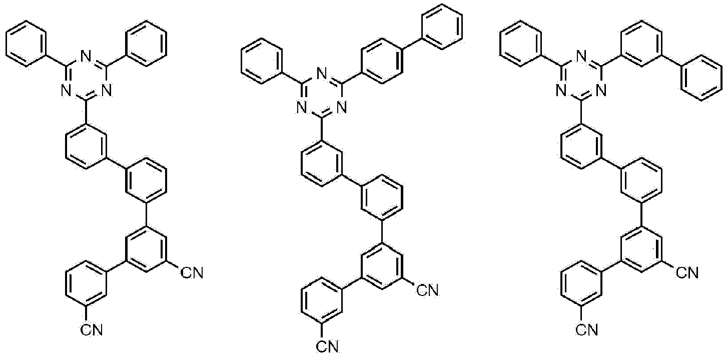

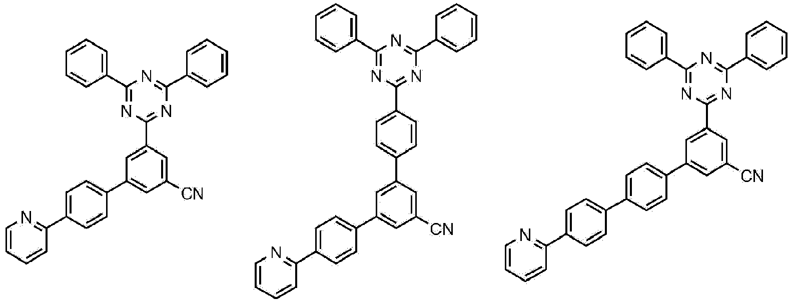

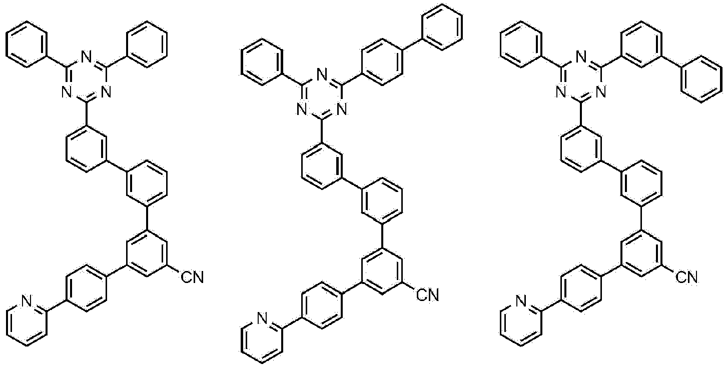

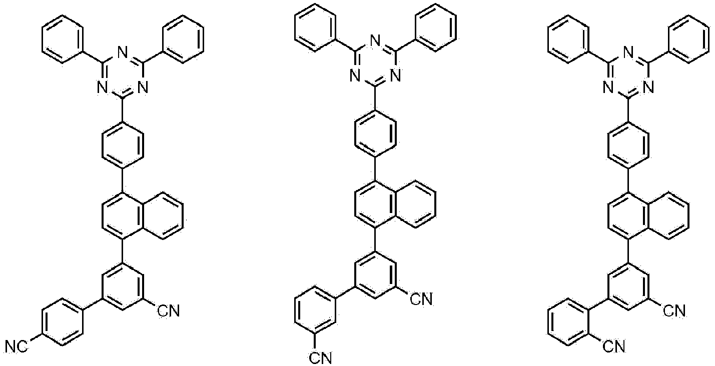

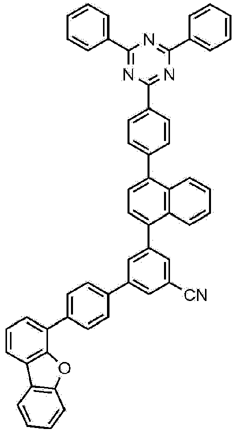

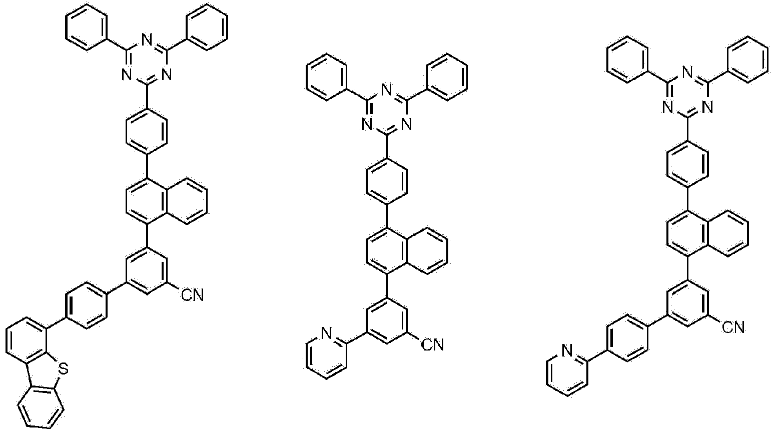

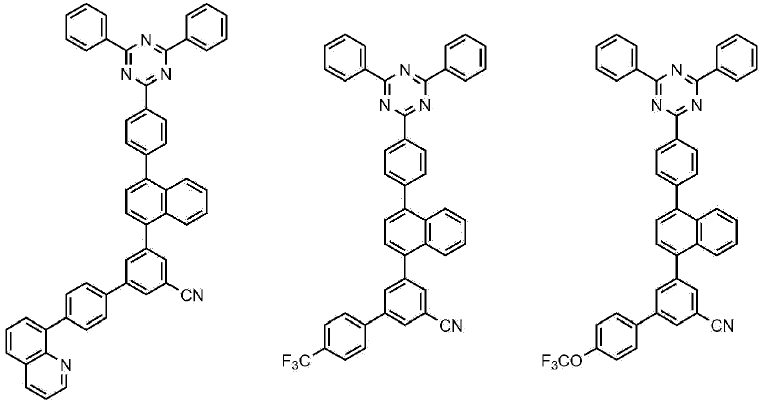

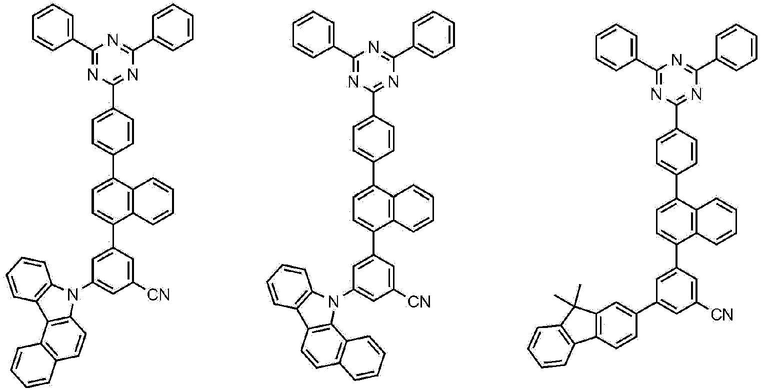

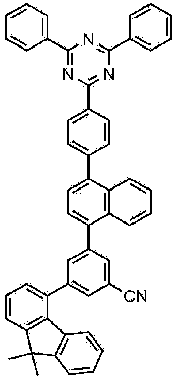

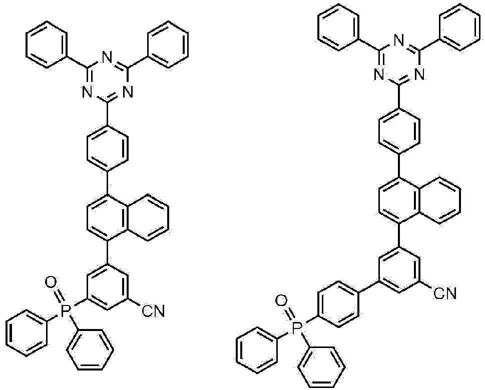

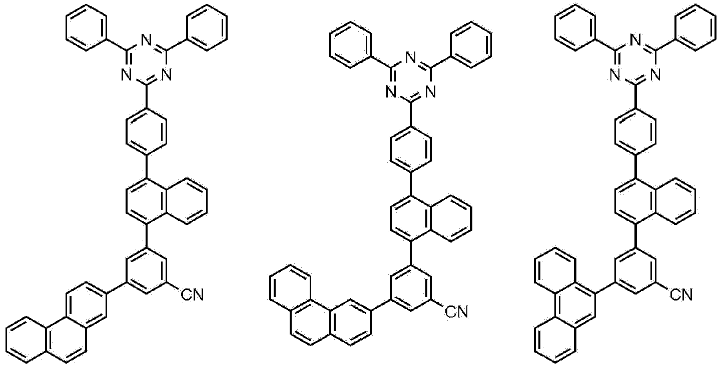

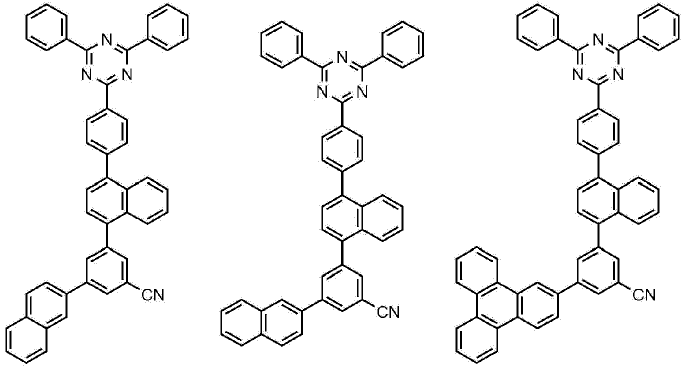

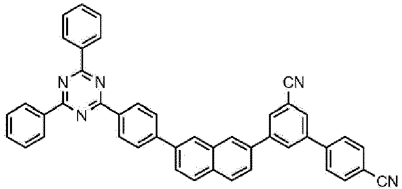

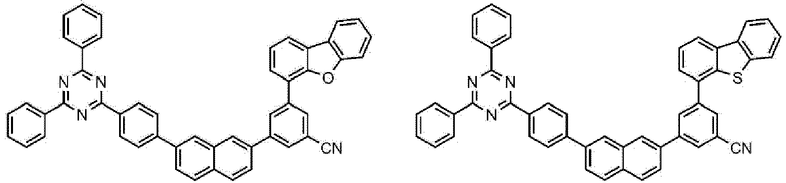

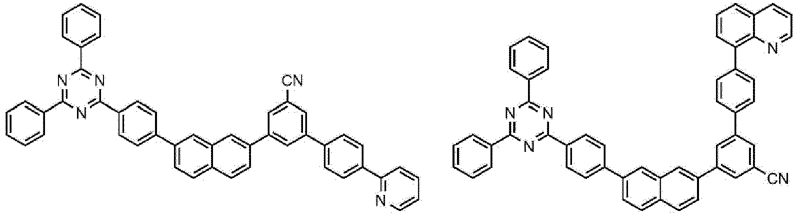

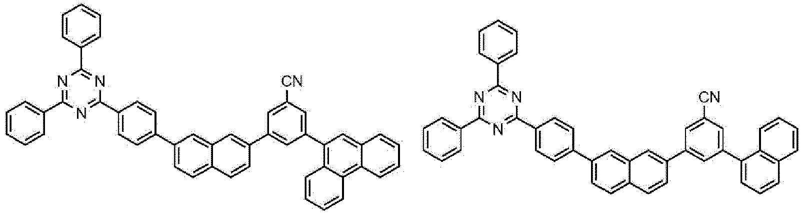

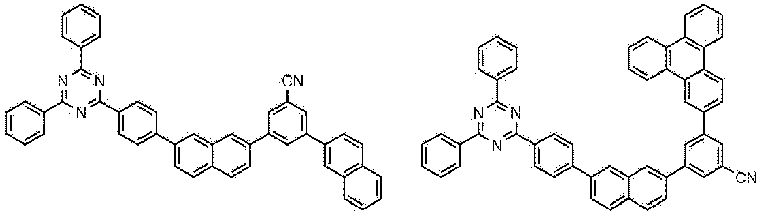

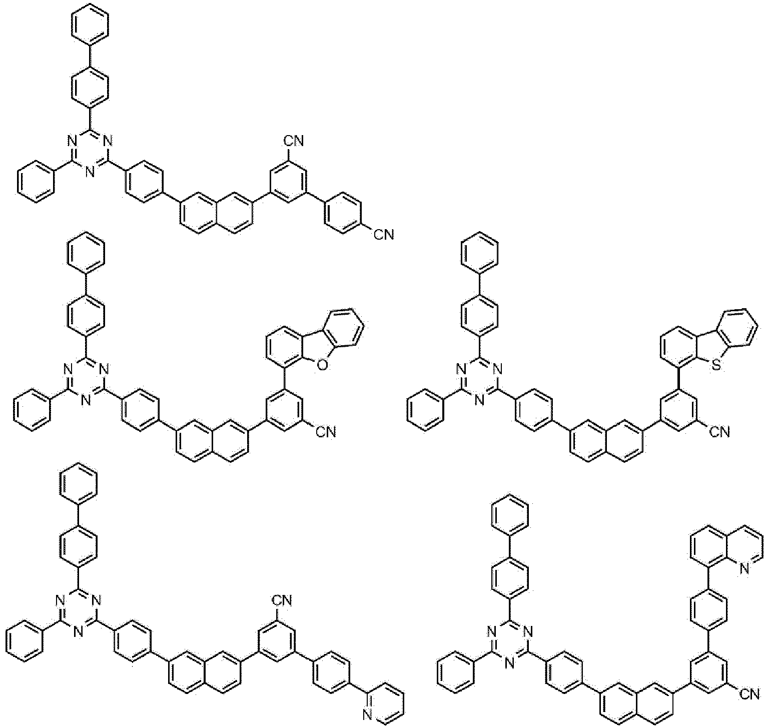

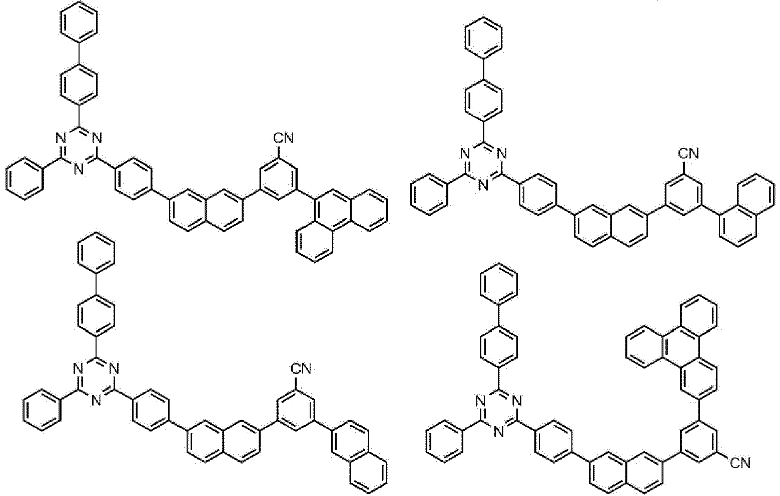

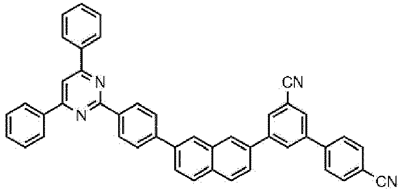

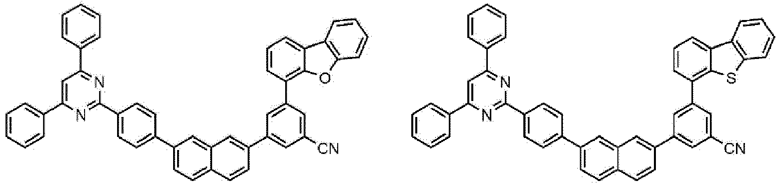

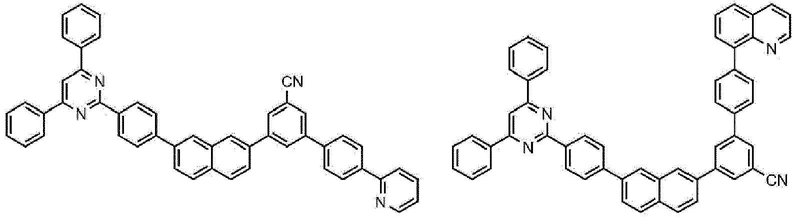

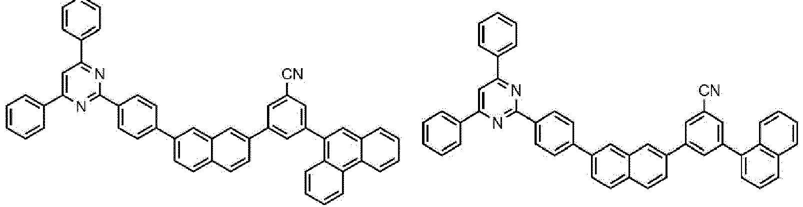

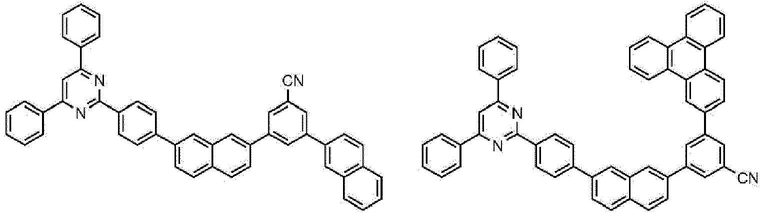

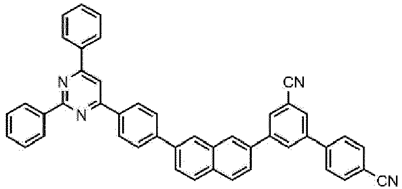

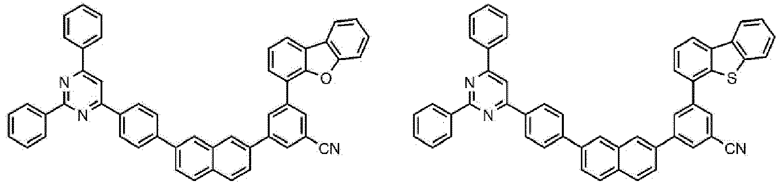

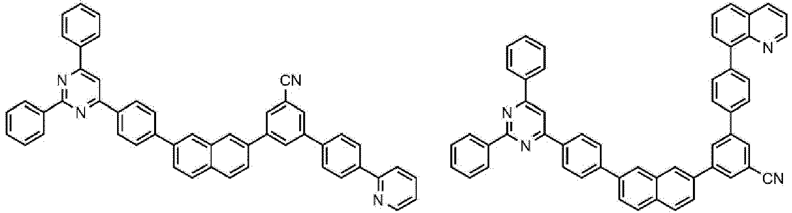

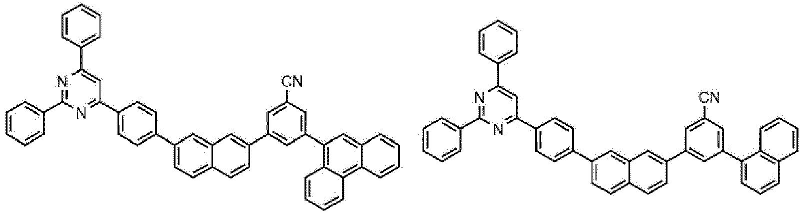

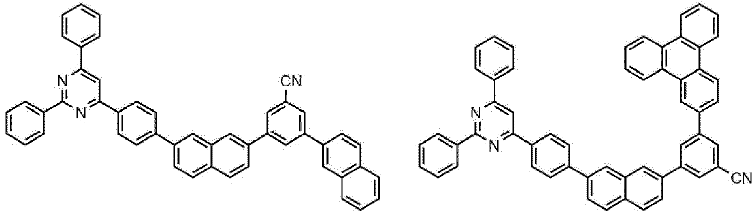

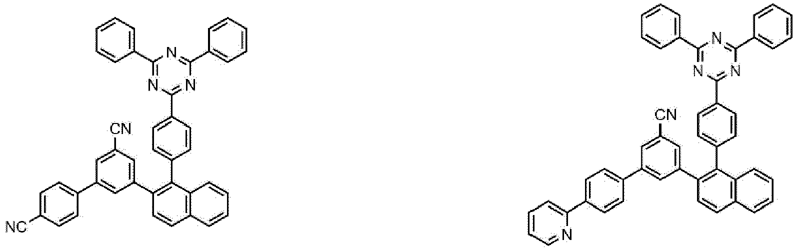

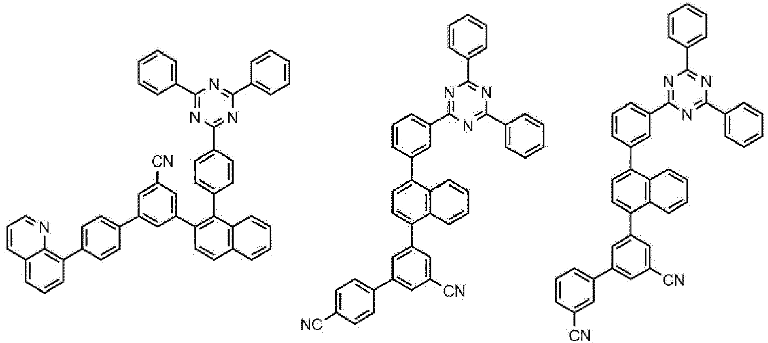

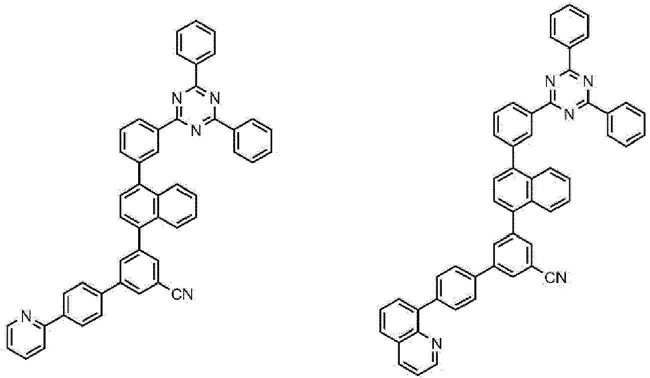

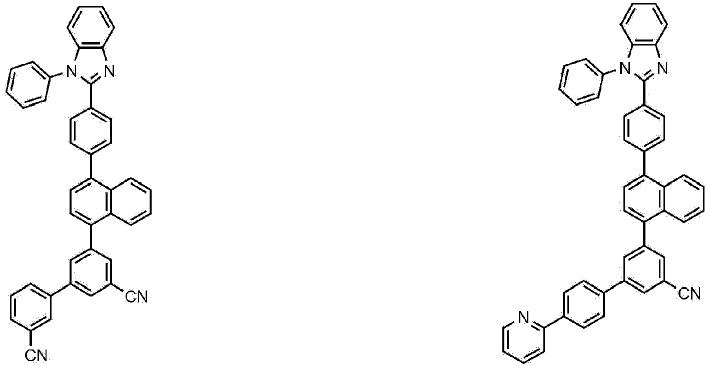

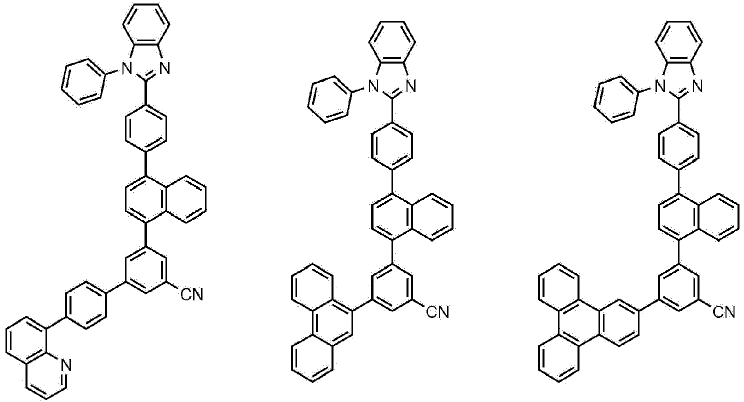

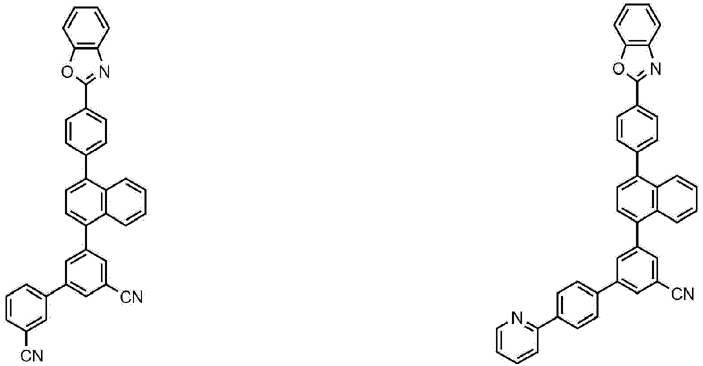

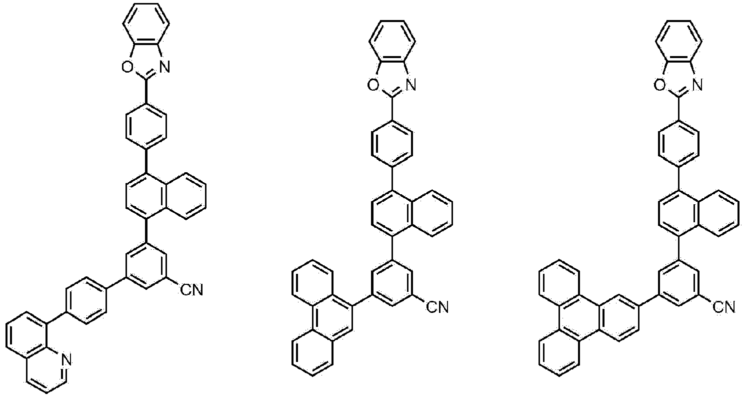

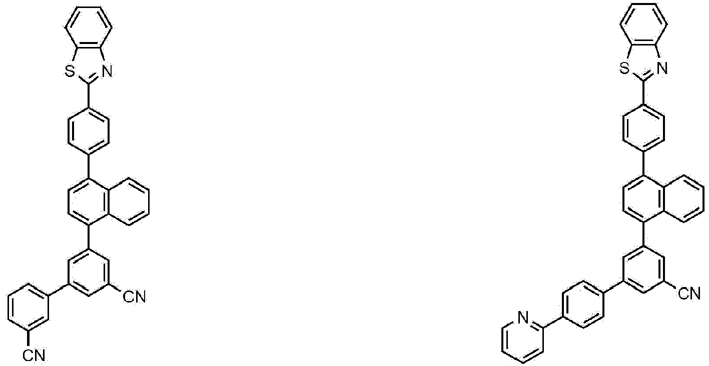

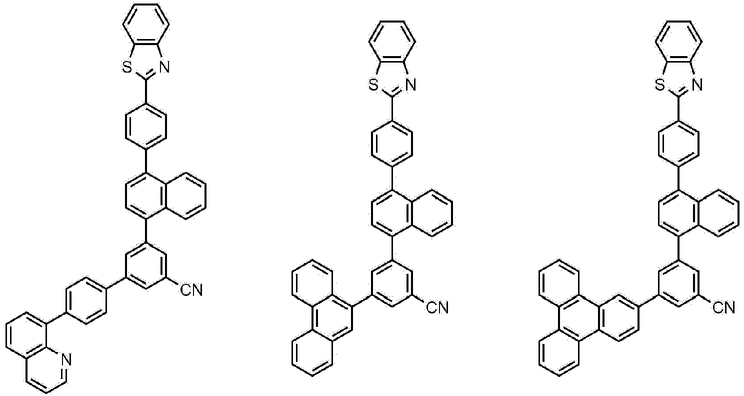

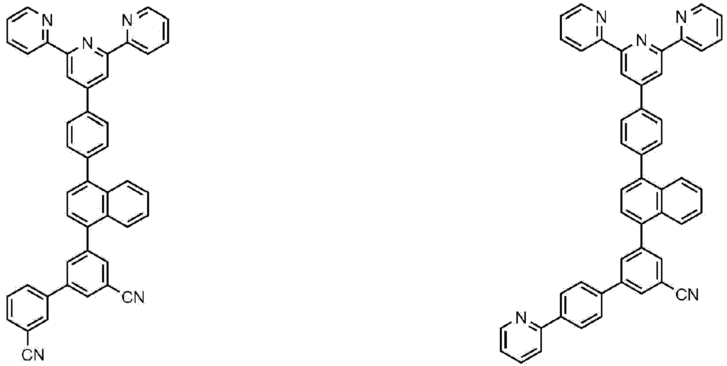

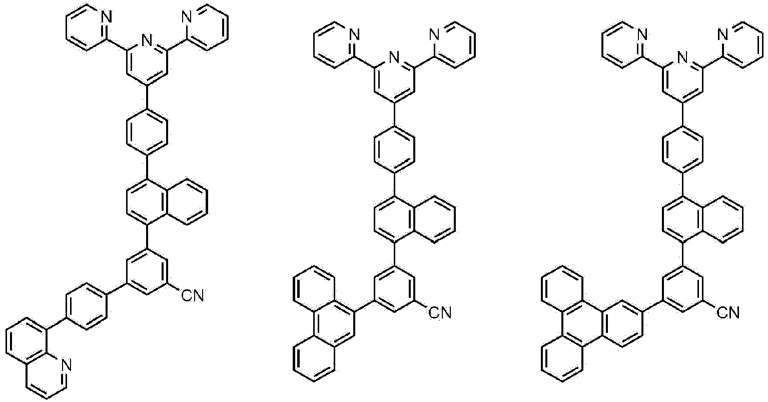

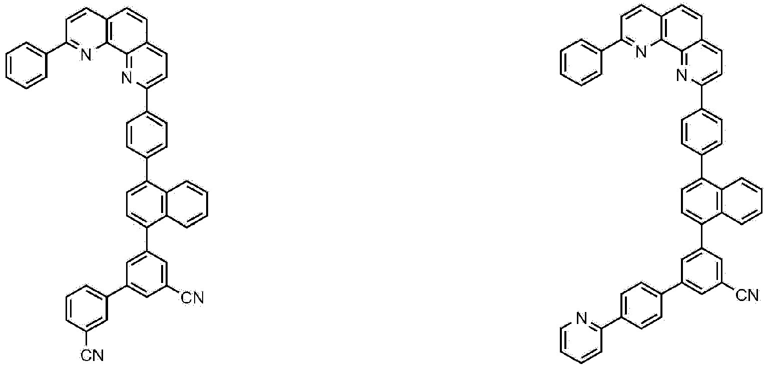

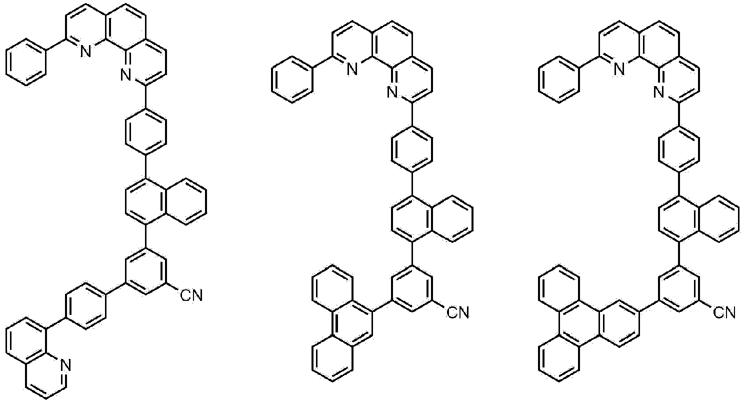

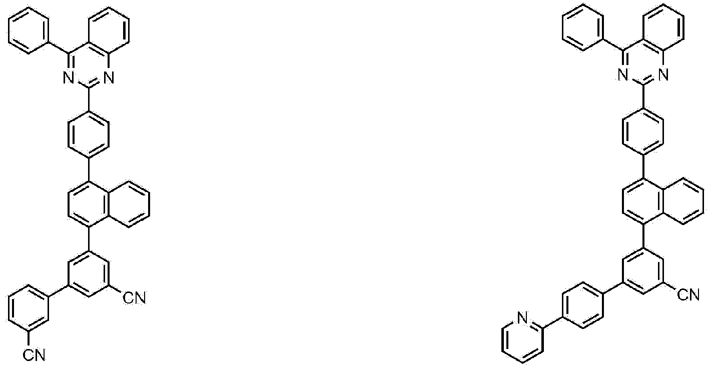

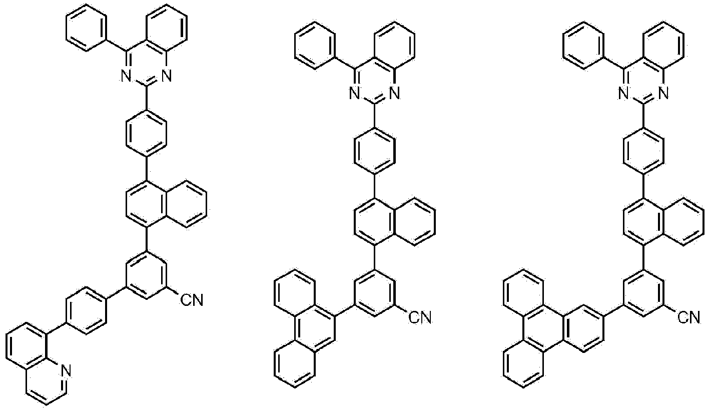

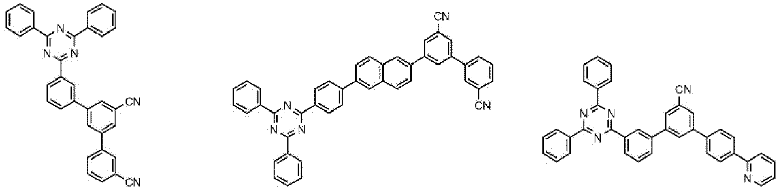

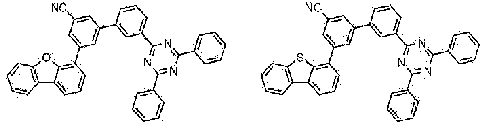

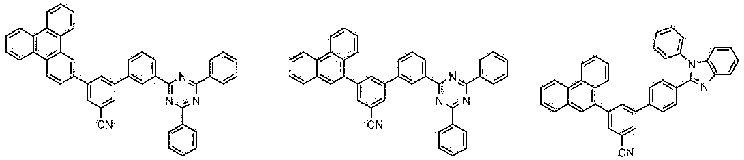

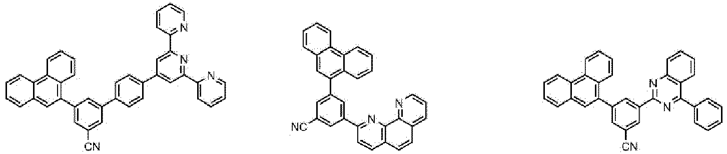

6. An organic light emitting device comprising a first electrode (2, 4), a second electrode (2, 4) provided to face the first electrode, and one or more organic material layers provided between the first electrode and the second electrode, wherein one or more of the organic material layers comprise the compound of any one of claims 1 to 5.

## Patentansprüche

1. Verbindung, dargestellt durch die folgende chemische Formel 1: 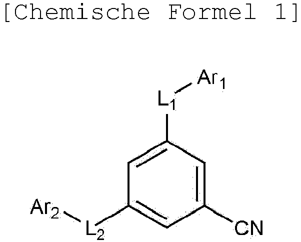 in der chemischen Formel 1
ist L₁ eine Einfachbindung oder substituiertes oder unsubstituiertes C₆₋₆₀-Arylen,
L₂ ist eine Einfachbindung, substituiertes oder unsubstituiertes C₆₋₆₀-Arylen oder substituiertes oder unsubstituiertes C₂₋₆₀-Heteroarylen, das mindestens eines von 0, N, Si und S enthält,
Ar₁ ist irgendeine der folgenden Strukturformeln: 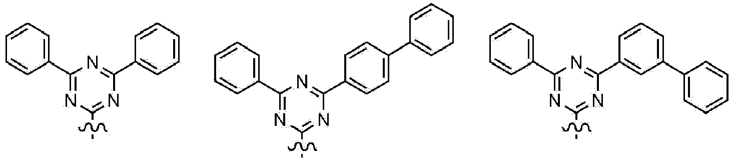 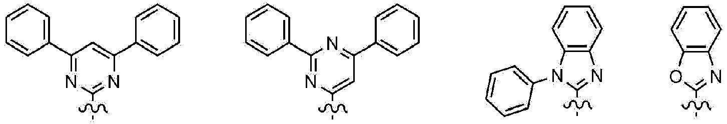 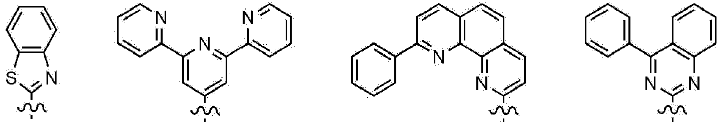
Ar₂ ist irgendeine der folgenden Strukturformeln: 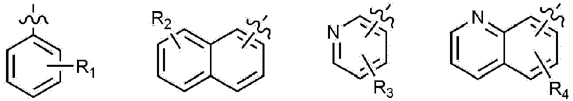 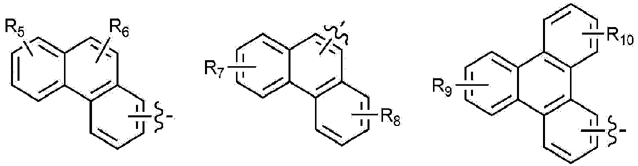 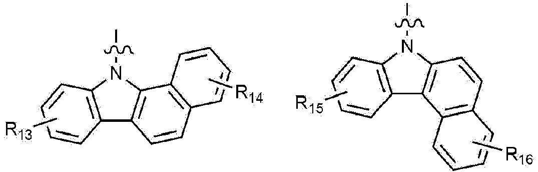 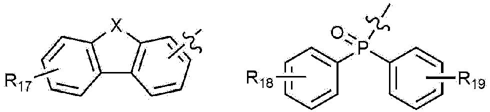
X ist C(R'₂) (R'₃), C(R'₄) (R'₅), S oder 0,
R₁ ist Cyano, Trifluormethyl oder Trifluormethoxy und
R'₂ bis R'₅ , R₂ bis R₁₀ und R₁₃ bis R₁₉ sind jeweils unabhängig Wasserstoff, Deuterium, Halogen, Cyano, Nitro, Amino, substituiertes oder unsubstituiertes C₁₋₆₀-Alkyl, C₁₋₆₀-Halogenalkyl, C₁₋₆₀-Halogenalkoxy, substituiertes oder unsubstituiertes C₃₋₆₀-Cycloalkyl, substituiertes oder unsubstituiertes C₂₋₆₀-Alkenyl, substituiertes oder unsubstituiertes C₆₋₆₀-Aryl oder eine substituierte oder unsubstituierte heterocyclische C₂₋₆₀-Gruppe, die mindestens eines von 0, N, Si und S enthält.

2. Verbindung gemäß Anspruch 1, wobei L₁ eine Einfachbindung oder irgendeine der folgenden Strukturformeln ist:

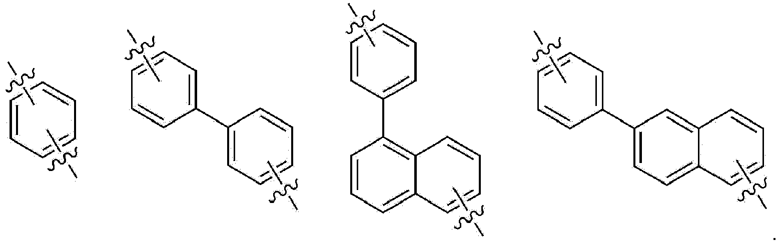

3. Verbindung gemäß Anspruch 1, worin L₂ eine Einfachbindung, 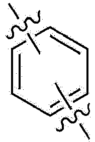 oder 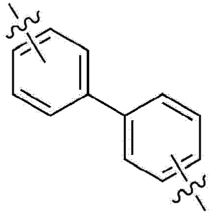 ist.

4. Verbindung gemäß Anspruch 1, worin R₂ bis R₁₀ und R₁₃ bis R₁₉ Wasserstoff sind.

5. Verbindung gemäß Anspruch 1, die aus den folgenden Verbindungen ausgewählt ist:

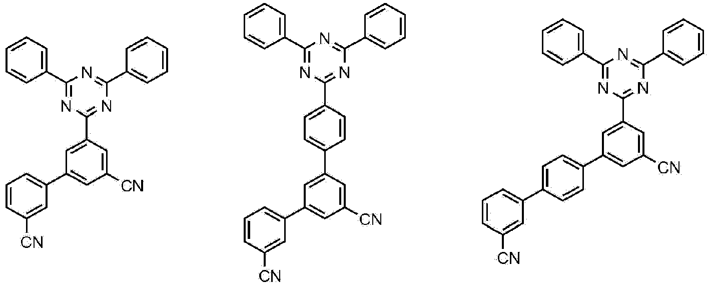

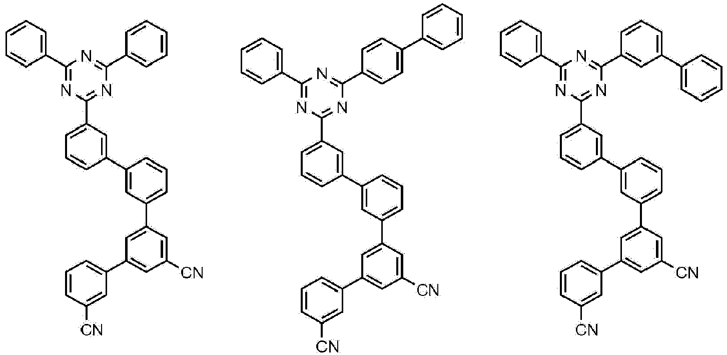

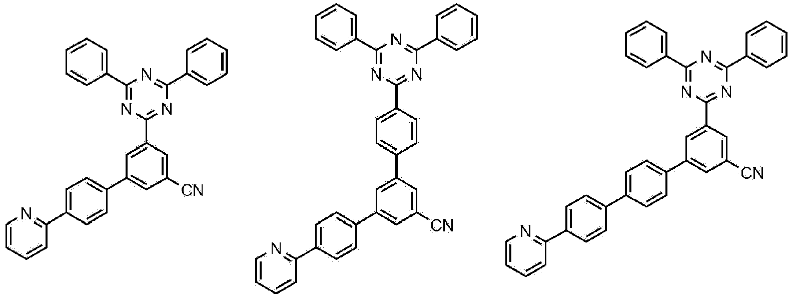

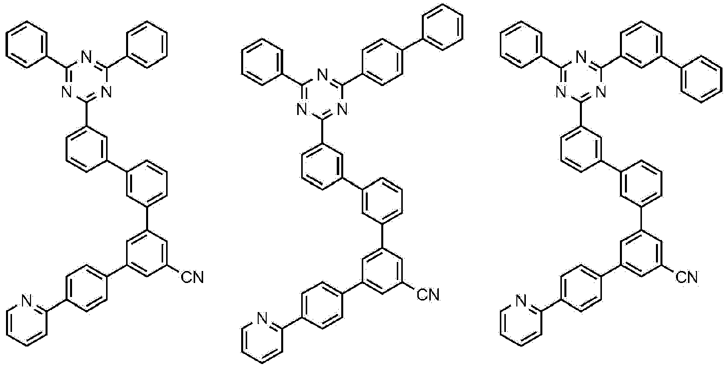

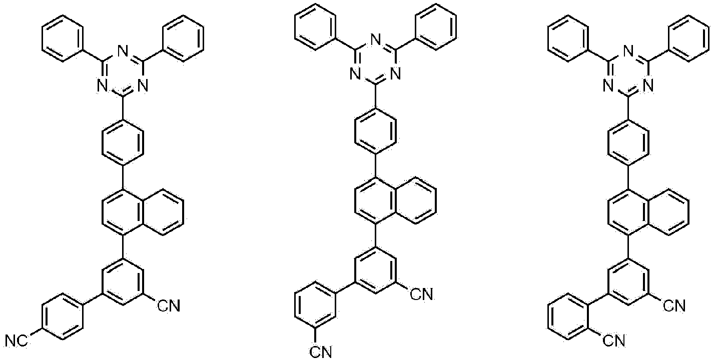

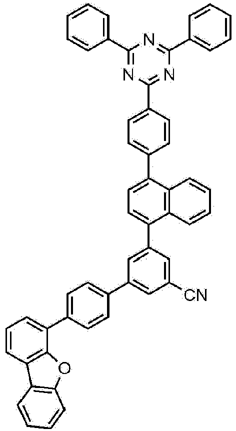

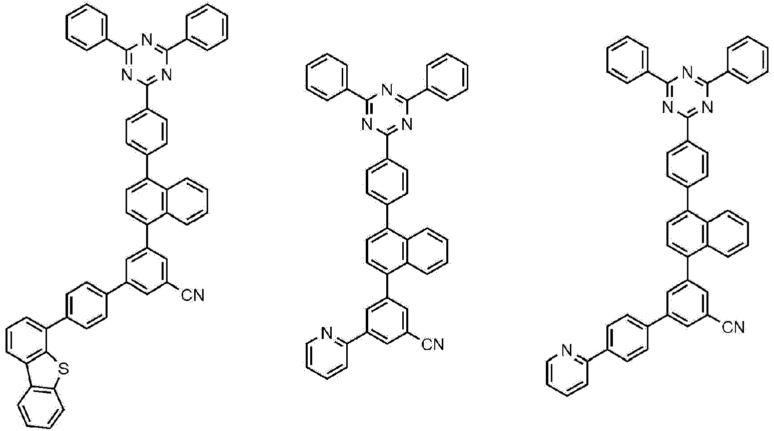

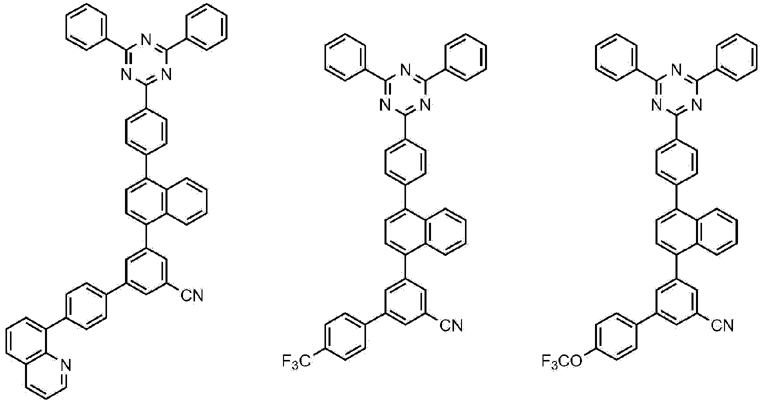

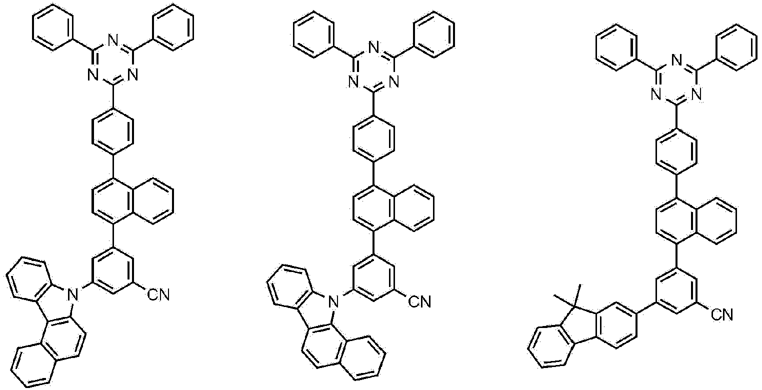

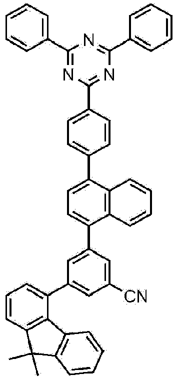

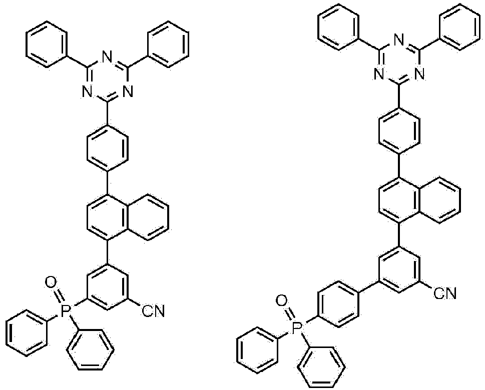

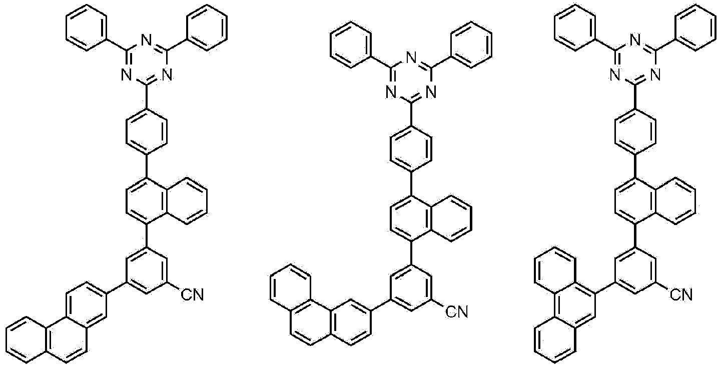

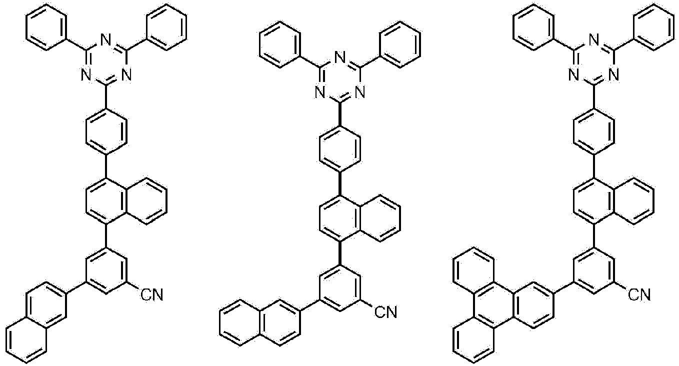

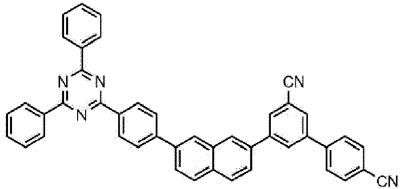

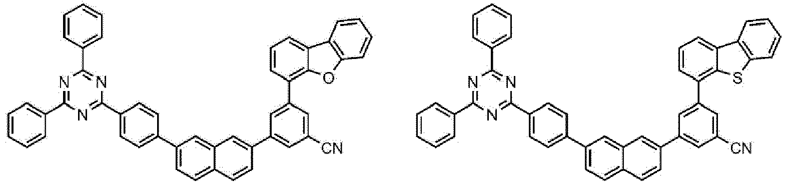

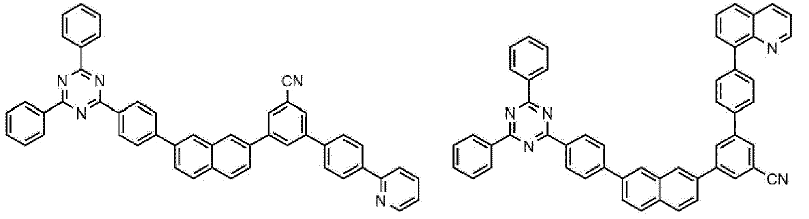

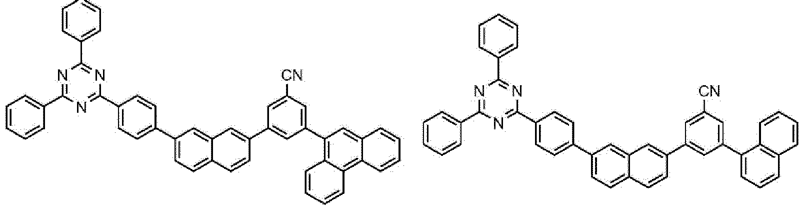

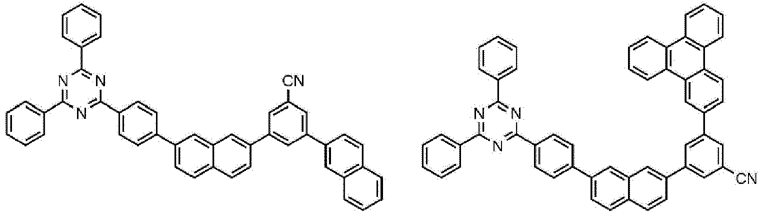

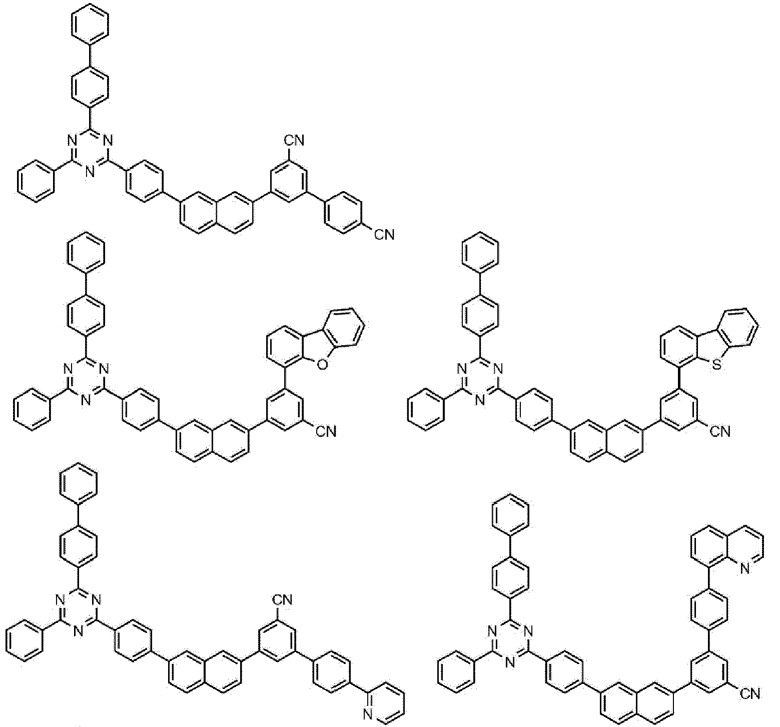

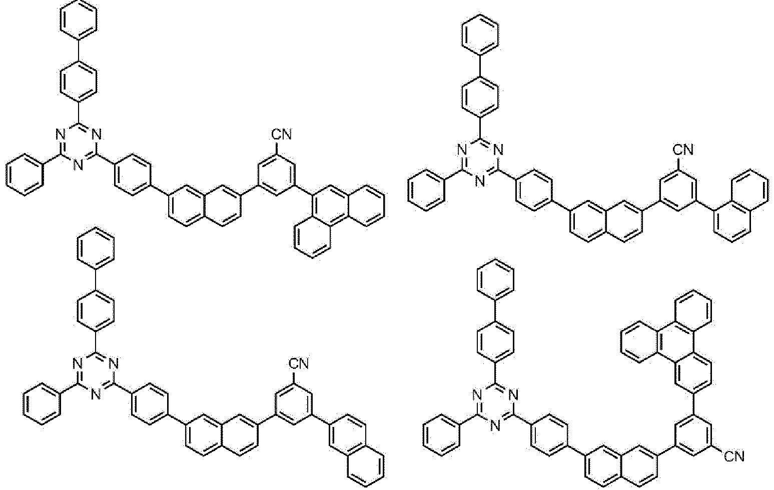

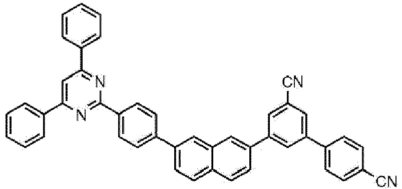

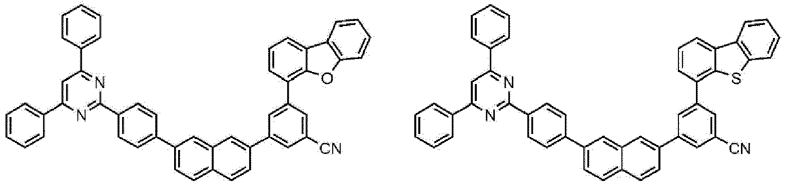

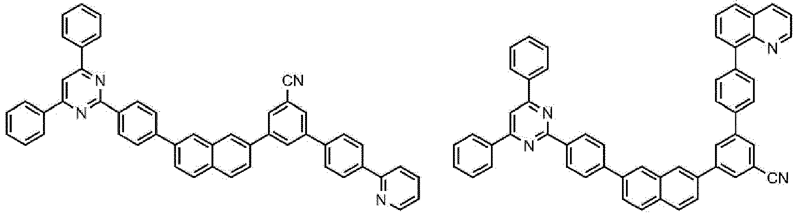

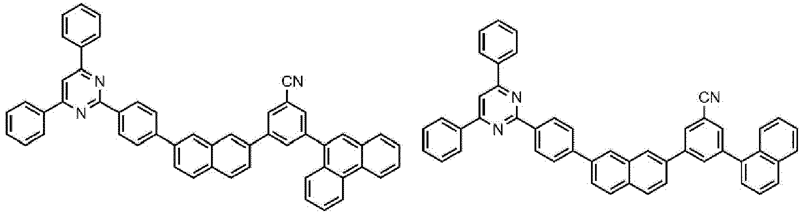

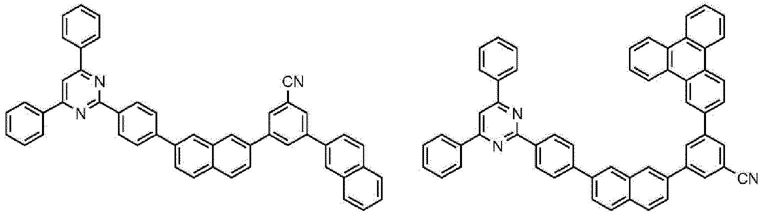

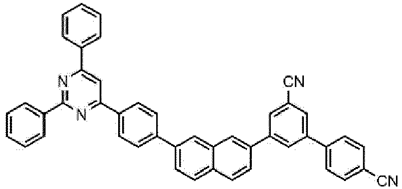

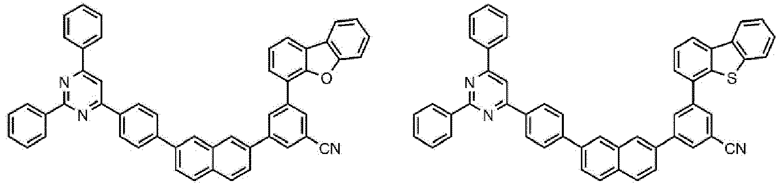

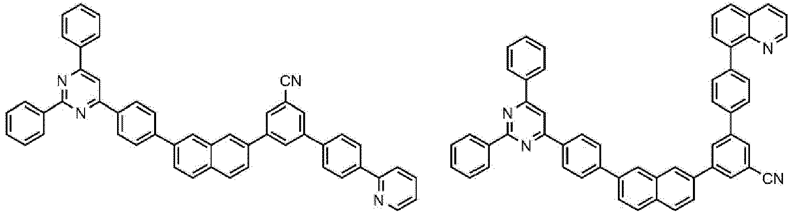

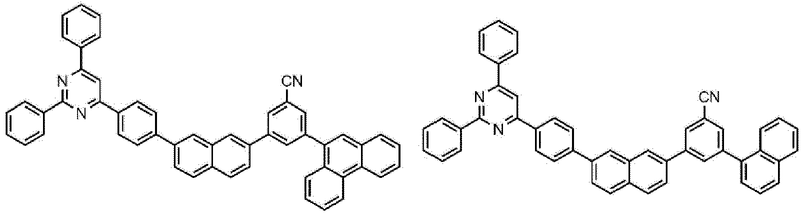

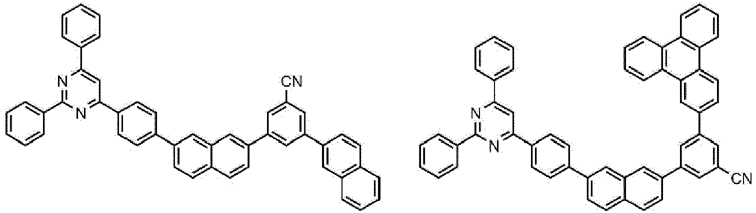

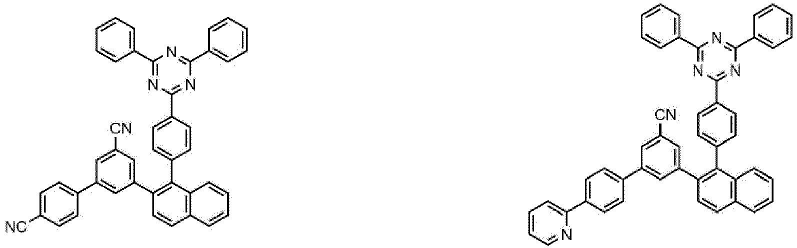

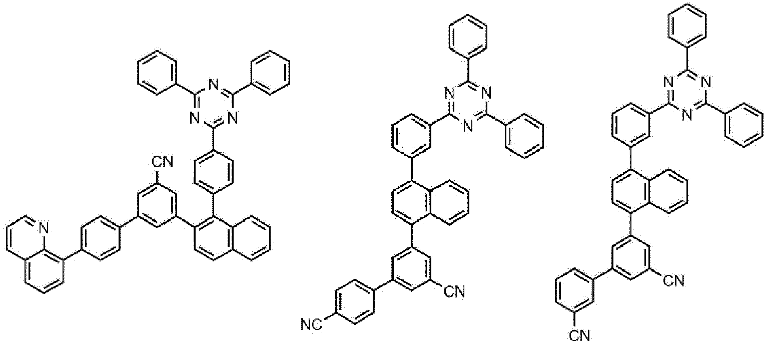

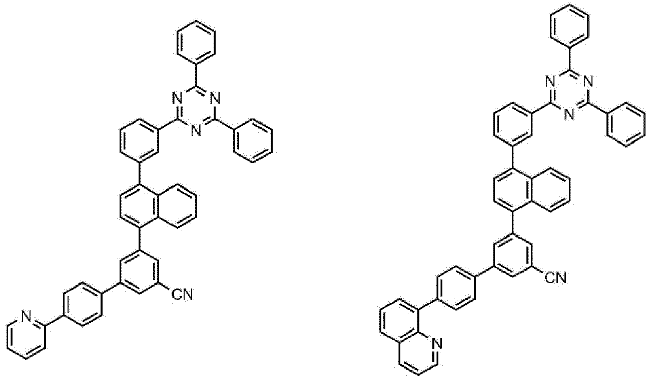

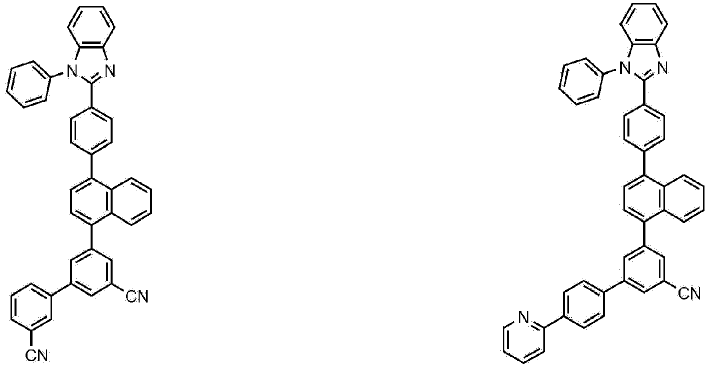

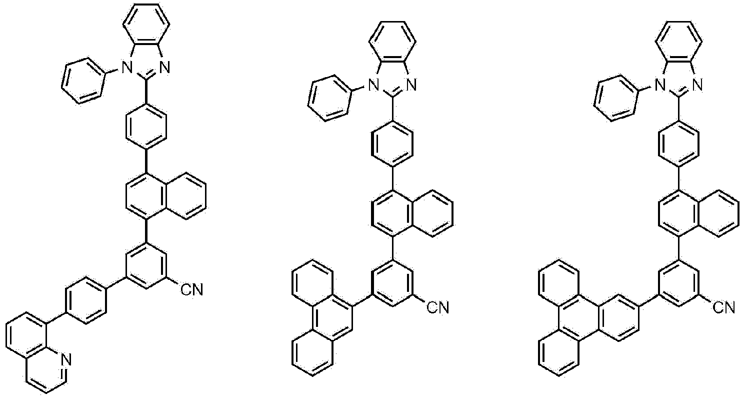

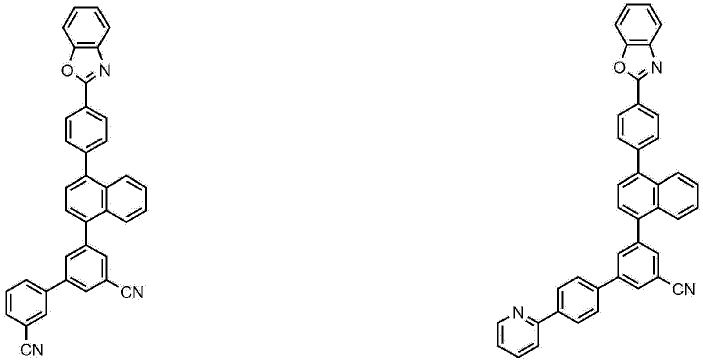

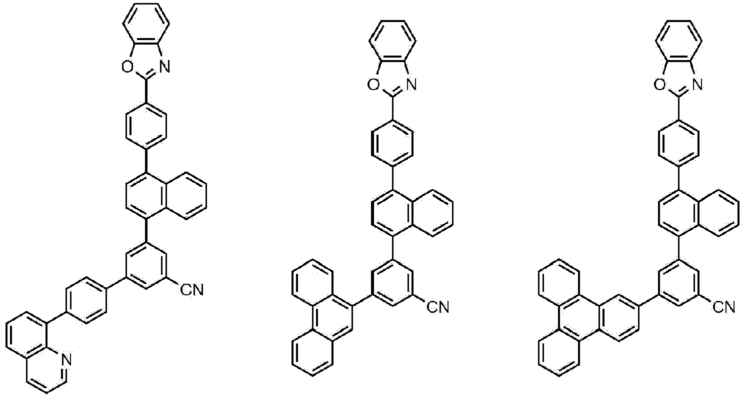

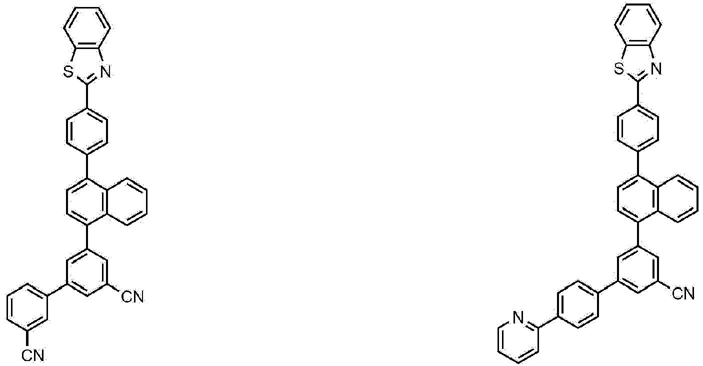

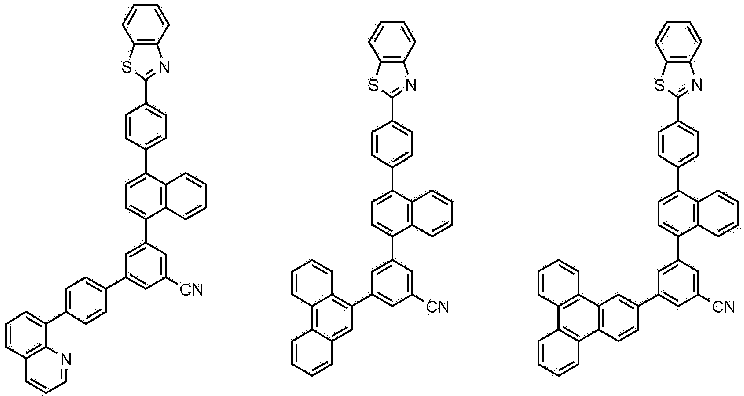

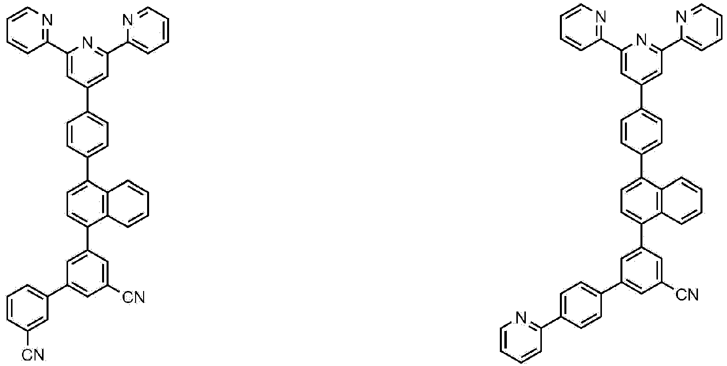

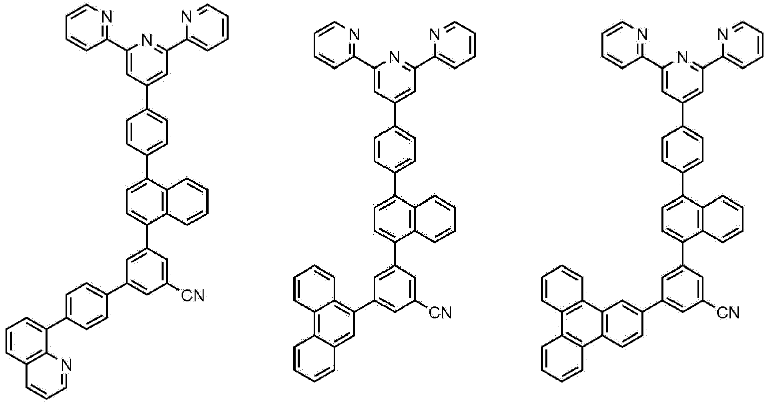

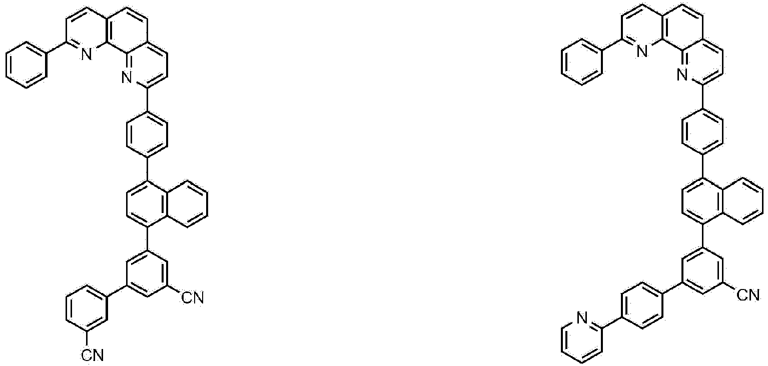

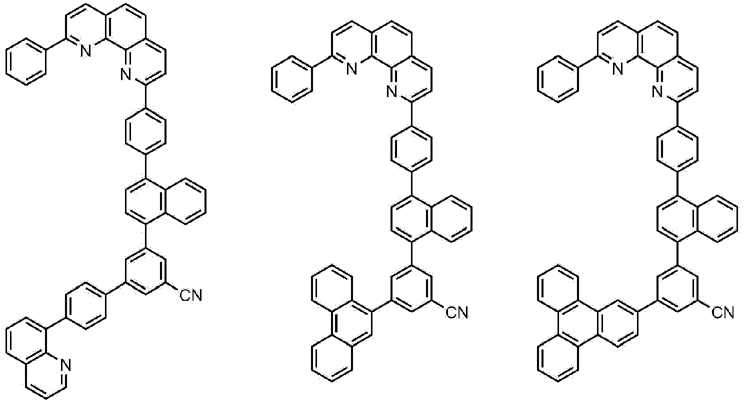

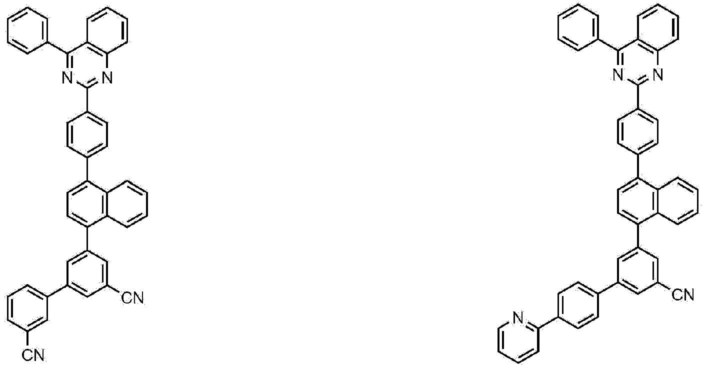

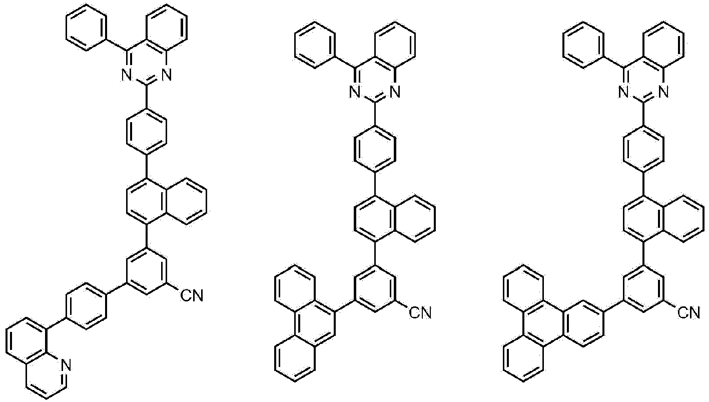

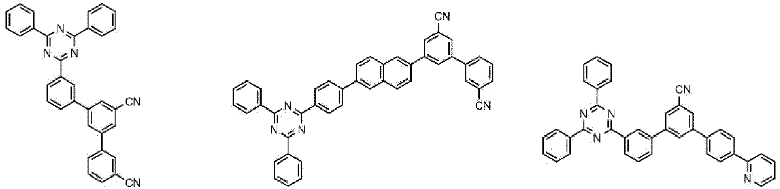

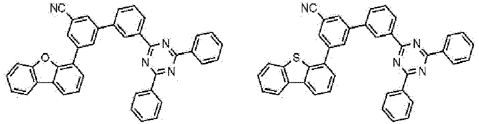

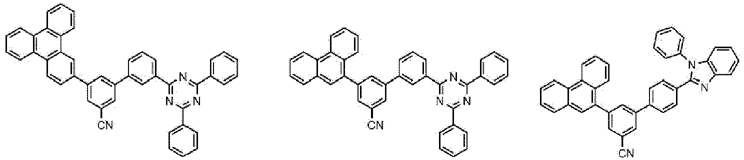

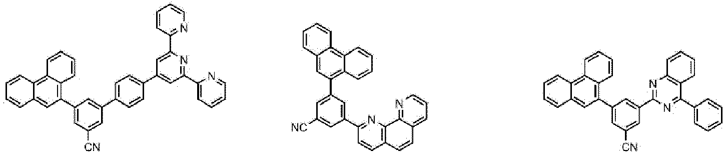

6. Organische lichtemittierende Vorrichtung, umfassend eine erste Elektrode (2, 4), eine zweite Elektrode (2, 4), die so angeordnet ist, dass sie der ersten Elektrode gegenübersteht, und eine oder mehrere organische Materialschichten, die zwischen der ersten Elektrode und der zweiten Elektrode angeordnet sind, wobei eine oder mehrere der organischen Materialschichten die Verbindung gemäß irgendeinem der Ansprüche 1 bis 5 umfasst.

## Revendications

1. Composé représenté par la formule chimique 1 suivante : 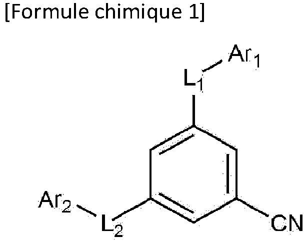 dans la formule chimique 1,
L₁ est une liaison simple ou un arylène en C₆₋₆₀ substitué ou non substitué ;
L₂ est une liaison simple, un arylène en C₆₋₆₀ substitué ou non substitué, ou un hétéroarylène en C₂₋₆₀ substitué ou non substitué contenant au moins l'un parmi O, N, Si et S;
Ar₁ est l'une quelconque des formules structurelles suivantes : 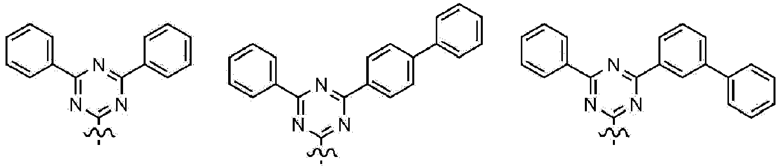 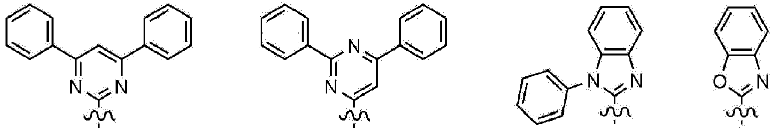 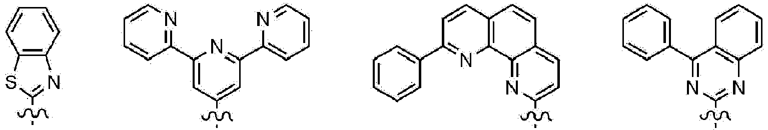
Ar₂ est l'une quelconque des formules structurelles suivantes : 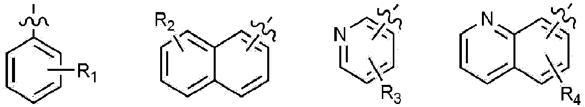 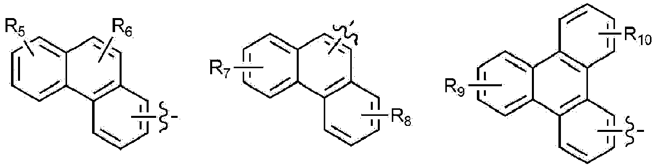 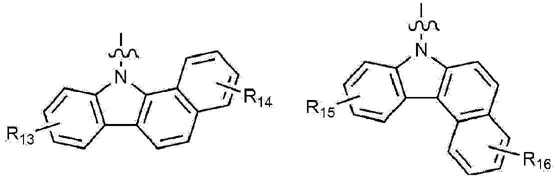 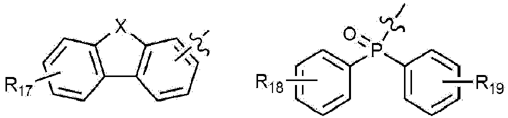
X est C(R'₂)(R'₃), Si(R'₄)(R'₅), S ou O ;
R₁ est cyano, trifluorométhyle ou trifluorométhoxy ; et
R'₂ à R'₅, R₂ à R₁₀ et R₁₃ à R₁₉ sont chacun indépendamment hydrogène, deutérium ; halogène, cyano, nitro, amino, alkyle en C₁₋₆₀ substitué ou non substitué, halogénoalkyle en C₁₋₆₀, halogénoalcoxy en C₁₋₆₀, cycloalkyle en C₃₋₆₀ substitué on non substitué, alcényle en C₂₋₆₀ substitué ou non substitué, aryle en C₆₋₆₀ substitué ou non substitué ou un groupe hétérocyclique en C₂₋₆₀ substitué ou non substitué contenant au moins l'un parmi O, N, Si et S.

2. Composé selon la revendication 1, dans lequel L₁ est une liaison simple ou l'une quelconque des formules structurelles suivantes :

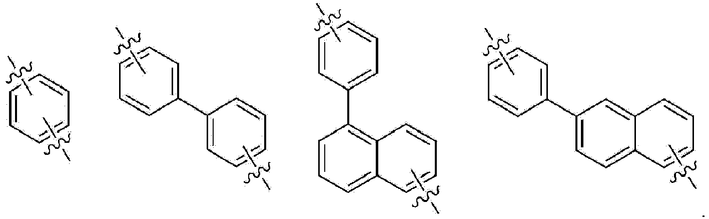

3. Composé selon la revendication 1, dans lequel L₂ est une liaison simple, 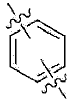 ou

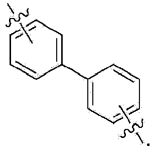

4. Composé selon la revendication 1, dans lequel R₂ à R₁₀ et R₁₃ à R₁₉ sont hydrogène.

5. Composé selon la revendication 1, qui est sélectionné à partir des composés suivants :

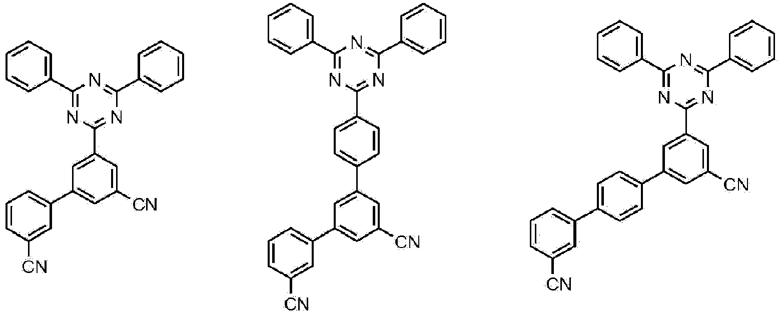

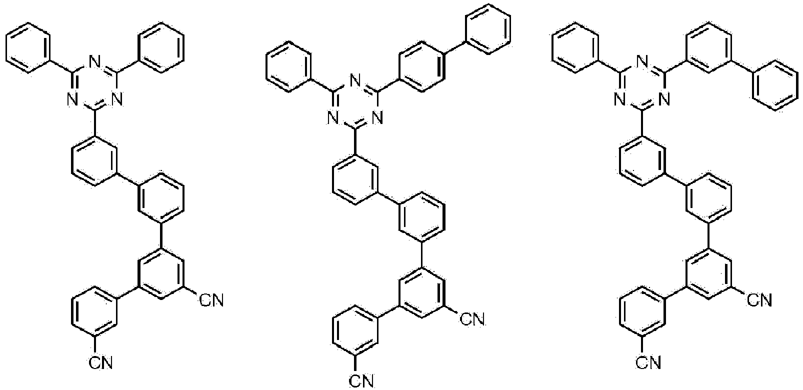

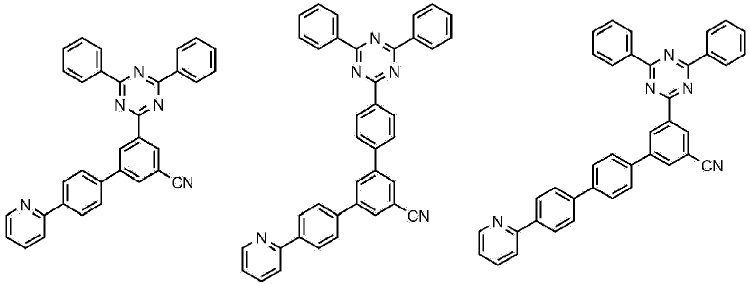

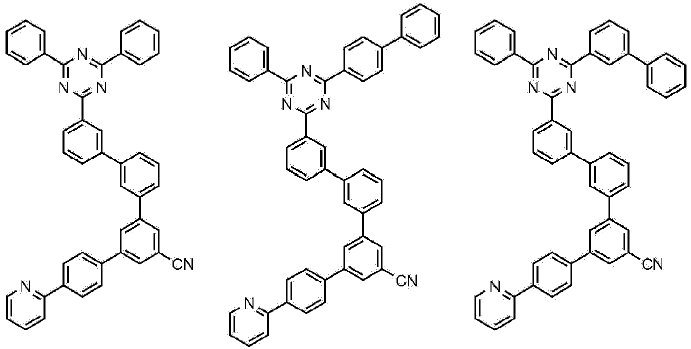

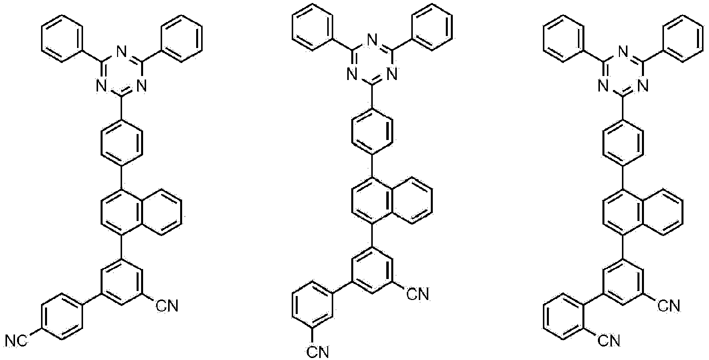

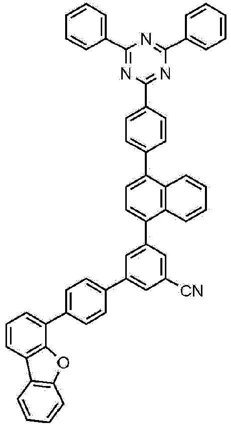

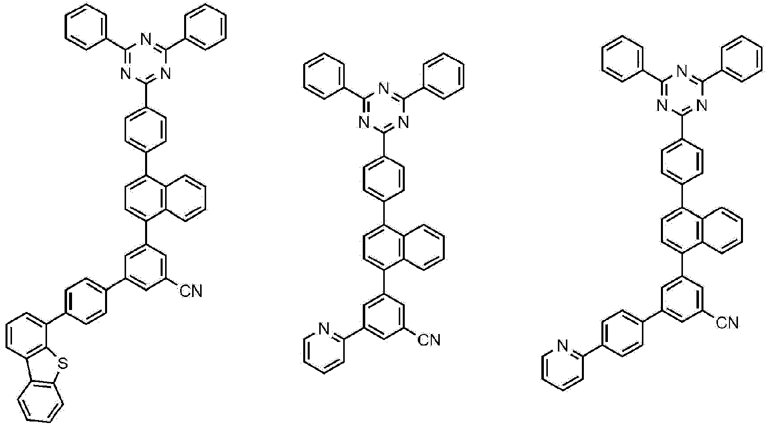

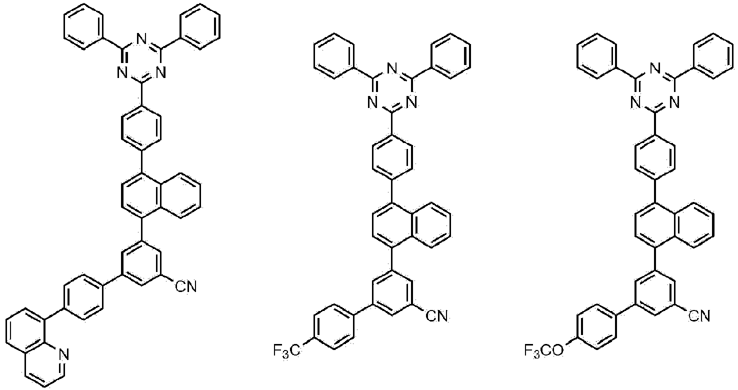

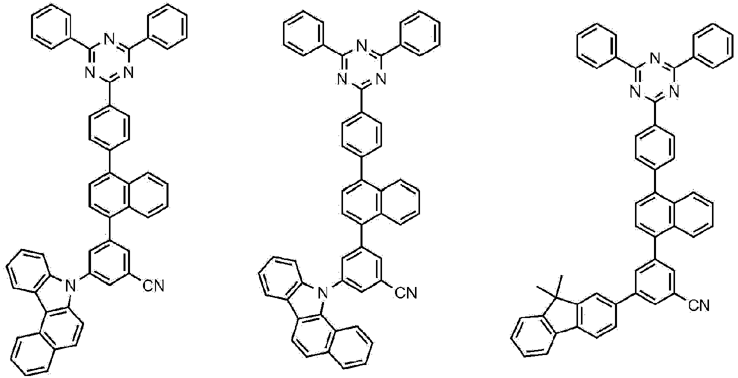

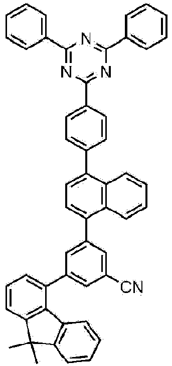

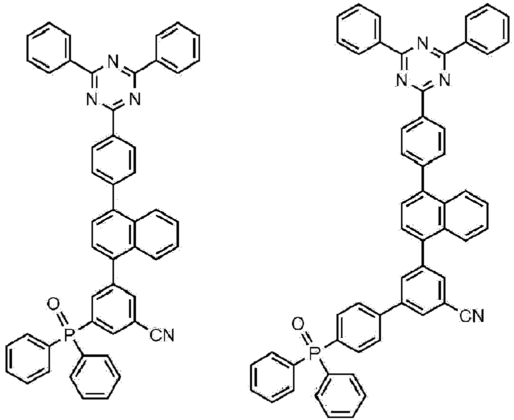

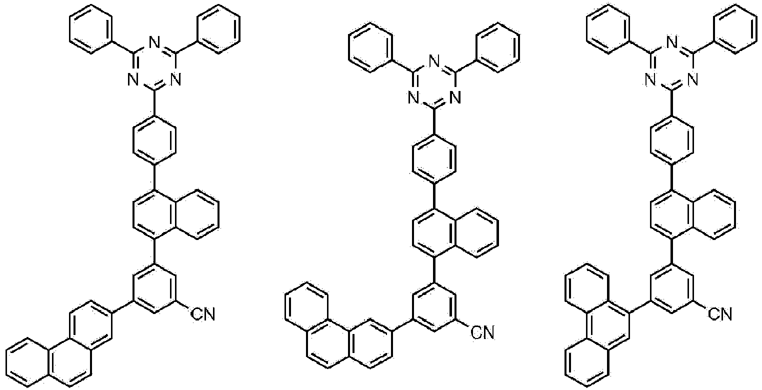

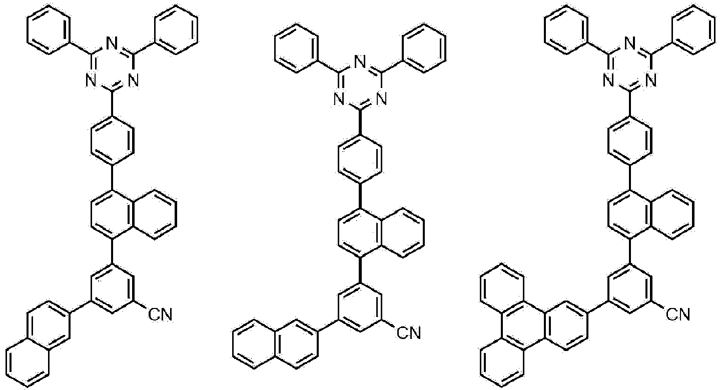

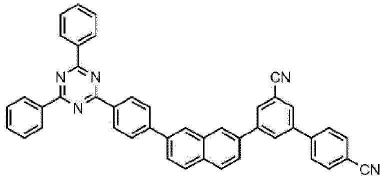

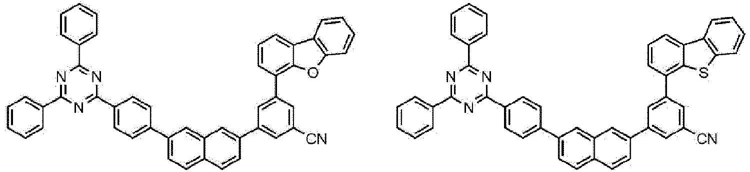

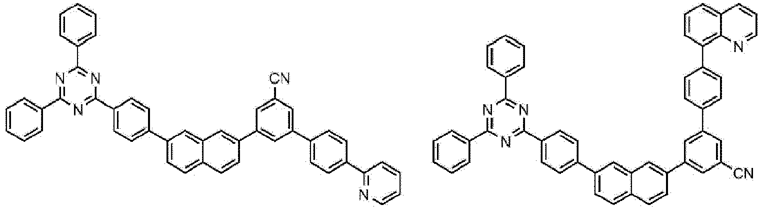

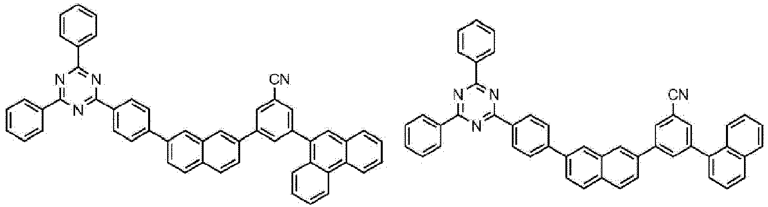

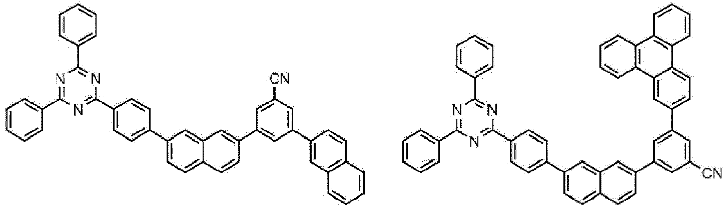

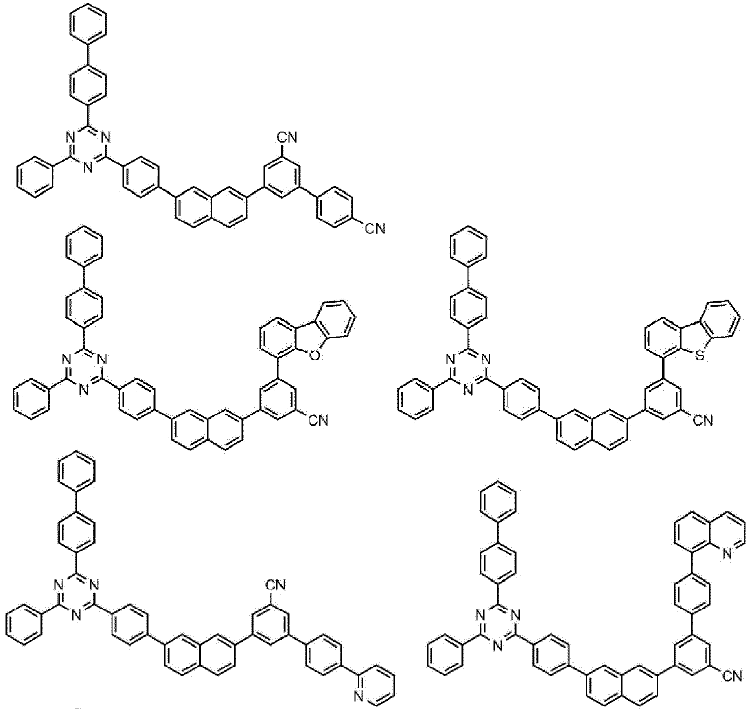

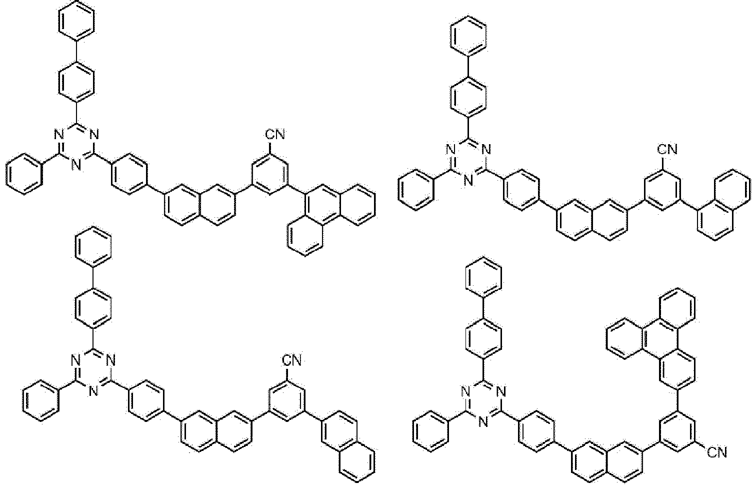

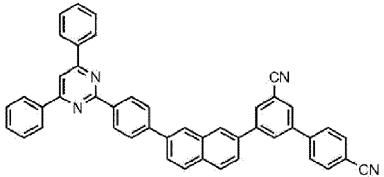

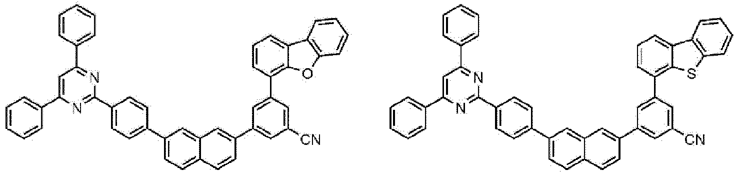

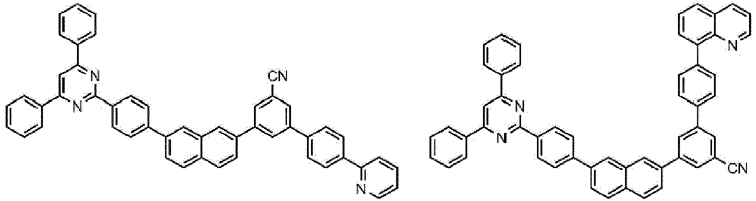

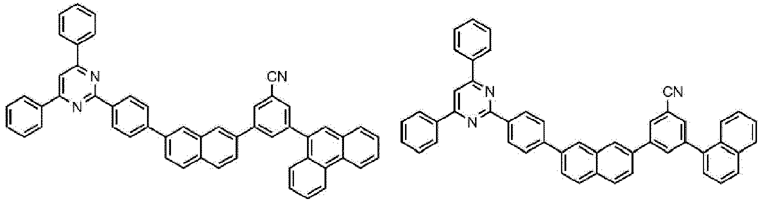

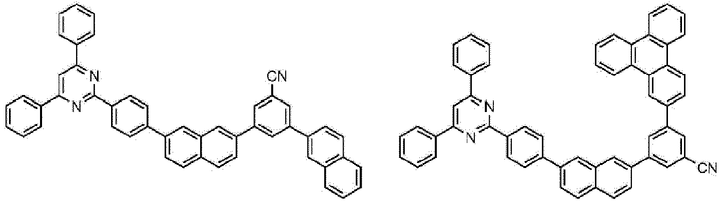

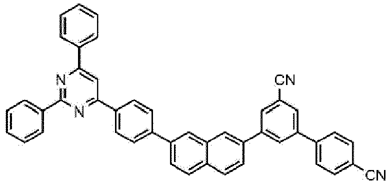

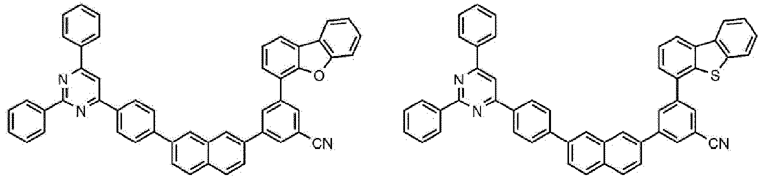

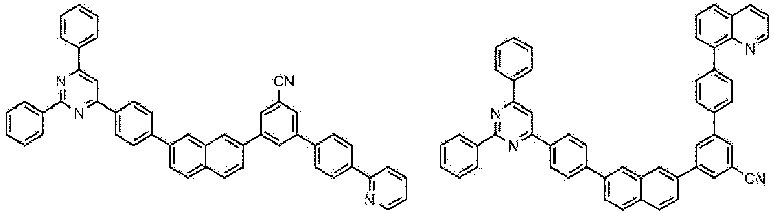

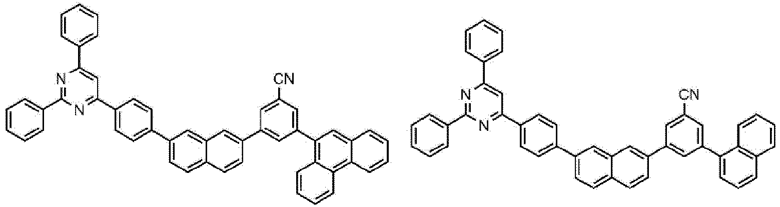

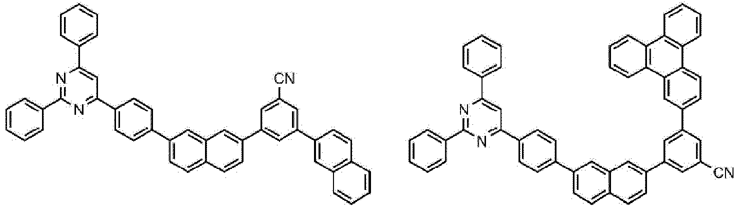

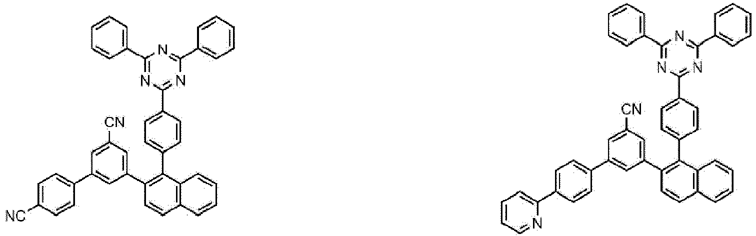

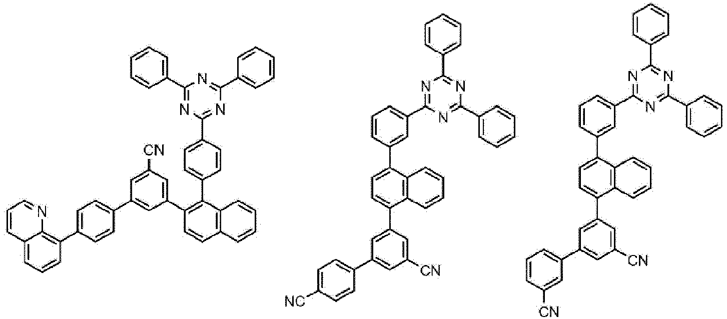

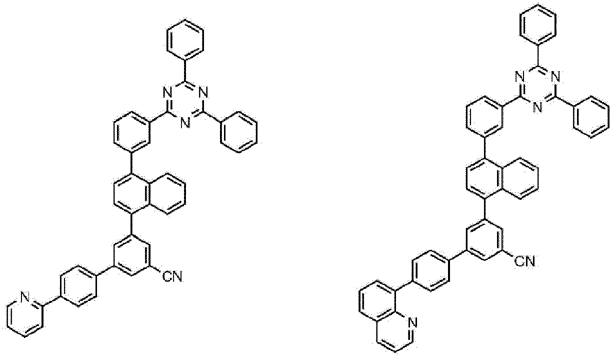

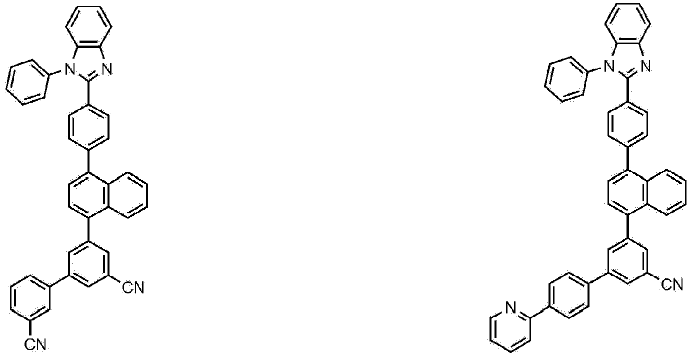

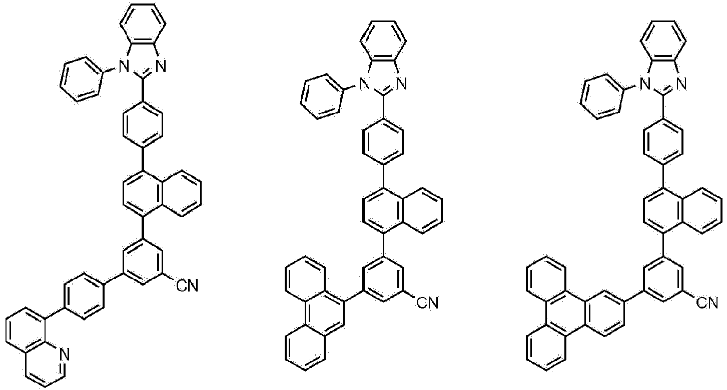

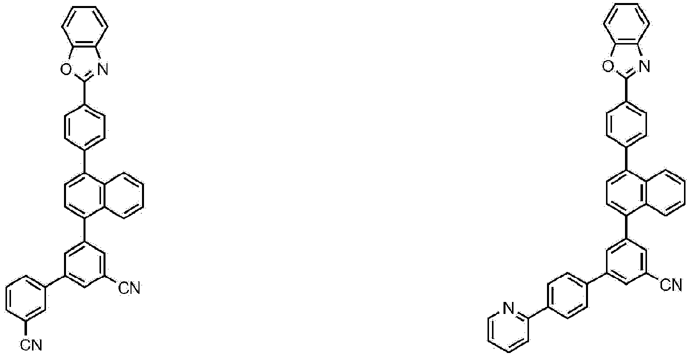

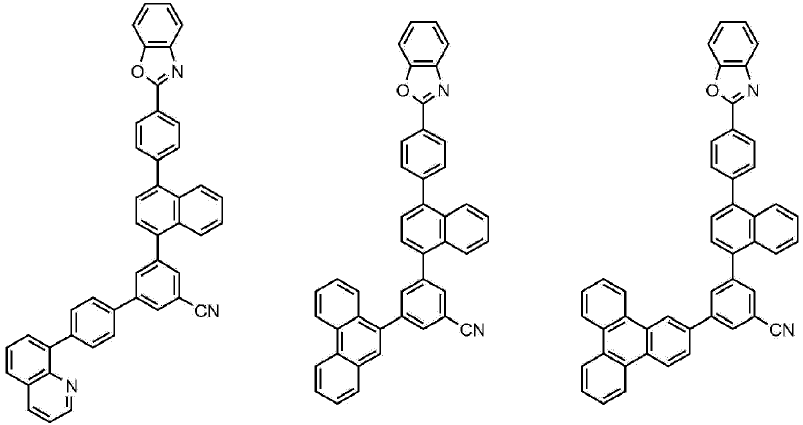

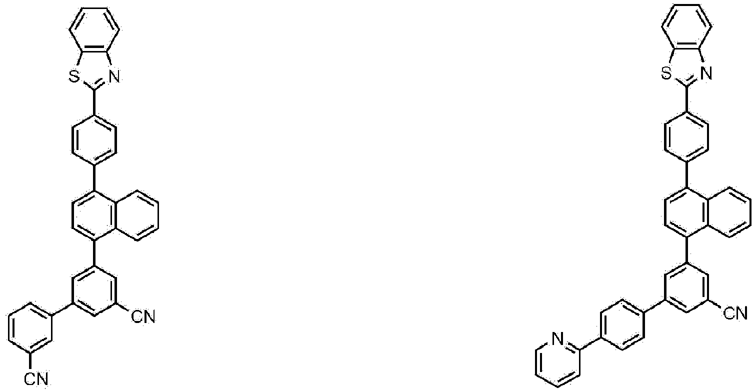

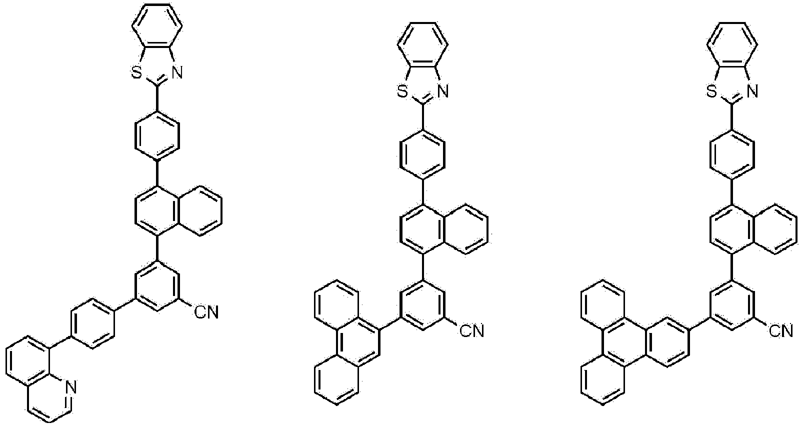

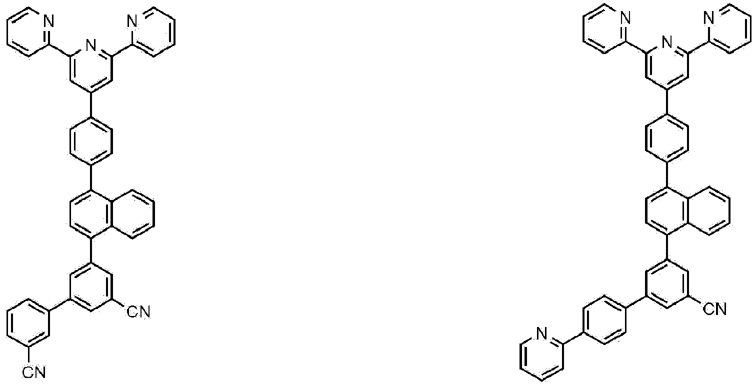

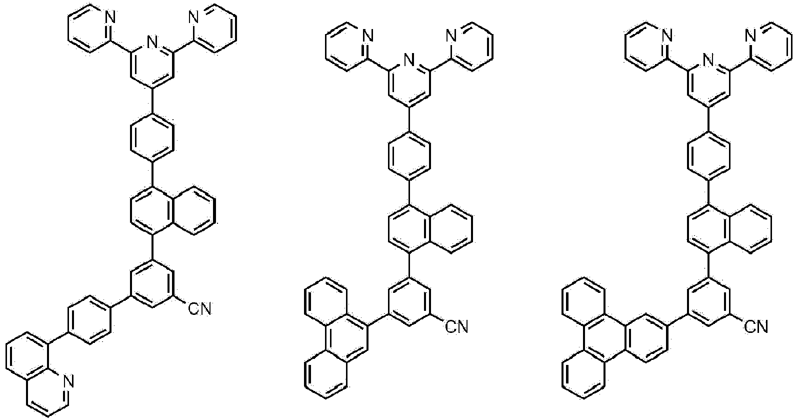

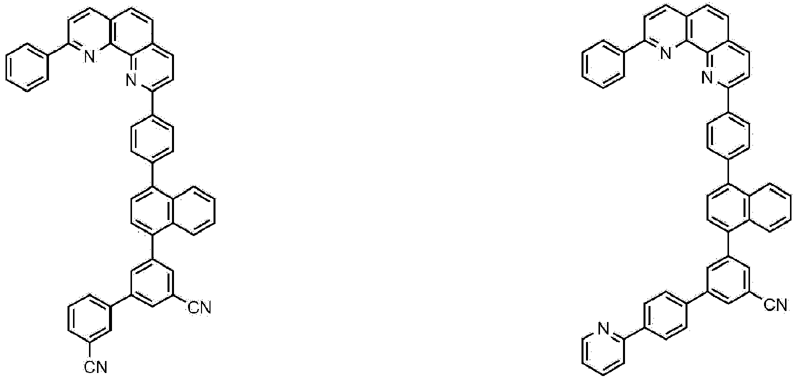

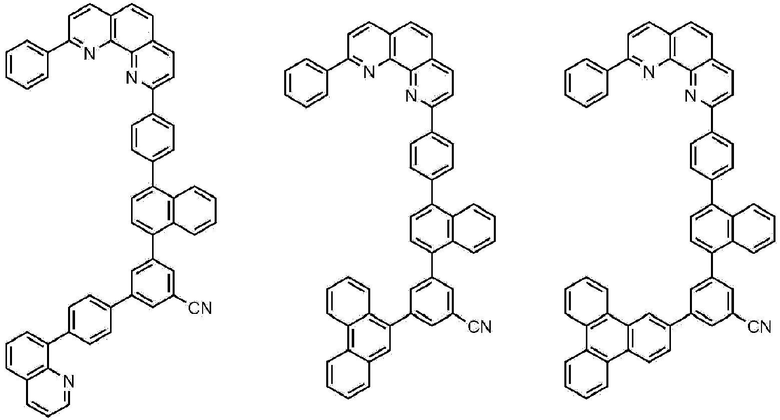

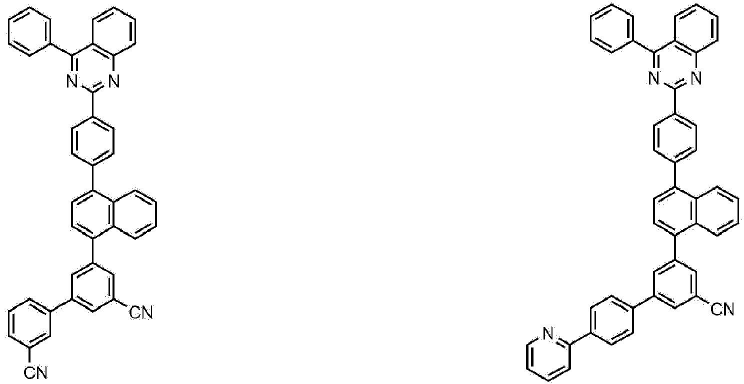

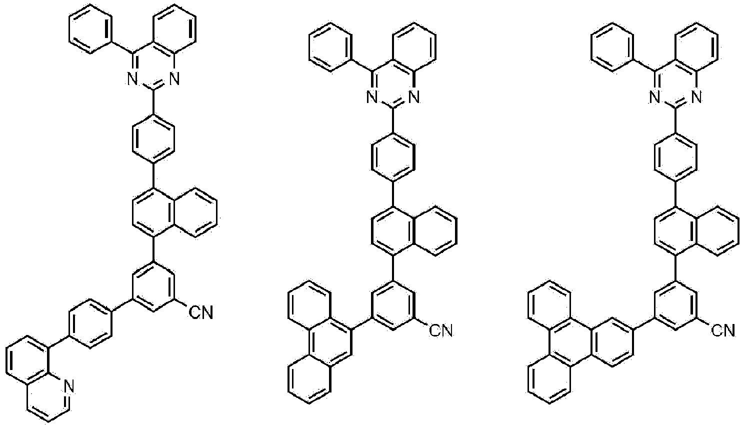

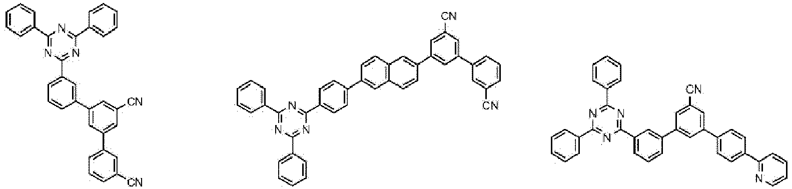

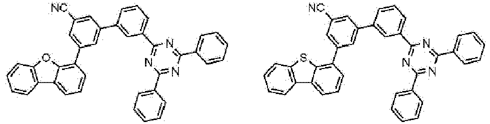

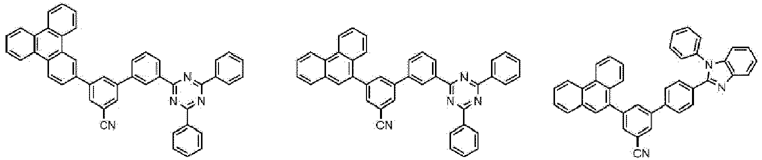

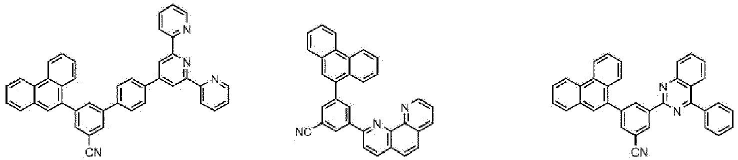

6. Dispositif électroluminescent organique comprenant une première électrode (2, 4), une seconde électrode (2, 4) prévue pour faire face à la première électrode, et une ou plusieurs couches de matériau organique prévues entre la première électrode et la seconde électrode, dans lequel une ou plusieurs des couches de matériau organique comprennent le composé selon l'une quelconque des revendications 1 à 5.
